(19) Europäisches Patentamt
European Patent Office
Office européen des brevets

(11) EP 2 363 821 B1

(12) **EUROPÄISCHE PATENTSCHRIFT**

(45) Veröffentlichungstag und Bekanntmachung des Hinweises auf die Patenterteilung:
**22.01.2020 Patentblatt 2020/04**

(51) Int Cl.:
***G16B 20/00*** *(2019.01)* ***G16B 30/00*** *(2019.01)*

(21) Anmeldenummer: **10012880.0**

(22) Anmeldetag: **23.12.2003**

(54) **Verfahren und Vorrichtung zum Optimieren einer Nucleotidsequenz zur Expression eines Proteins**

Method and device for optimising a nucleotide sequence for expressing a protein

Procédé et dispositif d'optimisation d'une séquence de nucléotide pour l'expression d'une protéine

(84) Benannte Vertragsstaaten:
**AT BE BG CH CY CZ DE DK EE ES FI FR GB GR HU IE IT LI LU MC NL PT RO SE SI SK TR**

(30) Priorität: **23.12.2002 DE 10260805**

(43) Veröffentlichungstag der Anmeldung:
**07.09.2011 Patentblatt 2011/36**

(62) Dokumentnummer(n) der früheren Anmeldung(en) nach Art. 76 EPÜ:
**03785937.8 / 1 584 058**

(73) Patentinhaber: **Thermo Fisher Scientific GENEART GmbH**
**93059 Regensburg (DE)**

(72) Erfinder:
- **Raab, David**
  **93176 Beratshausen (DE)**
- **Graf, Marcus**
  **93053 Regensburg (DE)**
- **Notka, Frank**
  **93059 Regensburg (DE)**
- **Wagner, Ralf**
  **93049 Regensburg (DE)**

(74) Vertreter: **Wöhler, Christian**
**Life Technologies**
**Frankfurter Straße 129 B**
**64293 Darmstadt (DE)**

(56) Entgegenhaltungen:
**EP-A- 1 156 112** **WO-A-98/34640**
**US-A1- 2002 072 864**

- **WILLIAMS D P ET AL: "DESIGN, SYNTHESIS AND EXPRESSION OF A HUMAN INTERLEUKIN-2 GENE INCORPORATING THE CODON USAGE BIAS FOUND IN HIGHLY EXPRESSED ESCHERICHIA COLI GENES", NUCLEIC ACIDS RESEARCH, OXFORD UNIVERSITY PRESS, SURREY, GB, Bd. 16, Nr. 22, 25. November 1988 (1988-11-25), Seiten 10453-10467, XP000007466, ISSN: 0305-1048**
- **HOOVER DAVID M ET AL: "DNAWorks: an automated method for designing oligonucleotides for PCR-based gene synthesis", NUCLEIC ACIDS RESEARCH, OXFORD UNIVERSITY PRESS, SURREY, GB, Bd. 30, Nr. 10, 15. Mai 2002 (2002-05-15), Seiten E43-1, XP002301503, ISSN: 0305-1048**
- **CHRISLAINE WITHERS-MARTINEZ ET AL: "PCR-based gene synthesis as an efficient approach for expression of the A+T-rich malaria genome", PROTEIN ENGINEERING, OXFORD UNIVERSITY PRESS, SURREY, GB, Bd. 12, Nr. 12, Dezember 1999 (1999-12), Seiten 1113-1120, XP002170719, ISSN: 0269-2139**
- **LEE F ET AL: "ISOLATION OF COMPLEMENTARY DNA FOR A HUMAN GRANULOCYTE-MACROPHAGE COLONY-STIMULATING FACTOR BY FUNCTIONAL EXPRESSION IN MAMMALIAN CELLS", PROCEEDINGS OF THE NATIONAL ACADEMY OF SCIENCES OF THE UNITED STATES OF AMERICA, Bd. 82, Nr. 13, 1985, Seiten 4360-4364, XP002341521, ISSN: 0027-8424**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- MEAZZA R ET AL: "Identification of a novel interleukin-15 (IL-15) transcript isoform generated by alternative splicing in human small cell lung cancer cell lines.", ONCOGENE. 16 MAY 1996, Bd. 12, Nr. 10, 16. Mai 1996 (1996-05-16), Seiten 2187-2192, XP009052726, ISSN: 0950-9232

## Beschreibung

Die Erfindung betrifft allgemein die Erzeugung synthetischer DNS-Sequenzen und deren Verwendung zur Erzeugung von Proteinen, indem diese DNS-Sequenzen in ein Expressionssystem, zum Beispiel in einen Wirtsorganismus/eine Wirtszelle oder ein System für eine Invitro-Expression eingebracht werden, der bzw. die das entsprechende Protein exprimiert. Sie betrifft insbesondere Verfahren, bei denen eine synthetische Nucleotidsequenz für das jeweilige Expressionssystem, also zum Beispiel für einen Organismus/für eine Wirtszelle, mit Hilfe eines Computers optimiert wird.

Eine Technik zur Herstellung und Synthetisierung von Proteinen ist das Klonen und Exprimieren der dem Protein entsprechenden Gensequenz in heterologen Systemen, z.B. Escherichia coli oder Hefe. Natürlich vorkommende Gene sind für diesen Zweck allerdings häufig suboptimal. Da in einer DNS-Sequenz, die ein Protein exprimiert, jeweils ein Triplett von Basen (Codon) eine Aminosäure exprimiert, ist es möglich, eine künstliche DNS-Sequenz zur Expression des gewünschten Proteins zu synthetisieren und für das Klonen und Exprimieren des Proteins zu verwenden. Ein Problem bei diesem Vorgehen besteht darin, daß einer vorgegebenen Aminosäurensequenz keine eindeutige Nucleotidsequenz entspricht. Dies wird als Degeneriertheit des genetischen Codes bezeichnet. Unterschiedliche Organismen verwenden Codons für die Expression einer Aminosäure mit unterschiedlicher Häufigkeit (sogenannte Codon usage). In der Regel gibt es in einem gegebenen Organismus ein Codon, das überwiegend verwendet wird und ein oder mehrere Codons, welche mit vergleichsweise geringer Häufigkeit von dem Organismus zur Expression der entsprechenden Aminosäure verwendet werden. Da die synthetisierte Nucleotidsequenz in einem bestimmten Organismus verwendet werden soll, sollte die Wahl der Codons an die Codon usage des entsprechenden Organismus angepaßt sein. Eine weitere wichtige Größe ist der GC-Gehalt (Gehalt der Basen Guanin und Cytosin in einer Sequenz). Weitere Faktoren, welche das Expressionsergebnis beeinflussen können, sind DNS-Motive und Wiederholungen oder invers komplementäre Wiederholungen in der Basensequenz. Bestimmte Basenabfolgen erzeugen in einem gegebenen Organismus bestimmte Funktionen, die innerhalb einer codierenden Sequenz nicht erwünscht sein können. Beispiele sind cis-aktive Sequenzmotive wie Spleißstellen oder Transkriptionsterminatoren. Das unbeabsichtigte Vorhandensein eines bestimmten Motivs kann die Expression reduzieren oder ganz unterdrücken oder sogar für den Wirtsorganismus eine toxische Wirkung haben. Sequenzwiederholungen können zu einer geringeren genetischen Stabilität führen und erschweren die Synthese repetitiver Abschnitte aufgrund der Gefahr von Fehlhybridisierungen. Invers komplementäre Wiederholungen können zur Bildung von unerwünschten Sekundärstrukturen auf der RNA-Ebene oder cruciformer Strukturen auf DNS-Ebene führen, welche die Transkription behindern und zu genetischer Instabilität führen, bzw. die Translationseffizienz negativ beeinflussen können.

Ein synthetisches Gen sollte daher hinsichtlich der Codon usage und des GC-Gehalts optimiert sein und andererseits die mit DNS-Motiven sowie Sequenzwiederholungen und invers komplementären Sequenzwiederholungen verbundenen Probleme weitgehend vermeiden. Diese Erfordernisse lassen sich in der Regel jedoch nicht gleichzeitig und in optimaler Weise erfüllen. Beispielsweise kann eine Optimierung auf die optimale Codon usage zu einer stark repetitiven Sequenz und einem erheblichen Abweichen von dem gewünschten GC-Gehalt führen. Es gilt daher, einen möglichst optimalen Kompromiß zwischen der Erfüllung der verschiedenen Erfordernisse herbeizuführen. Die große Anzahl von Aminosäuren in einem Protein führt jedoch zu einer kombinatorischen Explosion der Zahl der möglichen DNS-Sequenzen, welche - im Prinzip - das gewünschte Protein exprimieren können. Aus diesem Grund wurden verschiedene computergestützte Verfahren zum Ermitteln einer optimalen Codonsequenz vorgeschlagen.

P.S. Sarkar und Samir K. Brahmachari, Nucleic Acids Research 20 (1992), 5713 beschreiben Untersuchungen zur Rolle der Wahl der Codons bei der Bildung bestimmter räumlicher Strukturen einer DNS-Sequenz. Hierbei wurden alle möglichen degenerierten Nucleotidsequenzen generiert. Eine Bewertung der Sequenzen hinsichtlich des Vorhandenseins von strukturellen Motiven und strukturbildender Abschnitte erfolgte durch einen Computer unter Verwendung einer Wissensbasis. Die Verwendung einer Gütefunktion ist nicht offenbart.

D.M. Hoover und J. Lubkowski, Nucleic Acid Research 30 (2002), Nr. 10 e43 schlägt ein computergestütztes Verfahren vor, bei dem die Nucleotidsequenz in eine ungerade Anzahl von Abschnitten unterteilt wird, für die jeweils eine Gütefunktion (Score) berechnet wird. In die Gütefunktion gehen u.a. die Codon usage, die Möglichkeit der Bildung von Haarnadelstrukturen und die Abweichungen von der gewünschten Schmelztemperatur ein. Der Wert der Gütefunktion für die Gesamtsequenz bestimmt sich aus der Summe der Werte der Gütefunktion für die einzelnen Abschnitte. Die Besetzung mit Codons innerhalb eines Abschnittes wird durch ein sogenanntes Monte-Carlo-Verfahren optimiert. Dabei werden statistisch Codonpositionen ausgewählt, bei denen das Codon einer Ausgangssequenz durch ein statistisch ausgewähltes äquivalentes Codon ersetzt wird. Gleichzeitig werden in einer Iteration auch die Grenzen der Abschnitte neu definiert. Auf diese Weise wird eine vollständige Gensequenz statistisch generiert. Ist der Wert der Gütefunktion für die Gesamtsequenz kleiner als die bisherige Sequenz, wird die neue Sequenz beibehalten. Ist er größer, wird mit einer gewissen Wahrscheinlichkeit die neue Sequenz beibehalten, wobei diese Wahrscheinlichkeit durch eine Boltzmann-Statistik kontrolliert wird. Wenn sich innerhalb einer vorbestimmten Anzahl von Iterationen die Sequenz nicht ändert, wird diese Sequenz als optimale Sequenz bewertet.

Derartige statistische Verfahren haben den Nachteil, daß sie stark von der Wahl der Konvergenzkriterien abhängen.

Es ist die Aufgabe der Erfindung, ein alternatives Verfahren zum Optimieren einer Nucleotidsequenz zur Expression eines Proteins auf der Grundlage der Aminosäuresequenz des Proteins zur Verfügung zu stellen, welches sich mit relativ geringem Speicherplatz und relativ geringer Rechenzeit auf einem Computer implementieren läßt und welches insbesondere Nachteile der statistischen Verfahren vermeidet.

Erfindungsgemäß wird diese Aufgabe durch ein Verfahren zum Optimieren einer Nucleotidsequenz zur Maximierung der Expression eines Proteins in einem vorgegebenen Organismus auf der Grundlage der Aminosäuresequenz des Proteins gelöst, welches die folgenden auf einem Computer durchgeführten Schritte umfaßt:

- Generieren einer ersten Testsequenz von n Codons, welche n aufeinanderfolgenden Aminosäuren in der Proteinsequenz entsprechen, wobei n eine natürliche Zahl und kleiner oder gleich N, der Zahl der Aminosäuren der Proteinsequenz, ist,
- Festlegen von m Optimierungspositionen in der Testsequenz, welche der Position von m Codons, insbesondere von m aufeinanderfolgenden Codons, entsprechen, an denen die Besetzung mit einem Codon, bezogen auf die Testsequenz, optimiert werden soll, wobei $m \leq n$ und $m < N$ ist,
- Generieren einer oder mehrerer weiterer Testsequenzen aus der ersten Testsequenz, indem an einer oder mehreren der m Optimierungspositionen ein Codon der ersten Testsequenz durch ein anderes Codon ersetzt wird, welches dieselbe Aminosäure exprimiert,
- Bewerten jeder der Testsequenzen mit einer Gütefunktion und Ermitteln der hinsichtlich der Gütefunktion optimalen Testsequenz,
- Festlegen von p Codons der optimalen Testsequenz, welche sich an einer der m Optimierungspositionen befinden, als Ergebniscodons, welche die Codons der optimierten Nucleotidsequenz an den Positionen bilden, die der Position der besagten p Codons in der Testsequenz entspricht, wobei p eine natürliche Zahl und $p \leq m$ und in einem oder mehreren Iterationsschritten p gleich Eins ist,
- Iterieren der vorangehenden Schritte, wobei in jedem Iterationsschritt die Testsequenz an den Positionen, welche Positionen von festgelegten Ergebniscodons in der optimierten Nucleotidsequenz entsprechen, das entsprechende Ergebniscodon enthält und die Optimierungspositionen von Positionen von Ergebniscodons verschieden sind.

Gemäß der bevorzugten Ausführungsform der Erfindung werden die vorangehend genannten Schritte so oft iteriert, bis alle Codons der optimierten Nucleotidsequenz festgelegt, d.h. mit Ergebniscodons besetzt worden sind.

Erfindungsgemäß wird also die Sequenz nicht insgesamt, sondern sukzessiv auf Teilbereichen optimiert. Die in einem Iterationsschritt als optimal festgelegten p Ergebniscodons werden in den nachfolgenden Iterationsschritten nicht mehr verändert und vielmehr bei den jeweiligen Optimierungsschritten als gegeben vorausgesetzt. Vorzugsweise ist die Anzahl der Ergebniscodons, welche auf diese Weise für die weiteren Iterationen festgelegt und als vorgegeben behandelt werden, kleiner als die Anzahl m der Optimierungspositionen, an denen in einem Iterationsschritt die Codons variiert werden. Zumindest in der Mehrzahl der Iterationsschritte, bei einer besonderen Ausführungsform bei allen Iterationsschritten außer dem ersten, ist wiederum m kleiner als die Zahl der Codons der Testsequenz (n). Dies gestattet es, nicht nur lokale Effekte auf den m variierten Positionen, sondern auch längerreichweitige Korrelationen, z.B. im Zusammenhang mit der Entstehung von RNA-Sekundärstrukturen, zu berücksichtigen.

Gemäß den derzeit bevorzugten Ausführungsformen liegt m im Bereich von 3 bis 20, vorzugsweise im Bereich von 5 bis 10. Bei dieser Wahl dieses Parameters kann die Variation der Codons mit einem akzeptablen Aufwand an Speicher und Rechenzeit durchgeführt werden und gleichzeitig eine gute Optimierung der Sequenz erreicht werden.

Gemäß einer Ausführungsform muß m in den verschiedenen Iterationsschritten nicht gleich sein, sondern kann vielmehr auch in unterschiedlichen Iterationsschritten verschieden sein. Es kann auch vorgesehen sein, in einem Iterationsschritt die Variation der Testsequenz für verschiedene Werte von m durchzuführen und ggf. nur das Optimierungsergebnis für einen Wert von m zu berücksichtigen, um Einflüsse der Größe m auf das Optimierungsergebnis zu reduzieren bzw. um zu überprüfen, ob eine Vergrößerung der Zahl m zu einer Änderung des Ergebnisses führt.

Gemäß der bevorzugten Ausführungsform sind die m Optimierungspositionen oder zumindest ein Teil davon zusammenhängend und bilden somit ein Variationsfenster in der Testsequenz, auf welchem die Codonbesetzung variiert wird.

Die Erfindung kann insbesondere vorsehen, daß in zwei oder mehr aufeinanderfolgenden Iterationsschritten ein Teil der m Optimierungpositionen, auf welchen die Codons variiert werden, identisch sind. Sind die m Positionen zusammenhängend, bedeutet dies, daß das Variationsfenster bei einem Iterationsschritt mit dem Variationsfenster eines vorangehenden Iterationsschrittes überlappt.

Die Erfindung kann vorsehen, daß in einem oder mehreren Iterationsschritten die m Optimierungspositionen der Testsequenzen unmittelbar auf ein oder mehrere Ergebniscodons folgen, welche als Teil der optimierten Nucleotidsequenz festgelegt worden sind.

Die Erfindung kann ebenfalls vorsehen, daß in einem oder mehreren Iterationsschritten die p Codons, die als Ergebniscodons der optimierten Nucleotidsequenz festgelegt werden, p aufeinanderfolgende Codons sind, die vorzugsweise unmittelbar auf ein oder mehrere Ergebniscodons folgen, welche als Teil der optimierten Nucleotidsequenz in einem früheren Schritt festgelegt worden sind.

Die Erfindung kann vorsehen, daß die Nucleotidsequenz von einem ihrer Enden her optimiert wird. Insbesondere kann die Erfindung vorsehen, daß in jedem Iterationsschritt die Länge der Testsequenz des vorherigen Iterationsschritts um eine bestimmte Anzahl Codons, die in unterschiedlichen Iterationen verschieden sein kann, vergrößert wird, bis n = N ist. Ist n = N und die Zahl derjenigen Positionen, die in der Testsequenz nicht mit Ergebniscodons besetzt sind, kleiner oder gleich dem Wert von m, der in den vorangehenden Iterationen verwendet wurde, oder liegt diese Zahl, bei Verwendung unterschiedlicher Werte von m in verschiedenen Iterationen, im Bereich der in Frage kommenden Werte von m, kann in dem entsprechenden Iterationsschritt p = m gesetzt werden, wobei m gleichzeitig die Zahl der noch nicht festgelegten Codons ist. Die als optimal aufgefundene Besetzung der Optimierungspositionen wird dann für die Ergebniscodons an diesen Optimierungspositionen übernommen. Dies gilt insbesondere dann, wenn für jede mögliche Kombination von Besetzungen der Optimierungspositionen eine Testsequenz generiert wird.

Es kann jedoch auch vorgesehen sein, daß der Bereich der Testsequenz innerhalb der gesamten Sequenz in einem Iterationsschritt nicht oder nicht vollständig den Bereich einer Testsequenz in einem vorherigen Iterationsschritt umfaßt. Beispielsweise kann die Testsequenz selbst ein Fenster auf der Gesamtsequenz, z.B. ein Fenster fester Länge, bilden, das im Laufe der verschiedenen Iterationen auf der Gesamtsequenz verschoben wird.

Gemäß einer bevorzugten Ausführungsform wird die Testsequenz nach jedem Schritt um p Codons verlängert, wobei insbesondere m für alle Iterationsschritte konstant sein kann.

Analog zu der vorangehend beschriebenen Ausführungsform der Erfindung kann auch vorgesehen sein, daß die Nucleotidsequenz von einer Stelle in ihrem Inneren her optimiert wird. Dies kann z.B. in der Art geschehen, daß eine anfängliche Testsequenz, welche einem Bereich im Inneren der zu optimierenden Nucleotidsequenz entspricht, zunächst nach einer Seite sukzessiv vergrößert wird, bis das Ende der zu optimierenden Nucleotidsequenz oder ein anderer vorgegebener Punkt der zu optimierenden Nucleotidsequenz erreicht ist, und dann die Testsequenz zu der anderen Seite hin vergrößert wird, bis dort das andere Ende der zu optimierenden Nucleotidsequenz oder ein anderer vorgegebener Punkt der zu optimierenden Nucleotidsequenz erreicht ist.

Die Erfindung kann auch vorsehen, daß die Testsequenzen in einem Iterationsschritt aus einer optimierten oder anderweitig festgelegten Teilsequenz der Länge q und zwei auf beiden Seiten daran anschließenden Variationsbereichen mit einer Länge von $m_1$ bzw. $m_2$ Codons besteht, wobei $q+m_1+m_2 = n$ gilt. Die Besetzung der Variationsbereiche kann für beide Variationsbereiche gemeinsam optimiert werden, indem die Codons auf den $m_1$ und $m_2$ Plätzen gleichzeitig variiert und optimiert werden. Vorzugsweise werden in einem solchen Fall in jedem Iterationsschritt $p_1$ und $p_2$ Codons in dem ersten und zweiten Variationsbereich festgelegt, welche der weiteren Iteration als gegeben zugrunde gelegt werden. Es kann jedoch auch vorgesehen sein, daß die beiden Variationsbereiche unabhängig voneinander variiert und optimiert werden. Beispielsweise kann vorgesehen sein, daß die Besetzung nur in einem der beiden Variationsbereiche variiert wird und nur in dem einen Bereich Codons festgelegt werden, bevor die Variation und Optimierung in den zweiten Bereich stattfindet. In diesem Fall werden die $p_1$ festgelegten Codons in dem ersten Bereich bei der Optimierung des zweiten Bereichs als gegeben vorausgesetzt. Dieses Vorgehen ist dann sinnvoll, wenn allenfalls geringe Korrelationen zwischen den beiden Bereichen zu erwarten sind.

Gemäß dieser Ausführungsform kann vorgesehen sein, daß die Nucleotidsequenz von einem Punkt oder einem Bereich im Inneren der Sequenz ausgehend optimiert wird.

Die Erfindung kann insbesondere vorsehen, daß in jedem Iterationsschritt der Bereich der Testsequenz auf der Gesamtsequenz den Bereich der Testsequenzen in allen vorangehenden Iterationsschritten umfaßt und der Bereich einer Testsequenz in zumindest einigen der vorangehenden Iterationsschritte jeweils im Inneren oder jeweils am Rand des Bereichs der Testsequenz in dem aktuellen Iterationsschritt liegt.

Die Erfindung kann vorsehen, daß die Nucleotidsequenz auf verschiedenen Teilbereichen unabhängig optimiert wird. Die optimierte Nucleotidsequenz kann dann die Kombination der verschiedenen optimierten Teilsequenzen sein. Es kann auch vorgesehen sein, daß zumindest ein Teil der jeweiligen Ergebniscodons von zwei oder mehr optimierten Teilbereichen als Bestandteil einer Testsequenz in einer oder mehreren Iterationen verwendet wird.

Gemäß einer bevorzugten Ausführungsform der Erfindung ist vorgesehen, daß in einem Iterationsschritt Testsequenzen mit allen möglichen Codonbesetzungen für die m Optimierungspositionen aus der ersten Testsequenz generiert werden und die optimale Testsequenz unter allen möglichen Testsequenzen, bei denen an einer oder mehreren der m Optimierungspositionen ein Codon durch ein anderes Codon, welches dieselbe Aminosäure exprimiert, ersetzt wurde, ermittelt wird.

Gemäß einer Ausführungsform der Erfindung ist die zum Bewerten der Testsequenzen verwendete Gütefunktion bei allen oder zumindest der Mehrzahl der Iterationen gleich. Die Erfindung kann jedoch auch vorsehen, unterschiedliche Gütefunktionen in unterschiedlichen Iterationen, zum Beispiel in Abhängigkeit von der Länge der Testsequenzen, zu verwenden.

Das erfindungsgemäße Verfahren kann insbesondere die folgenden Schritte umfassen:

- Bewerten jeder Testsequenz mit einer Gütefunktion,
- Ermitteln eines Extremwertes innerhalb der Werte der Gütefunktion für alle in einem Iterationsschritt generierten Teilsequenzen,
- Festlegen von p Codons der Testsequenz, welche dem extremalen Wert der Gewichtsfunktion entspricht, als Ergebniscodons an den entsprechenden Positionen, wobei p eine natürliche Zahl und $p \leq m$ ist.

Die Gütefunktion kann so definiert sein, daß die Sequenz entweder umso näher an dem Optimum liegt, je größer der Wert der Gütefunktion ist, oder umso näher an dem Optimum liegt, je kleiner ihr Wert ist. Entsprechend wird man bei dem Schritt des Ermittelns des Extremwertes das Minimum oder das Maximum der Gütefunktion unter den generierten Codonsequenzen ermitteln.

Die Erfindung kann vorsehen, daß die Gütefunktion eines oder mehrere der folgenden Kriterien berücksichtigt: Codon usage für einen vorgegebenen Organismus, GC-Gehalt, Sequenzmotive, repetitive Sequenzen, Sekundärstrukturen, inverse Repeats.

Die Erfindung kann insbesondere vorsehen, daß die Gütefunktion eines oder mehrere der folgenden Kriterien berücksichtigt:

- cis-aktive Sequenz-Motive, insbesondere DNS/Protein-Interaktionsbindestellen und RNS/Protein-Interaktionsbindestellen, bevorzugt Spleißmotive, Transkriptionsfaktorbindestellen, Transkriptionsterminatorenbindestellen, Polyadenylierungssignale, Endonucleaseerkennungssequenzen, immunomodulatorische DNS-Motive, Ribosomenbindestellen, Erkennungssequenzen für rekombinationsaktive Enzyme, Erkennungssequenzen für DNS-modifizierende Enzyme, Erkennungssequenzen für RNSmodifizierende Enzyme, Sequenzmotive, die in einem vorgegebenen Organismus unterrepräsentiert sind.

Die Erfindung kann auch vorsehen, daß die Gütefunktion eines oder mehrere der folgenden Kriterien berücksichtigt:

- Ausschluß oder weitgehender Ausschluß von invers komplementären Sequenzidentitäten von mehr als 20 Nukleotiden zum Transkriptom eines vorgegebenen Organismus,
- Ausschluß oder weitgehender Ausschluß von Homologiebereichen von mehr als 1.000 Basenpaaren, bevorzugt 500 Basenpaaren, stärker bevorzugt 100 Basenpaaren zu einer vorgegebenen DNS-Sequenz, zum Beispiel zu dem Genom eines vorgegebenen Organismus oder zu der DNS-Sequenz eines vorgegebenen Vektorkonstrukts.

Das erste dieser beiden Kriterien betrifft den Ausschluß des als RNA-Indifferenz bekannten Mechanismus, mit dem ein Organismus RNA-Sequenzen mit mehr als 20 Nukleotiden exakter Identität zu einer anderen RNA-Sequenz eliminiert oder deaktiviert. Mit dem zweiten Kriterium soll verhindert werden, daß eine Rekombination, das heißt ein Einbau der Sequenz in das Erbgut des Organismus, oder eine Mobilisierung von DNS-Sequenzen durch Rekombination mit anderen Vektoren stattfindet. Beide Kriterien können als absolute Ausschlußkriterien verwendet werden, d.h. Sequenzen, bei denen eines oder beide dieser Kriterien erfüllt sind, werden nicht berücksichtigt. Die Erfindung kann auch, wie nachfolgend noch genauer im Zusammenhang mit Sequenzmotiven erläutert wird, vorsehen, daß diesen Kriterien ein Gewicht zugeordnet ist, das betragsmäßig größer ist als der größte Beitrag von Kriterien zu der Gütefunktion, welche keine Ausschlußkriterien sind.

Die Erfindung kann auch, gegebenenfalls zusammen mit anderen Kriterien, das Kriterium vorsehen, daß keine Homologiebereiche erzeugt werden, die mehr als 90 % Ähnlichkeit und/oder 99 % Identität zu einer vorgegebenen DNS-Sequenz, zum Beispiel zu der entsprechenden Genomsequenz des vorgegebenen Organismus oder zu der DNS-Sequenz eines vorgegebenen Vektorkonstrukts aufweisen. Auch dieses Kriterium kann entweder als absolutes Ausschlußkriterium realisiert sein oder in einer Weise, daß es einen sehr großen Beitrag zu der Gütefunktion leistet, welcher den Beitrag anderer Kriterien, die nicht Ausschlußkriterien sind, überwiegt.

Insbesondere kann vorgesehen sein, daß die Gütefunktion eine Funktion von verschiedenen Einzeltermen, insbesondere eine Summe von Einzeltermen ist, die jeweils ein Kriterium aus der folgenden Liste von Kriterien bewerten: Codon usage für einen vorgegebenen Organismus, GC-Gehalt, DNS - Motive, repetitive Sequenzen, Sekundärstrukturen, inverse Repeats.

Die besagte Funktion von Einzeltermen kann insbesondere eine Linearkombination von Einzeltermen oder eine rationale Funktion von Einzeltermen sein.Die genannten Kriterien müssen nicht notwendigerweise vollständig in der Gewichtsfunktion berücksichtigt werden. Es kann auch nur ein Teil der Kriterien in der Gewichtsfunktion verwendet werden.

Die verschiedenen Einzelterme in der besagten Funktion werden nachfolgend Kriteriumsgewichte genannt.

Die Erfindung kann vorsehen, daß das Kriteriumsgewicht betreffend die Codon Usage (CU Score) proportional zu $\Sigma_i f_{ci}/f_{cmaxi}$ ist, wobei

- $f_{ci}$ die Häufigkeit des an der Stelle i der Testsequenz gesetzten Codons für den betreffenden Organismus zur Expression der Aminosäure an der Stelle i der Aminosäurensequenz des zu exprimierenden Proteins ist und
- $f_{cmaxi}$ die Häufigkeit des Codons ist, welches in dem entsprechenden Organismus am häufigsten die Aminosäure an der Stelle i exprimiert.

Das Maß $f_{ci}/f_{cmaxi}$ ist als "Relative Adaptiveness" bekannt (vgl. P. M. Sharp, W. H. Li, Nucleic Acids Research 15 (3) (1987), 1281 bis 1295).

Das lokale Gewicht des am häufigsten vorkommenden Codons wird dabei, unabhängig von der absoluten Häufigkeit, mit der dieses Codon vorkommt, auf einen bestimmten Wert, zum Beispiel 1, gesetzt. Damit wird vermieden, daß die Positionen, an denen nur wenige Codons zur Auswahl stehen, stärker zu dem Gesamtgewicht beitragen als diejenigen, an denen eine größere Anzahl von Codons zur Expression der Aminosäure zur Auswahl stehen. Der Index i kann über die gesamten n Codons der Testsequenz oder einen Teil davon laufen. Insbesondere kann in einer Ausführungsform vorgesehen sein, daß i nur über die m Codons der Optimierungspositionen läuft.

Die Erfindung kann vorsehen, daß das Kriteriumsgewicht betreffend die Codonusage nur für die m Ordnungspositionen verwendet wird.

Anstelle der Relative Adaptiveness kann auch die sogenannte RSCU (Relative Synonymous Codon Usage; vgl. P. M. Sharp, W. H. Li, a.a.O.) verwendet werden. Die RSCU für eine Codonposition ist definiert durch

$$RSCU_{ci} = f_{ci}d_i/(\textstyle\sum_c f_{ci})$$

definiert, wobei die Summe im Nenner über alle Codons läuft, welche die Aminosäure an der Stelle i exprimieren und wobei $d_i$ die Zahl der Codons angibt, welche die besagte Aminosäure exprimieren. Um ein Kriteriengewicht auf der Grundlage der RSCU zu definieren, kann vorgesehen sein, daß die RSCU für die jeweilige Testsequenz über alle Codons der Testsequenz oder einen Teil davon, insbesondere über die m-Codons der Optimierungspositionen, summiert wird. Der Unterschied zu dem von der Relative Adaptiveness abgeleiteten Kriteriumsgewicht besteht darin, daß bei dieser Gewichtung jede Codonposition mit dem Grad der Degeneriertheit, $d_i$, gewichtet wird, so daß solche Positionen, an denen mehr Codons zur Auswahl stehen, stärker in das Kriteriumsgewicht eingehen als solche Positionen, an denen nur wenige Codons oder sogar nur ein einziges Codon zur Auswahl stehen.

Bei den vorangehend beschriebenen Kriteriumsgewichten für die Codon-Usage wurde das arithmetische Mittel über die lokalen Gewichte (Relative Adaptiveness, RSCU) gebildet.

Es kann auch vorgesehen sein, daß das Kriteriumsgewicht betreffend die Codon-Usage proportional zu den geometrischen Mittel der lokalen Relative Adaptiveness bzw. der lokalen RSCU ist, so daß also gilt

$$CUScore = K(\Pi_i\, RSCU_i)^{1/L}$$

oder

$$CUScore = K\, (\Pi_i f_{ci}/f_{cmaxi})^{1/L}$$

ist, wobei K ein Skalierungsfaktor ist und L die Anzahl der Positionen ist, über welche das Produkt gebildet wird. Auch hier kann das Produkt wieder über die gesamte Testsequenz oder einen Teil, insbesondere über die m Optimierungspositionen, gebildet werden.

In diesem Zusammenhang stellt ein Beispiel auch ein Verfahren zum Optimieren einer Nukleotidsequenz zur Expression eines Proteins auf der Grundlage der Aminosäuresequenz des Proteins zur Verfügung, welches die folgenden auf einem Computer durchgeführten Schritte umfaßt

- Generieren einer oder mehrerer Testsequenzen von n Codons, welche n aufeinanderfolgende Aminosäuren in der Proteinsequenz entsprechen, wobei n eine natürlich Zahl kleiner oder gleich N, der Zahl der Aminosäuren der Proteinsequenz, ist,
- Bewerten der einen oder mehreren Testsequenzen auf der Grundlage einer Gütefunktion, welche ein geometrisches oder arithmetisches Mittel der Relative Adaptiveness oder der RSCU über eine Anzahl von L Codonpositionen enthält, wobei L kleiner oder gleich N ist,
- Generierung einer oder mehrerer neuer Testsequenzen in Abhängigkeit von dem Ergebnis der besagten Bewertung.

Dabei kann die Generierung einer oder mehrerer neuer Testfunktionen in der oben beschriebenen Weise derart erfolgen, daß die neuen Testsequenzen eine bestimmte Anzahl aufgrund der vorangehenden Iterationen festgelegte Ergebniscodons enthalten, aber z.B. auch so, daß eine bestimmte Testsequenz mit einer bestimmten Wahrscheinlichkeit, die von dem Wert der Gütefunktion abhängt, als Grundlage für weitere Iterationen, insbesondere die weitere Erzeugung von Testsequenzen, verwendet wird, wie dies bei Monte-Carlo-Verfahren der Fall ist.

Während die Qualität eines Codons bei den obengenannten Verfahren durch die Nutzungshäufigkeit im Transkriptom oder einem Gen-Referenzset des Expressionsorganismus definiert wird, kann die Güte eines bestimmten Codons alternativ auch durch die biophysikalischen Eigenschaften des Codons selbst beschrieben werden. So ist zum Beispiel bekannt, daß Codons mit einer mittleren Codon-Anticodon-Bindungsenergie besonders effizient translatiert werden. Als Maß für die translatorische Effizienz einer Testsequenz kann daher zum Beispiel der P2-Index verwendet werden, welcher das Verhältnis der Häufigkeit von Codons mit mittlerer Bindungsenergie und Codons mit extrem starker bzw. schwacher Bindungsenergie angibt. Alternativ können auch experimentell oder durch theoretische Berechnungen gewonnene Daten zur translatorischen Effizienz oder translationsgenauigkeit eines Codons zur Gütebewertung genutzt werden. Die oben genannten Bewertungskriterien können besonders dann von Vorteil sein, wenn die tRNA-Frequenzen des Expressionssystems nicht berücksichtigt werden müssen, da diese wie zum Beispiel bei in Vitro-Translationssystemen vom Experimentator festgelegt werden können.

Die Erfindung kann vorsehen, daß das Kriteriumsgewicht betreffend den GC-Gehalt (GCScore) eine Funktion des Betrags der Differenz des ermittelten GC-Gehalts der Teilsequenz, GCG, zu dem optimalen GC-Gehalt, $GCG_{opt}$ ist, wobei unter dem GG-Gehalt der relative Anteil von Guanin und Cytosin, zum Beispiel in Form eines bestimmten prozentualen Anteils, zu verstehen ist.

Insbesondere kann das Kriteriumsgewicht GCScore die folgende Form haben:

$$GCScore = \left|\overline{GCG} - GCG_{opt}\right|^{g} \cdot h$$

wobei

$\overline{GCG}$ der tatsächliche GC - Gehalt der Testsequenz oder eines vorbestimmten Teils der Testsequenz, GCG, oder der mittlere GC - Gehalt der Testsequenz oder eines vorbestimmten Teils der Testsequenz, <GCG>, ist,
$GCG_{opt}$ der gewünschte (optimale) GC - Gehalt ist,
g eine positive reelle Zahl, vorzugsweise im Bereich von 1 bis 3 , insbesondere 1,3 ist, h eine positive reelle Zahl ist.

Der Faktor h ist im wesentlichen ein Gewichtungsfaktor, welcher das relative Gewicht des Kriteriumsgewichts GCScore gegenüber den anderen Kriteriumsgewichten definiert. Vorzugsweise wird h so gewählt, daß der Betrag des maximal erreichbaren Wertes von GCScore in einem Bereich von einem Hundertstel bis zu dem Hundertfachen eines anderen Kriteriumsgewichtes, insbesondere aller Kriteriumsgewichte, welche keine Ausschlußbedingung darstellen, wie zum Beispiel die Gewichte für ein erwünschtes bzw. unerwünschtes Sequenzmotiv, beträgt.

Zur Bestimmung des mittleren GC-Gehalts kann vorgesehen sein, daß ein auf eine bestimmte Basenposition bezogener lokaler GC-Gehalt durch den GC-Gehalt auf einem Fenster bestimmter Größe definiert wird, welches diese Base enthält und welches insbesondere bezüglich dieser Base zentriert sein kann. Dieser lokale GC-Gehalt wird dann über die Testsequenz oder einen Teilbereich der Testsequenz, insbesondere über die m Optimierungspositionen, gemittelt, wobei auch hier sowohl ein arithmetisches als auch ein geometrisches Mittel verwendet werden kann. Verwendet man einen auf diese Weise definierten mittleren GC-Gehalt, ergeben sich geringere Schwankungen zwischen Testsequenzen mit einer verschiedenen Längen.

Die Erfindung kann vorsehen, daß der GC-Gehalt über einem Fenster ermittelt wird, welches größer als der Bereich der m Optimierungspositionen ist und diesen einschließt. Wenn die Optimierungspositionen ein zusammenhängendes Variationsfenster bilden, kann vorgesehen sein, daß b Basen vor und/oder nach dem Variationsfenster in die Bestimmung des Kriteriumsgewichts für den GC-Gehalt (GCScore) einbezogen werden, wobei b in einem Bereich von 15 bis 45 Basen (entspricht 5 bis 15 Codons), vorzugsweise in einem Bereich von 20 bis 30 Basen liegen kann.

Die Erfindung kann weiterhin vorsehen, daß, soweit die Gütefunktion maximiert wird, bei der Ermittlung des Werts der Gütefunktion für jedes Vorkommen eines nicht erlaubten oder unerwünschten Sequenzmotivs ein fester Betrag abgezogen und für jedes erwünschte oder geforderte Motiv ein fester Betrag addiert wird (bei einer Minimierung der Gütefunktion verhält es sich umgekehrt). Bei unerwünschten oder geforderten Motiven kann dieser Betrag deutlich größer sein als alle anderen Kriteriumsgewichte, so daß die anderen Kriterien demgegenüber nicht ins Gewicht fallen. Dadurch wird ein Ausschlußkriterium realisiert, während gleichzeitig eine Differenzierung danach stattfindet, ob ein Motiv einmal oder mehrfach aufgetreten ist. Ebenso läßt sich jedoch auch dann noch eine sinnvolle Gütefunktion definieren bzw. eine Bewertung der Testsequenzen mit der Gütefunktion durchführen, wenn die Bedingung hinsichtlich des Sequenzmotivs (Nichtvorhandensein eines bestimmten Motivs/Vorhandensein eines bestimmten Motivs) für alle in einem Iterationsschritt erzeugten Testsequenzen nicht erfüllt werden kann. Dies wird insbesondere dann der Fall sein, wenn die Länge n der Testsequenzen relativ klein gegenüber N ist, da aufgrund der vorgegebenen Aminosäuren der Proteinsequenz ein bestimmtes Motiv häufig erst bei größeren n auftreten kann.

Die Erfindung kann weiterhin vorsehen, daß die gesamte Testsequenz oder ein Teil davon daraufhin überprüft wird, ob bestimmte partielle Sequenzabschnitte oder zu bestimmten partiellen Sequenzabschnitten ähnliche Sequenzabschnitte in einem anderen Bereich der Testsequenz oder eines gegebenen Bereichs der Testsequenz auftreten oder ob bestimmte partielle Sequenzabschnitte oder zu bestimmten partiellen Sequenzabschnitten ähnliche Sequenzabschnitte in der invers komplementären Testsequenz oder eines Teils der invers komplementären Testsequenz vorkommen, und in Abhängigkeit hiervon ein Kriteriumsgewicht für Sequenzwiederholungen (repeats) und/oder inverse Sequenzwiederholungen (inverse repeats) berechnet wird. Im Regelfall wird dabei die Sequenz nicht nur darauf überprüft, ob ein bestimmter Sequenzabschnitt identisch in der Testsequenz bzw. der invers-komplementären Testsequenz bzw. eines Teilbereichs davon enthalten ist, sondern auch darauf, ob eine ähnliche, also nur teilweise übereinstimmende Sequenz in der Testsequenz bzw. der invers-komplementären Testsequenz bzw. eines Teils davon enthalten ist. Algorithmen zum Auffinden von globalen Übereinstimmungen (Global-Alignment-Algorithmen) oder lokalen Übereinstimmungen (Local Alignment-Algorithmen) zweier Sequenzen sind in der Bioinformatik allgemein bekannt. Zu den geeigneten Verfahren zählen beispielsweise die in der Bioinformatik allgemein bekannten Dynamic Programming - Algorithmen, z.B. der sogenannte Needleman-Wunsch-Algorithmus für globales Alignment und der Smith-Waterman-Algorithmus für lokales Alignment. Insoweit wird beispielsweise auf Michael S. Waterman, Introduction to Computational Biology, London, New York 2000, insbesondere S. 207 bis 209 oder Dan Gusfield, Algorithms on Strings, Trees and Sequences, Cambridge, 1999, insbesondere S. 215 bis 235, verwiesen.

Die Erfindung kann insbesondere vorsehen, daß jede Wiederholung eines partiellen Sequenzabschnittes in einem anderen Teil der Testsequenz oder eines vorgegebenen Bereichs der Testsequenz mit einem bestimmten Gewicht gewichtet wird, welches ein Maß für den Grad der Übereinstimmung und/oder die Größe der zueinander ähnlichen Abschnitte darstellt, und daß die Gewichte der einzelnen Wiederholungen zur Ermittlung des Kriteriumsgewichts betreffend die Wiederholungen bzw. invers komplementären Wiederholungen addiert werden. Es kann ebenfalls vorgesehen sein, daß die Gewichte der einzelnen Wiederholungen mit einem vorgegebenen Exponenten, dessen Wert vorzugsweise zwischen 1 und 2 liegt, potenziert werden und anschließend die Summation zur Ermittlung des Kriteriumsgewichts betreffend die Wiederholungen bzw. invers komplementäre Wiederholungen durchgeführt wird. Dabei kann vorgesehen sein, daß Wiederholungen unterhalb einer bestimmten Länge und/oder Wiederholungen, deren Gewichtsanteil unterhalb einer gewissen Schwelle liegt, nicht berücksichtigt werden. Die Erfindung kann vorsehen, daß zur Berechnung des entsprechenden Kriteriumsgewichts nur die Wiederholungen oder invers komplementären Wiederholungen eines partiellen Sequenzabschnitts berücksichtigt werden, der in einem vorgegebenen Teilbereich der Testsequenz (Testbereich), z.B. an dessen Ende und/oder in einem Variationsfenster liegt. Beispielsweise kann vorgesehen sein, daß nur die letzten 36 Basen der Testsequenz daraufhin überprüft werden, ob ein bestimmter Sequenzabschnitt innerhalb dieser 36 Basen mit einem anderen Sequenzabschnitt der gesamten Testsequenz oder der gesamten invers komplementären Testsequenz übereinstimmt.

Die Erfindung kann vorsehen, daß bei den Kriteriumsgewichten betreffend Wiederholungen, invers komplementäre Wiederholungen und/oder DNS-Motive nur der oder die M Abschnitte der Testsequenz berücksichtigt werden, welche den größten bzw. betragsmäßig größter Beitrag zu dem Kriteriumsgewicht liefern, wobei M eine natürliche Zahl, vorzugsweise zwischen 1 und 10, ist.

Gemäß einer Ausführungsform der Erfindung kann vorgesehen sein, daß eine Matrix generiert wird, deren Spaltenzahl der Anzahl der Positionen des Bereichs der Testsequenz (Testbereich) entspricht, der auf Wiederholungen in anderen Bereichen überprüft werden soll, und dessen Zeilenzahl der Anzahl der Positionen des Bereichs der Testsequenz entspricht, mit dem verglichen werden soll (Vergleichsbereich). Sowohl der Testbereich als auch der Vergleichsbereich können die gesamte Testsequenz umfassen.

Die Erfindung kann weiterhin vorsehen, daß die gesamte Gewichtsfunktion GesScore sich wie folgt bestimmt:

$$\text{GesScore} = \text{CUScore} - \text{GCScore} - \text{REPScore} - \text{SiteScore},$$

wobei CUScore das Kriteriumsgewicht für die Codon Usage ist, GCScore das Kriteriumsgewicht für den GC-Gehalt ist, REPScore das Kriteriumsgewicht für Wiederholungen und invers komplementäre Wiederholungen von gleichen oder ähnlichen Sequenzabschnitten ist und SiteScore das Kriteriumsgewicht für das Auftreten von unerwünschten bzw. geforderten Motiven ist.

Das Gewicht REPScore kann gemäß einer Ausführungsform der Erfindung aus einer Summe von zwei Bestandteilen bestehen, von denen der erste das Kriteriumsgewicht für die Wiederholung von gleichen oder ähnlichen Sequenzabschnitten in der Testsequenz selbst bzw. eines Teilbereichs davon angibt und der zweite Bestandteil das Kriteriumsgewicht für invers komplementäre Wiederholungen von gleichen oder ähnlichen Sequenzabschnitten in der Testsequenz oder eines Teilbereichs davon angibt.

Wenn die Gütefunktion sich aus Anteilen mehrerer Testkriterien zusammensetzt, insbesondere dann, wenn die Gütefunktion aus einer Linearkombination von Kriteriumsgewichten besteht, muß in einem Iterationsschritt eine Testsequenz nicht notwendigerweise nach allen Kriterien bewertet werden. Vielmehr kann die Bewertung bereits dann abgebrochen werden, wenn absehbar ist, daß der Wert der Gütefunktion geringer oder, allgemeiner gesprochen, weniger optimal, als der Wert der Gütefunktion einer bereits bewerteten Testsequenz ist. Bei den vorangehend beschriebenen Ausführungsformen gehen die meisten Kriterien, wie die Kriteriumsgewichte für repetitive Elemente, auszuschließende Motive usw., negativ in die Gütefunktion ein. Wenn nach Berechnung der Kriteriumsgewichte, welche positiv in die Gütefunktion eingehen und ggf. einem Teil der Kriteriumsgewichte, welche negativ in die Gütefunktion eingehen, sich bei der Aufsummation entsprechend der durch die Gütefunktion definierten Linearkombination der entsprechenden bereits berechneten Kriteriumsgewichte einen Wert ergibt, der kleiner ist als ein bereits berechneter Wert der vollständigen Gütefunktion für eine andere Testsequenz, kann die aktuell bewertete Testsequenz bereits ausgeschieden werden. Ebenso kann zum Beispiel dann, wenn ein Kriteriumsgewicht betragsmäßig wesentlich größer ist als alle anderen Gewichte, häufig die Bewertung bereits nach der Ermittlung des entsprechenden Kriteriumsgewichts abgebrochen werden. Wenn beispielsweise in einer ersten Testsequenz ein unerwünschtes Motiv nicht aufgetaucht ist und in einer zweiten Testsequenz das unerwünschte Motiv auftaucht, kann die zweite Testsequenz sofort ausgeschlossen werden, da das Kriteriumsgewicht für die Motivsuche so groß ist, daß es nicht durch andere Kriteriumsgewichte kompensiert werden kann.

Insbesondere kann die Erfindung vorsehen, daß bei Ausführungsformen, bei denen die Gütefunktion iterativ berechnet werden kann, zumindest bei einer Iteration eine obere (bzw. bei Optimierung auf das Minimum der Gütefunktion untere) Grenze bestimmt wird, unterhalb (bzw. oberhalb) derer der Wert der vollständigen Gütefunktion liegt, und die Iteration der Gütefunktion abgebrochen wird, wenn dieser Wert unter (bzw. über) dem Wert der vollständigen Gütefunktion für eine Testsequenz liegt, der vorangehend ermittelt wurde.

Die Erfindung kann in diesen Fällen vorsehen, daß im weiteren Verfahren für diese Testsequenz als Wert der Gütefunktion die besagte obere bzw. untere Grenze, falls erforderlich, verwendet wird und/oder daß die entsprechende Testsequenz in dem Algorithmus ausgeschieden wird, etwa dadurch, daß die Variable für die optimierte Testsequenz mit einer vorangehend aufgefundenen Testsequenz besetzt bleibt, bei der die Gütefunktion einen höheren Wert als die oben genannte Grenze, und der Algorithmus zu der Bewertung der nächsten Testsequenz übergeht. Die Erfindung kann dabei, insbesondere wenn die Gütefunktion eine Linearkombination von Kriteriumsgewichten ist, vorsehen, daß in den ersten Iterationen derjenige Beitrag oder diejenigen Beiträge berechnet werden, deren höchster Wert bzw. deren minimaler Wert den höchsten Absolutbetrag besitzt.

Die Erfindung kann vorsehen, daß bei einer Gütefunktion, die auf ihr Maximum optimiert wird und die durch eine Linearkombination von Kriteriumsgewichten gebildet wird, zunächst die positiven Anteile der Linearkombination berechnet werden und die Iteration abgebrochen wird, wenn in einer Iteration nach der Berechnung aller positiven Kriteriumsgewichte der Wert der Gütefunktion in dieser Iteration kleiner ist als der Wert der vollständigen Gütefunktion für eine andere Testsequenz.

Die Erfindung kann auch vorsehen, daß eine Iteration der Gütefunktion abgebrochen wird, wenn in einer Iteration festgestellt wird, daß die Summe aus dem in dieser Iteration berechneten Wert der Gütefunktion und dem Höchstwert des Beitrags der noch nicht berechneten Kriteriumsgewichte unterhalb des Werts der vollständigen Gütefunktion einer anderen Testsequenz liegt.

Das erfindungsgemäße Verfahren kann den Schritt des Synthetisierens der optimierten Nucleotidsequenz umfassen.

Dabei kann vorgesehen sein, daß der Schritt des Synthetisierens der optimierten Nucleotidsequenz in einer Vorrichtung zum automatischen Synthetisieren von Nucleotidsequenzen, zum Beispiel in einem Oligonucleotidsynthesizer, stattfindet, welcher von dem Rechner angesteuert wird, der die Nucleotidsequenz optimiert.

Die Erfindung kann insbesondere vorsehen, daß der Rechner, sobald der Optimierungsprozeß abgeschlossen ist, die ermittelten Daten über die optimale Nucleotidsequenz an einen Oligonucleotidsynthesizer weitergibt und diesen veranlaßt, die Synthese der optimierten Nucleotidsequenz durchzuführen.

Diese Nucleotidsequenz kann dann, wie gewünscht, hergestellt werden. Zur Expression des Proteins wird die entsprechende Nucleotidsequenz in Wirtszellen eines Wirtsorganismus eingebracht, auf welchen sie optimiert ist und welcher dann letztendlich das Protein erzeugt.

Die Erfindung stellt auch eine Vorrichtung zum Optimieren einer Nucleotidsequenz zur Maximierung der Expression eines Proteins auf der Grundlage der Aminosäurensequenz des Proteins zur Verfügung, welche eine Recheneinrichtung aufweist, welche umfaßt:

- eine Einrichtung zum Generieren einer ersten Testsequenz von n Codons, welche n aufeinanderfolgenden Aminosäuren in der Proteinsequenz entsprechen, wobei n eine natürliche Zahl kleiner oder gleich N, der Zahl der Aminosäuren der Proteinsequenz ist,

- eine Einrichtung zum Festlegen von n Optimierungspositionen in der Testsequenz, welche der Position von m Codons entsprechen, an denen die Besetzung mit einem Codon, bezogen auf die Testsequenz, optimiert werden soll, wobei $m \leq n$ und $m < M$ ist,
- eine Einrichtung zum Generieren einer oder mehrerer weiterer Testsequenzen aus der ersten Testsequenz, indem an einer oder mehreren der m Optimierungspositionen ein Codon der ersten Testsequenz durch ein anderes Codon ersetzt wird, welches dieselbe Aminosäure exprimiert,
- eine Einrichtung zum Bewerten jeder der Testsequenzen mit einer Gütefunktion und zum Ermitteln der hinsichtlich der Gütefunktion optimalen Testsequenz,
- eine Einrichtung zum Festlegen von p Codons der optimalen Testsequenz, welche sich an einem der m Optimierungspositionen befinden, als Ergebniscodons, welche die Codons der optimierten Nucleotidsequenz an den Positionen bilden, die den Positionen der besagten p Codons in der Testsequenz entsprechen, wobei p eine natürliche Zahl und $p \leq m$ und in einem oder mehreren Iterationsschritten p gleich Eins ist,
- eine Einrichtung zum Iterieren der vorangehenden Schritte,

wobei in jedem Iterationsschritt die Testsequenz an den Positionen, welche Positionen von festgelegten Ergebniscodons in der optimierten Nucleotidsequenz entsprechen, das entsprechende Ergebniscodon enthält und die Optimierungspositionen von Positionen von Ergebniscodons verschieden sind.

Die vorangehend genannten Einrichtungen müssen nicht verschieden sein, sondern können insbesondere durch eine einzige Vorrichtung realisiert werden, welche die Funktionen der vorangehend genannten Einrichtungen realisiert.

Die erfindungsgemäße Vorrichtung kann allgemein eine Einrichtung zum Durchführen der Schritte der vorangehend beschriebenen Verfahren aufweisen.

Die erfindungsgemäße Vorrichtung kann einen Oligonucleotidsynthesizer aufweisen, welcher von dem Rechner so angesteuert wird, daß er die optimierte Nucleotidsequenz synthetisiert.

Bei dieser Ausführungsform der Erfindung kann entweder automatisch oder durch einen entsprechenden Befehl des Benutzers die optimierte Nucleotidsequenz synthetisiert werden, ohne daß Datentransfers, Einstellung von Parametern und dergleichen nötig sind.

Die Erfindung stellt auch ein Computerprogramm zur Verfugung, welches von einem Computer ausführbaren Programmcode enthält, der, wenn er auf einem Computer ausgeführt wird, den Computer veranlaßt, ein erfindungsgemäßes Verfahren durchzuführen.

Dabei kann der Programmcode, wenn er auf einem Computer ausgeführt wird, eine Vorrichtung zum automatischen Synthetisieren von Nucleotidsequenzen veranlassen, die optimierte Nucleotidsequenz herzustellen.

Die Erfindung stellt auch einen computerlesbaren Datenträger zur Verfügung, auf welchem in computerlesbarer Form ein erfindungsgemäßes Programm gespeichert ist.

Ein Beispiel stellt eine nach einem Verfahren hergestellte oder herstellbare Nukleinsäure und einen Vektor, der eine solche Nukleinsäure enthält, zur Verfügung. Das Beispiel stellt weiterhin eine Zelle, die einen solchen Vektor oder eine solche Nukleinsäure enthält, zur Verfügung sowie einen nicht-menschlichen Organismus bzw. ein nicht-menschliches Lebewesen, das eine solche Zelle enthält, wobei ein solches nicht-menschliches Lebewesen auch ein Säugetier sein könnte.

Während bei statistischen Verfahren keinerlei Korrelation zwischen einer Sequenz in einem vorangehenden Iterationsschritt und der Sequenz in einem nachfolgenden Iterationsschritt besteht, wird erfindungsgemäß in jedem Iterationsschritt zumindest ein Codon neu festgelegt. Da die Testsequenz nur auf einem Teil der Gesamtsequenz variiert wird, ist das Verfahren mit einem geringeren Aufwand durchführbar. Insbesondere ist es möglich, in dem Variationsbereich sämtliche möglichen Kombinationen von Codons zu evaluieren. Die Erfindung macht sich in vorteilhafter Weise den Umstand zunutze, daß langreichweitige Korrelationen innerhalb einer Nucleotidsequenz von untergeordneter Bedeutung sind, d.h. daß zur Erzielung eines akzeptablen Optimierungsergebnisses die Codons an einer Position weitgehend unabhängig von den Codons an einer weiter entfernten Position variiert werden können.

Das erfindungsgemäße Verfahren eröffnet in größerem Umfang als die bisherigen Verfahren die Möglichkeit, relevante biologische Kriterien in die Bewertung einer Testsequenz einzubeziehen. Beispielsweise können mit dem erfindungsgemäßen Verfahren erwünschte oder unerwünschte Motive in der synthetischen Nukleotidsequenz berücksichtigt werden. Da bei einer Motivsuche bereits ein individuelles Codon dafür ausschlaggebend sein kann, ob ein bestimmtes Motiv vorhanden ist oder nicht, werden rein stochastische Verfahren nicht oder nur mit einer sehr geringen Wahrscheinlichkeit optimierte Sequenzen liefern, welche ein gefordertes Motiv enthalten. Bei dem erfindungsgemäßen Verfahren ist dies jedoch deswegen möglich, da über einem Teilbereich der Sequenz sämtliche Codonkombinationen durchgetestet werden. Gegebenenfalls kann man, um das Vorhandensein bzw. Nichtvorhandensein eines bestimmten Sequenzmotivs zu gewährleisten, die Anzahl m der Optimierungspositionen so groß machen, daß diese größer ist als die Zahl der Codonpositionen (oder die Anzahl der Basenpositionen, geteilt durch 3) des entsprechenden Motivs. Wenn die m Optimierungspositionen zusammenhängend sind, ist damit gewährleistet, daß das Auftauchen eines bestimmten Sequenzmotivs zuverlässig erfaßt und das entsprechende Motiv in der Sequenz gewährleistet bzw. aus dieser ausgeschlossen werden kann. Die numerische Berechnung der Gütefunktion hat besondere Vorteile bei der Verwendung von Gewichtsmatrix-Scans. Da hierbei den verschiedenen Basen einer Erkennungssequenz eine unterschiedlich starke Bedeutung für die Erkennung bzw. die biologische Aktivität zugeordnet werden kann, kann bei dem erfindungsgemäßen Verfahren, bei dem über einen Teilbereich der Sequenz alle möglichen Codonkombinationen durchgetestet werden, die Sequenz gefunden werden, die zum Beispiel ein DNA-Motiv durch Eliminierung der für die Aktivität wichtigsten Basen am effektivsten ausschaltet bzw. es kann eine optimierte Kompromißlösung unter Einbeziehung anderer Kriterien gefunden werden.

Die Erfindung ist grundsätzlich nicht auf einen bestimmten Organismus beschränkt. Organismen, für welche eine Optimierung einer Nukleotidsequenz zur Expression eines Proteins mit dem erfindungsgemäßen Verfahren von besonderem Interesse ist, sind z.B. Organismen aus der folgenden Gruppe:

- Viren, insbesondere Vaccinia-Viren,
- Prokaryonten, insbesondere Escherichia coli, Caulobacter cresentus, Bacillus subtilis, Mycobacterium spec.,
- Hefen, insbesondere Saccharomyces cerevisiae, Schizosaccharomyces pombe, Pichia pastoris, Pichia angusta,
- Insekten, insbesondere Sprodoptera frugiperda, Drosophila spec,
- Säuger, insbesondere Homo sapiens, Macaca mulata, Mus musculus, Bos taurus, Capra hircus, Ovis aries, Oryctolagus cuniculus, Rattus norvegicus, chinese hamster ovary,
- monokotyle Pflanzen, insbesondere Oryza sativa, Zea mays, Triticum aestivum
- dikotyle Pflanzen, insbesondere Glycin max, Gossypium hirsutum, Nicotiana tabacum, Arabidopsis thaliana, Solanum tuberosum.

Proteine, für die eine optimierte Nucleotidsequenz mit dem erfindungsgemäßen Verfahren generiert werden kann, sind zum Beispiel:

- Enzyme, insbesondere Polymerasen, Endonukleasen, Ligasen, Lipasen, Proteasen, Kinasen, Phosphatasen, Topoisomerasen,
- Cytokine, Chemokine, Transkriptionsfaktoren, Oncogene,
- Proteine aus thermophilen Organismen, aus cryophilen Organismen, aus halophilen Organismen, aus acidophilen Organismen, aus basophilen Organismen,
- Proteine mit repetitiven Sequenzelementen, insbesondere strukturgebende Proteine,
- Humane Antigene, insbesondere Tumorantigene, Tumormarker, Autoimmunantigene, diagnostische Marker,
- Virale Antigene, insbesondere von HAV, HBV, HCV, HIV, SIV, FIV, HPV, Rinoviren, Influenzaviren, Herpesviren, Poliomaviren, Hendra Virus, Dengue Virus, AAV, Adenoviren, HTLV, RSV,
- Antigene von Protozoen und/oder parasitären Erregern, insbesondere Erreger von Malaria, Leishmania, Trypanosoma, Toxoplasmen, Amöba,
- Antigene von bakteriellen Erregern oder Pathogene, insbesondere von den Genera Chlamydia, Staphylococcen, Klebsiella, Streptococcus, Salmonella, Listeria, Borrelia, Escherichia coli,
- Antigene von Organismen der Sicherheitstufe L4, insbesondere Bacillus anthracis, Ebola-Virus, Marburg-Virus, Pockenviren.

Die vorangehende Aufzählung von Organismen bzw. Proteinen, für welche die Erfindung Anwendung findet, ist in keiner Weise einschränkend und lediglich als Beispiel zur besseren Veranschaulichung gedacht.

Die Erfindung kann insbesondere in einer der folgenden Ausführungsformen realisiert werden:

1. Verfahren zum Optimieren einer Nucleotidsequenz zur Expression eines Proteins auf der Grundlage der Aminosäurensequenz des Proteins, welches die folgenden auf einem Computer durchgeführten Schritte umfaßt:

   - Generieren einer ersten Testsequenz von n Codons, welche n aufeinanderfolgenden Aminosäuren in der Proteinsequenz entsprechen, wobei n eine natürliche Zahl und kleiner oder gleich N, der Zahl der Aminosäuren der Proteinsequenz, ist,
   - Festlegen von m Optimierungspositionen in der Testsequenz, welche der Position von m Codons entsprechen, an denen die Besetzung mit einem Codon, bezogen auf die Testsequenz, optimiert werden soll, wobei $m \leq n$ und $m < N$ ist,
   - Generieren einer oder mehrerer weiterer Testsequenzen aus der ersten Testsequenz, indem an einer oder mehreren der m Optimierungspositionen ein Codon der ersten Testsequenz durch ein anderes Codon ersetzt wird, welches dieselbe Aminosäure exprimiert,

- Bewerten jeder der Testsequenzen mit einer Gütefunktion und Ermitteln der hinsichtlich der Gütefunktion optimalen Testsequenz,
- Festlegen von p Codons der optimalen Testsequenz, welche sich an einer der m Optimierungspositionen befinden, als Ergebniscodons, welche die Codons der optimierten Nucleotidsequenz an den Positionen bilden, die der Position der besagten p Codons in der Testsequenz entspricht, wobei p eine natürliche Zahl und $p \leq m$ ist,
- Iterieren der vorangehenden Schritte, wobei in jedem Iterationsschritt die Testsequenz an den Positionen, welche Positionen von festgelegten Ergebniscodons in der optimierten Nucleotidsequenz entsprechen, das entsprechende Ergebniscodon enthält und die Optimierungspositionen von Positionen von Ergebniscodons verschieden sind.

2. Verfahren nach Ausführungsform 1, dadurch gekennzeichnet, daß in einem oder mehreren Iterationsschritten die m Optimierungspositionen der Testsequenzen unmittelbar auf ein oder mehrere Ergebniscodons folgen, welche als Teil der optimierten Nucleotidsequenz festgelegt worden sind.

3. Verfahren nach Ausführungsform 1 oder 2, dadurch gekennzeichnet, daß in einem oder mehreren Iterationsschritten die p Codons, die als Ergebniscodons der optimierten Nucleotidsequenz festgelegt werden, p aufeinanderfolgende Codons sind.

4. Verfahren nach einer der Ausführungsformen 1 bis 3, dadurch gekennzeichnet, daß in einem Iterationsschritt Testsequenzen mit allen möglichen Codonbesetzungen für die m Optimierungspositionen aus der ersten Testsequenz generiert werden und die optimale Testsequenz unter diesen Testsequenzen ermittelt wird.

5. Verfahren nach einer der Ausführungsformen 1 bis 4, gekennzeichnet durch:

- Bewerten jeder Testsequenz mit einer Gütefunktion,
- Ermitteln eines Extremwertes innerhalb der Werte der Gütefunktion für alle in einem Iterationsschritt generierten Teilsequenzen,
- Festlegen von p Codons der Testsequenz, welche dem extremalen Wert der Gewichtsfunktion entspricht, als Ergebniscodons an den entsprechenden Positionen, wobei p eine natürliche Zahl und $p \leq m$ ist.

6. Verfahren nach Ausführungsform 5, dadurch gekennzeichnet, daß die Gütefunktion eines oder mehrere der folgenden Kriterien berücksichtigt:
Codon usage für einen vorgegebenen Organismus, GC-Gehalt, repetitive Sequenzen, Sekundärstrukturen, invers komplementäre Sequenzwiederholungen und Sequenzmotive.

7. Verfahren nach Ausführungsform 6, dadurch gekennzeichnet, daß die Gütefunktion eine Funktion von verschiedenen Einzeltermen ist, die jeweils ein Kriterium aus der folgenden Liste von Kriterien bewerten:
Codon usage für einen vorgegebenen Organismus, GC-Gehalt, Sequenzmotive, repetitive Sequenzen, Sekundärstrukturen, invers komplementäre Sequenzwiederholungen.

8. Verfahren nach einer der Ausführungsformen 1 bis 6, dadurch gekennzeichnet, daß die Gütefunktion eines oder mehrere der folgenden Kriterien berücksichtigt:

- Ausschluß von invers komplementären Sequenzidentitäten von mehr als 20 Nucleotiden zum Transkriptom eines vorgegebenen Organismus,
- Ausschluß von Homologiebereichen von mehr als 100 Basenpaaren zu einer vorgegebenen DNS-Sequenz,
- Ausschluß von Homologiebereichen mit mehr als 90 % Ähnlichkeit der Nucleotidsequenz zu einer vorgegebenen DNS-Sequenz.

9. Verfahren nach einer der Ausführungsformen 1 bis 8, gekennzeichnet durch den Schritt des Synthetisierens der optimierten Nucleotidsequenz.

10. Verfahren nach Ausführungsform 9, dadurch gekennzeichnet, daß der Schritt des Synthetisierens der optimierten Nucleotidsequenz in einer Vorrichtung zum automatischen Synthetisieren von Nucleotidsequenzen stattfindet, welcher von dem Rechner angesteuert wird, der die Nucleotidsequenz optimiert.

11. Vorrichtung zum Optimieren einer Nucleotidsequenz zur Expression eines Proteins auf der Grundlage der Aminosäurensequenz des Proteins, welche eine Recheneinrichtung aufweist, welche umfaßt:

- eine Einrichtung zum Generieren einer ersten Testsequenz von n Codons, welche n aufeinanderfolgenden Aminosäuren in der Proteinsequenz entsprechen, wobei n eine natürliche Zahl und kleiner oder gleich N, der Zahl der Aminosäuren der Proteinsequenz ist,
- eine Einrichtung zum Festlegen von m Optimierungspositionen in der Testsequenz, welche der Position von m Codons entsprechen, an denen die Besetzung mit einem Codon, bezogen auf die Testsequenz, optimiert werden soll, wobei $m \leq n$ und $m < M$ ist,
- eine Einrichtung zum Generieren einer oder mehrerer weiterer Testsequenzen aus der ersten Testsequenz, indem an einer oder mehreren der m Optimierungspositionen ein Codon der ersten Testsequenz durch ein anderes Codon ersetzt wird, welches dieselbe Aminosäure exprimiert,
- eine Einrichtung zum Bewerten jeder der Testsequenzen mit einer Gütefunktion und zum Ermitteln der hinsichtlich der Gütefunktion optimalen Testsequenz,
- eine Einrichtung zum Festlegen von p Codons der optimalen Testsequenz, welche sich an einem der m Optimierungspositionen befinden, als Ergebniscodons, welche die Codons der optimierten Nucleotidsequenz an den Positionen bilden, die den Positionen der besagten p Codons in der Testsequenz entsprechen, wobei p eine natürliche Zahl und $p \leq m$ ist,
- eine Einrichtung zum Iterieren der Schritte des Generierens mehrerer Testfunktionen, der Bewertung der Testsequenzen und des Festlegens von Ergebniscodons, wobei in jedem Iterationsschritt die Testsequenz an den Positionen, welche Positionen von festgelegten Ergebniscodons in der optimierten Nucleotidsequenz entsprechen, das entsprechende Ergebniscodon enthält und die Optimierungspositionen von Positionen von Ergebniscodons verschieden sind.

12. Vorrichtung nach Ausführungsform 11, gekennzeichnet durch eine Einrichtung zum Durchführen der Schritte eines Verfahrens nach einer der Ausführungsformen 1 bis 7.

13. Vorrichtung nach einer der Ausführungsformen 11 oder 12, gekennzeichnet durch eine Vorrichtung zum automatischen Synthetisieren von Nucleotidsequenzen, welcher von dem Rechner so angesteuert wird, daß er die optimierte Nucleotidsequenz synthetisiert.

14. Computerprogramm, welches von einem Computer ausführbaren Programmcode enthält, der, wenn er auf einem Computer ausgeführt wird, den Computer veranlaßt, ein Verfahren nach einer der Ausführungsformen 1 bis 8 durchzuführen.

15. Computerprogramm nach Ausführungsform 14, wobei der Programmcode, wenn er auf einem Computer ausgeführt wird, eine Vorrichtung zum automatischen Synthetisieren von Nucleotidsequenzen veranlassen kann, die optimierte Nucleotidsequenz herzustellen.

16. Computerlesbarer Datenträger, auf welchem in computerlesbarer Form ein Programm nach einer der Ausführungsformen 14 oder 15 gespeichert ist.

17. Nukleinsäure, welche eine für ein Protein codierende Nucleotidsequenz umfaßt, erhältlich durch ein Verfahren nach Ausführungsform 9.

18. Nukleinsäure nach Ausführungsform 17, dadurch gekennzeichnet, daß diese eine Nucleotidsequenz umfaßt, welche in einem vorgegebenen Organismus für ein Protein codiert, wobei die besagte Nucleotidsequenz in dem natürlich vorkommenden Genom des Organismus nicht enthalten ist.

19. Nukleinsäure nach Ausführungsform 18, dadurch gekennzeichnet, daß der Organismus ausgewählt aus der folgenden Gruppe ist:

- Viren, insbesondere Vaccinia-Viren,
- Prokaryonten, insbesondere Escherichia coli, Caulobacter cresentus, Bacillus subtilis, Mycobacterium spec.,
- Hefen, insbesondere Saccharomyces cerevisiae, Schizosaccharomyces pombe, Pichia pastoris, Pichia angusta,
- Insekten, insbesondere Sprodoptera frugiperda, Drosophila spec,
- Säuger, insbesondere Homo sapiens, Macaca mulata, Mus musculus, Bos taurus, Capra hircus, Ovis aries, Oryctolagus cuniculus, Rattus norvegicus, chinese hamster ovary,
- Monokotyle Pflanzen, insbesondere Oryza sativa, Zea mays, Triticum aestivum
- Dikotyle Pflanzen, insbesondere Glycin max, Gossypium hirsutum, Nicotiana tabacum, Arabidopsis thaliana, Solanum tuberosum.

20. Nukleinsäure nach einer der Ausführungsformen 1 bis 19, dadurch gekennzeichnet, daß das durch die Nucleotidsequenz codierte Protein eines der folgenden Proteine ist und/oder in einer der folgenden Proteinklassen fällt:

- Enzyme, insbesondere Polymerasen, Endonukleasen, Ligasen, Lipasen, Proteasen, Kinasen, Phosphatasen, Topoisomerasen,
- Cytokine, Chemokine, Transkriptionsfaktoren, Oncogene,
- Proteine aus thermophilen Organismen, aus cryophilen Organismen, aus halophilen Organismen, aus acidophilen Organismen, aus basophilen Organismen,
- Proteine mit repetitiven Sequenzelementen, insbesondere strukturgebende Proteine,
- Humane Antigene, insbesondere Tumorantigene, Tumormarker, Autoimmunantigene, diagnostische Marker,
- Virale Antigene, insbesondere von HAV, HBV, HCV, HIV, SIV, FIV, HPV, Rinoviren, Influenzaviren, Herpesviren, Poliomaviren, Hendra Virus, Dengue Virus, AAV, Adenoviren, HTLV, RSV,
- Antigene von parasitären Erregern, z.B. Protozoen, insbesondere Erreger von Malaria, Leishmania, Trypanosoma, Toxoplasmen, Amöba,
- Antigene von bakteriellen Erregern oder Pathogene, insbesondere von den Genera Chlamydia, Staphylococcen, Klebsiella, Streptococcus, Salmonella, Listeria, Borrelia, Escherichia coli,
- Antigene von Organismen der Sicherheitstufe L4, insbesondere Bacillus anthracis, Ebola-Virus, Marburg-Virus, Pockenviren.

21. Nukleinsäure nach einer der Ausführungsformen 18 oder 19, dadurch gekennzeichnet, daß die Gütefunktion zumindest eines der folgenden Kriterien berücksichtigt:

- GC-Gehalt,
- Codon Usage des vorgegebenen Organismus,
- Ausschluß von invers komplementären Sequenzidentitäten von mehr als 20 Nucleotiden zum Transkriptom eines vorgegebenen Organismus,
- vollständiger oder weitgehender Ausschluß von Homologiebereichen von mehr als 100 Basenpaaren zu einer vorgegebenen DNS-Sequenz,
- vollständiger oder weitgehender Ausschluß von Homologiebereichen mit einer Ähnlichkeit von mehr als 90 % zu einer vorgegebenen DNS-Sequenz.

22. Vektor, umfassend eine Nukleinsäure nach einer der Ausführungsformen 17 bis 21.

23. Zelle, umfassend einen Vektor nach Ausführungsform 22 oder eine Nukleinsäure nach einer der Ausführungsformen 17 bis 21.

24. Organismus, umfassend mindestens eine Zelle nach Ausführungsform 23.

25. Nukleinsäure, insbesondere nach Ausführungsform 9, umfassend eine Nucleotidsequenz, die ausgewählt ist aus der Gruppe, umfassend: SEQ ID NO: 2, 4, 6, 8.

26. Vektor, umfassend eine Nukleinsäure nach Ausführungsform 25.

27. Zelle, umfassend einen Vektor nach Ausführungsform 26 oder eine Nukleinsäure nach Ausführungsform 25.

| | |
|---|---|
| Figur 1a, 1b | zeigen ein Flußdiagramm eines Ausführungsbeispiels des Verfahrens der Erfindung, |
| Figur 2 | illustriert das Verhältnis von Testsequenz, optimierter DNS-Sequenz, Kombinations-DNS-Sequenz und Aminosäuresequenz für ein Ausführungsbeispiel der Erfindung, |
| Figur 3 | zeigt die Bereiche für die Bestimmung der Sequenzwiederholung, |
| Figur 4a und 4b | zeigen schematisch ein Schema für die Bestimmung von Sequenzwiederholungen, |
| Figur 5a | zeigt die Codon usage bei einer ausschließlichen Optimierung auf die Codon usage, |
| Figur 5b | zeigt den GC-Gehalt bei einer ausschließlichen Optimierung auf die Codon usage, |
| Figur 6a | zeigt die Codon usage bei Verwendung einer ersten Gütefunktion, |
| Figur 6b | zeigt den GC-Gehalt bei Verwendung einer ersten Gütefunktion, |
| Figur 7a | zeigt die Codon usage bei Verwendung einer zweiten Gütefunktion, |
| Figur 7b | zeigt den GC-Gehalt bei Verwendung einer zweiten Gütefunktion, |

| | |
|---|---|
| Figur 8a | zeigt die Codon usage bei Verwendung einer dritten Gütefunktion, |
| Figur 8b | zeigt den GC-Gehalt bei Verwendung einer dritten Gütefunktion, |
| Fig. 9 | zeigt eine repräsentative Maus-MIP1alpha-Eichgerade im Zusammenhang mit dem Beispiel 3, |
| Fig. 10 | illustriert die prozentuale Steigerung der Gesamtproteinmenge nach Transfektion von synthetischen Expressionskonstrukten gegenüber Wildtyp-Expressionskonstrukten im Zusammenhang mit dem Beispiel 3, |
| Fig. 11 | zeigt eine repräsentative ELISA-Analyse der Zellysate und Überstände transfizierter H1299-Zellen im Zusammenhang mit dem Beispiel 3 und |
| Fig. 12A bis 12C | zeigt die Expressionsanalyse der synthetischen Leserahmen und der Wildtyp-Leserahmen im Zusammenhang mit dem Beispiel 3. |

Gemäß einer bevorzugten Ausführungsform der Erfindung wird in einer Iteration die Wahl des Codons für die i-te Aminosäure einer Aminosäuresequenz der Länge N betrachtet. Dazu werden sämtliche möglichen Codonkombinationen der verfügbaren Codons für die Aminosäuren an den Positionen i bis i + m - 1 gebildet. Diese Positionen bilden ein Variationsfenster und legen die Optimierungspositionen fest, auf denen die Sequenz variiert werden soll. Jede Kombination von Codons auf diesem Variationsfenster resultiert in einer DNS-Sequenz mit 3 m Basen, die im folgenden Kombinations-DNS-Sequenz (KDS) genannt wird. In jedem Iterationsschritt wird zu jeder KDS eine Testsequenz gebildet, welche die KDS an ihrem Ende enthält. Im ersten Iterationsschritt bestehen die Testsequenzen nur aus den Kombinations-DNS-Sequenzen. Die Testsequenzen werden mit einer nachfolgend näher beschriebenen Gütefunktion gewichtet und das erste Codon derjenigen KDS, welche den maximalen Wert der Gütefunktion aufweist, wird für alle weiteren Iterationen als Codon der optimierten Nucleotidsequenz (Ergebniscodon) beibehalten. Dies bedeutet, daß dann, wenn in einer Iteration das i-te Codon festgelegt wurde, jede der Testsequenzen in der nächsten Iteration dieses Codon an der Position i enthält und an den Positionen i + 1 bis i + m die Codons der verschiedenen Kombinations-DNS-Sequenzen. Bei der j-ten Iteration bestehen also alle Testsequenzen an den Positionen 1 bis j - 1 aus den in den vorangehenden Iterationen als optimal aufgefundenen Codons, während die Codons an den Positionen j bis j + m - 1 variiert werden. Die Güte der DNS-Sequenz läßt sich für jedes individuelle Testkriterium als Kriteriumsgewicht (Einzelscore) ausdrücken. Durch Addition der nach benutzerdefinierten Vorgaben gewichteten Kriteriumsgewichte wird ein Gesamtgewicht (Gesamtscore) gebildet, welches den Wert der Gütefunktion für die gesamte Testsequenz angibt. Wenn j = N - m + 1 ist, ist die optimale Testsequenz gleichzeitig die optimierte Nucleotidsequenz nach dem Verfahren der Erfindung.

Daher werden in diesem (letzten) Schritt sämtliche Codons der optimal KDS als Codons der optimierten Nucleotidsequenz festgelegt.

Der vorangehend beschriebene Ablauf ist schematisch in Figur 1 illustriert. Der Algorithmus beginnt bei der ersten Aminosäure (i=1). Es wird nun eine erste KDS der Codons für die Aminosäuren i bis i + m -1 gebildet (bei der ersten Iteration sind dies die Aminosäuren 1 bis m). Diese KDS wird mit der bereits optimierten DNS-Sequenz zu einer Testsequenz zusammengefügt. Im ersten Schritt besteht die optimierte DNS-Sequenz aus 0 Elementen. Daher besteht die Testsequenz bei der ersten Iteration nur aus der zuvor gebildeten (ersten) KDS.

Die Testsequenz wird nun nach benutzerdefinierten Kriterien evaluiert. Der Wert einer Gütefunktion wird berechnet, indem Kriteriumsgewichte für verschiedene Bewertungskriterien berechnet und in einer Bewertungsfunktion verrechnet werden. Wenn der Wert der Gütefunktion besser als ein gespeicherter Wert der Gütefunktion ist, wird der neue Wert der Gütefunktion gespeichert. Gleichzeitig wird auch das erste Codon der zugehörigen KDS, welches die Aminosäure i repräsentiert, gespeichert. Wenn der Wert der Gütefunktion schlechter als der gespeicherte Wert ist, erfolgt keine Maßnahme. Im nächsten Schritt wird überprüft, ob alle möglichen KDS gebildet worden sind. Ist dies nicht der Fall, wird die nächstmögliche KDS gebildet und mit der bereits optimierten DNS-Sequenz zu einer neuen Testsequenz zusammengefügt. Die Schritte des Evaluierens, des Bestimmens einer Gütefunktion und des Vergleichs des Wertes der Gütefunktion mit einem gespeicherten Wert wiederholen sich dann. Sind dagegen alle möglichen KDS gebildet worden, wird, sofern $i \neq N - m + 1$ ist, das gespeicherte Codon an die bereits gebildete optimierte DNS-Sequenz an der Position i angefügt. Bei der ersten Iteration wird die optimierte DNS-Sequenz dadurch gebildet, daß das gespeicherte Codon auf die Position 1 der optimierten DNS-Sequenz gesetzt wird. Der Prozeß wiederholt sich dann für die nächste Aminosäure (i + 1). Ist dagegen i = N - m + 1, wird die gesamte KDS der optimalen Testsequenz an die bereits gebildete optimierte DNS-Sequenz angehängt, da sie bereits hinsichtlich der Bewertungskriterien optimiert ist. Es folgt dann die Ausgabe der optimierten Sequenz.

Das Verhältnis der verschiedenen Bereiche ist diagrammatisch in Figur 2 dargestellt. Man erkennt die Kombinations-DNS-Sequenz und den Bereich der bereits festgelegten optimierten DNS-Sequenz.

Der Parameter m kann in weiten Bereichen variiert werden, wobei im Sinne einer bestmöglichen Optimierung eine möglichst hohe Zahl von variierten Codons angestrebt wird. Mit den derzeit verfügbaren Rechnern läßt sich mit einer Größe des Variationsfensters von m = 5 bis m = 10 in einer akzeptablen Zeit ein sinnvolles Optimierungsergebnis erreichen.

Neben der individuellen Gewichtung der Kriteriumsgewichte können sowohl das Gesamtgewicht als auch die Kriteriumsgewichte durch geeignete mathematische Funktionen definiert sein, die gegenüber den einfachen Relationen, wie Differenz oder Proportion, modifiziert sind, z.B. durch abschnittsweise definierte Funktionen, welche einen Schwellenwert definieren, oder nichtlineare Funktionen. Ersteres ist beispielsweise bei der Bewertung von Wiederholungen oder invers komplementären Wiederholungen sinnvoll, die erst ab einer bestimmten Größe berücksichtigt werden sollen. Letzteres ist z.B. bei der Bewertung der Codon usage oder des CG-Gehalts sinnvoll.

Nachfolgend werden verschiedene beispielhafte Gewichtungskriterien erläutert, die erfindungsgemäß verwendet werden können, ohne daß die Erfindung auf diese Kriterien bzw. die nachfolgend beschriebenen Gewichtungsfunktionen beschränkt wäre.

Die Anpassung der Codon usage des synthetischen Gens an die Codonusage des Wirtsorganismus ist eines der wichtigsten Kriterien bei der Optimierung. Hierbei muß die unterschiedliche Degeneriertheit der verschiedenen Codons (einfach bis sechsfach) berücksichtigt werden. Hierfür geeignete Größen sind z.B. die RSCU (relative synonymous codon usage) oder relative Häufigkeiten (Relative Adaptiveness), die auf die Häufigkeit des am meisten von dem Organismus genutzten Codons normiert sind (das am meisten genutzte Codon hat also die "Codon usage" 1), vgl. P. M. Sharp, W. H. Li, Nucleic Acid Research 15 (1987), 1281 bis 1295.

Zur Bewertung einer Testsequenz wird bei einer Ausführungsform der Erfindung die durchschnittliche Codon usage auf dem Variationsfenster verwendet.

Bei der Bewertung des GC-Gehalts ist eine möglichst geringe Abweichung des durchschnittlichen GC-Gehaltes von dem vorgegebenen gewünschten GC-Gehalt erforderlich. Weiterhin ist es anzustreben, Schwankungen des GC-Gehaltes über dem Verlauf der Sequenz gering zu halten.

Zur Evaluierung einer Testsequenz wird der durchschnittliche prozentuale GC-Gehalt desjenigen Bereichs der Testsequenz ermittelt, der die KDS und vor dem Beginn der KDS liegende Basen umfaßt, deren Anzahl b vorzugsweise zwischen 20 und 30 Basen liegt. Das Kriteriumsgewicht wird aus dem Absolutwert der Differenz zwischen dem gewünschten GC-Gehalt und dem ermittelten GC-Gehalt für die Testsequenz ermittelt, wobei dieser Absolutwert als Argument in eine nichtlineare Funktion, z.B. in eine Exponentialfunktion eingehen kann.

Wenn das Variationsfenster eine Breite von mehr als 10 Codonpositionen hat, können Schwankungen des GC-Gehalts innerhalb der KDS von Bedeutung sein. In diesen Fällen wird, wie vorangehend erläutert, der GC-Gehalt für jede Basenposition auf einem Fenster ermittelt, das bezüglich der Basenposition in einer bestimmten Weise ausgerichtet ist und eine bestimmte Anzahl, zum Beispiel 40 Basen, umfassen kann, und die Absolutwerte der Differenz zwischen dem gewünschten GC-Gehalt und dem für jede Basenposition ermittelten "lokalen" GC-Gehalt werden aufsummiert. Teilt man die Summe durch die Anzahl der ermittelten Einzelwerte, so erhält man als Kriteriumsgewicht die durchschnittliche Abweichung von dem gewünschten GC-Gehalt. Bei dem vorangehend beschriebenen Vorgehen kann die Lage des Fensters so definiert sein, daß die besagte Basenposition zum Beispiel am Rand oder im Zentrum des Fensters liegt. Alternativ kann auch als Kriterium der Absolutbetrag der Differenz zwischen dem tatsächlichen GC-Gehalt in der Testsequenz oder auf einem Teilbereich davon zu dem gewünschten GC-Gehalt oder der Absolutbetrag der Differenz zwischen dem Mittelwert des vorangehend erwähnten "lokalen" GC-Gehalts über die Testsquenz oder einem Teil davon und dem gewünschten GC-Gehalt als Kriterium verwendet werden. In einer weiteren Abwandlung kann auch vorgesehen sein, daß das entsprechende Kriteriumsgewicht proportional zu dem Quadrat der Differenz zwischen dem tatsächlichen GC-Gehalt und dem gewünschten GC-Gehalt, dem Quadrat der Differenz zwischen dem über die Basenpositionen gemittelten GC-Gehalt und dem gewünschten GC-Gehalt bzw. der Mittelwert des Quadrats der Differenzen zwischen dem lokalen GC-Gehalt und dem gewünschten GC-Gehalt als Kriterium verwendet werden. Das Kriteriumsgewicht für den GC-Gehalt hat das entgegengesetzte Vorzeichen wie das Kriteriumsgewicht für die Codon usage.

Lokale Erkennungssequenzen bzw. biophysikalische Charakteristika spielen in der Zell- und Molekularbiologie eine entscheidende Rolle. Eine unbeabsichtigte Generierung entsprechender Motive innerhalb der Sequenz des synthetisierten Gens kann unerwünschte Wirkungen haben. Zum Beispiel kann die Expression stark reduziert oder ganz unterdrückt werden; es kann auch eine für den Wirtsorganismus toxische Wirkung entstehen. Bei der Optimierung der Nucleotidsequenz ist es daher wünschenswert, die unbeabsichtige Generierung solcher Motive auszuschließen. Im einfachsten Fall läßt sich die Erkennungssequenz durch eine gut charakterisierte Consensussequenz (z.B. Restriktionsenzym-Erkennungssequenz) unter Verwendung entsprechender IUPAC-Basensymbole darstellen. Führt man eine einfache Regular-Expressionssuche innerhalb der Testsequenz durch, so erhält man für die Berechnung des entsprechenden Gewichts die Anzahl der aufgefundenen Positionen. Läßt man eine bestimmte Anzahl von Fehlstellen (mismatches) zu, muß die Anzahl der Fehlstellen bei einer erkannten Übereinstimmung bei der Ermittlung der Gewichtsfunktion berücksichtigt werden, zum Beispiel derart, daß das lokale Gewicht für eine Basenposition umgekehrt proportional zu der Anzahl der Basen ist, die einem IUPAC-Consensussymbol zugeordnet sind. In vielen Fällen ist die Consensussequenz jedoch nicht ausreichend eindeutig (vgl. zum Beispiel K. Quandt u.a., Nucleic Acid Research 23 (1995), 4878). In solchen Fällen kann man auf eine Matrizendarstellung der Motive zurückgreifen oder andere Erkennungsmethoden, z.B. mittels neuronaler Netze, verwenden.

Bei der bevorzugten Ausführungsform der Erfindung wird für jedes aufgefundene Motiv ein Wert zwischen 0 und 1 bestimmt, der im Idealfall die Bindungsaffinität der gefundenen (potentiellen) Stelle bzw. deren biologische Aktivität oder auch deren Erkennungssicherheit widerspiegelt. Für die Berechnung des Kriteriumsgewichts für DNS-Motive wird dieser Wert mit einem geeigneten Gewichtungsfaktor multipliziert und die Einzelwerte für jede aufgefundene Übereinstimmung werden addiert.

Das Gewicht für unerwünschte Motive geht mit dem umgekehrten Vorzeichen wie dasjenige für die Codon usage in die Gesamtgütefunktion ein.

In der gleichen Weise kann in die Gewichtung das Vorhandensein bestimmter erwünschter DNS-Motive, z.B. RE-Schnittsequenzen, bestimmte Enhancersequenzen oder immunstimulatorische bzw. immunsupprimierende CpG-Motive einbezogen werden. Das Gewicht für erwünschte DNS-Motive geht mit dem gleichen Vorzeichen wie das Gewicht für die Codon usage in die Gesamtbewertung ein.

Stark repetitive Sequenzabschnitte können zum Beispiel zu einer geringen genetischen Stabilität führen. Die Synthese repetitiver Abschnitte ist auch wegen der Gefahr von Fehlhybridisierung deutlich erschwert. Gemäß der bevorzugten Ausführungsform der Erfindung geht daher in die Bewertung einer Testsequenz ein, ob diese an unterschiedlichen Stellen identische oder einander ähnliche Sequenzabschnitte enthält. Das Vorhandensein entsprechender Abschnitte kann beispielsweise mit Hilfe einer Variante eines Dynamic Programming - Algorithmus zur Generierung eines lokalen Alignments der einander ähnlichen Sequenzabschnitte festgestellt werden. Wichtig bei dieser Ausführungsform der Erfindung ist, daß der verwendete Algorithmus einen Wert generiert, welcher geeignet ist, den Grad der Übereinstimmung und/oder die Länge der einander ähnlichen Sequenzabschnitte quantitativ zu beschreiben (Alignmentgewicht): Hinsichtlich weiterer Einzelheiten betreffend einen möglichen Algorithmus wird auf die oben genannten Lehrbücher von Gusfield oder Waterman bzw. M. S. Waterman, M. Eggert, J. Mol. Biology, (1987) 197, 723 bis 728, verwiesen.

Zur Berechnung des Kriteriumsgewichts hinsichtlich der repetitiven Elemente summiert man die Einzelgewichte aller lokalen Alignments, bei denen das Alignmentgewicht einen bestimmten Schwellenwert übersteigt. Die Addition dieser Einzelgewichte ergibt das Kriteriumsgewicht, welches die Repetitivität der Testsequenz charakterisiert.

Gemäß einer Abwandlung der vorangehend beschriebenen Ausführungsform wird nur der eine Bereich am Ende der Testsequenz, welcher das Variationsfenster sowie eine gewisse Anzahl weiterer Basen, z.B. 20 bis 30, umfaßt, daraufhin überprüft, ob ein Teilabschnitt der Testsequenz in diesem Bereich einer anderen Stelle der Testsequenz in gleicher oder ähnlicher Weise vorkommt. Dies ist schematisch in Figur 3 dargestellt. Die durchgezogene Linie in der Mitte stellt die gesamte Testsequenz dar. Die obere Linie stellt die KDS dar, während der untere Bereich den Vergleichsbereich der Testsequenz darstellt, welcher mit der restlichen Testsequenz auf übereinstimmende Sequenzabschnitte überprüft wird. Die Überprüfung der Testsequenzen auf übereinstimmende oder ähnliche Abschnitte des Vergleichsbereichs (vgl. Figur 3) mit der Dynamic Programming-Matrixtechnik ist in Figur 4a und 4b illustriert. Figur 4a zeigt den Fall, daß ähnliche oder übereinstimmende Sequenzabschnitte A und B in dem Vergleichsbereich selbst vorhanden sind. Figur 4b zeigt den Fall, daß ein Sequenzabschnitt B in dem Vergleichsbereich mit einem Sequenzabschnitt A außerhalb des Vergleichsbereichs übereinstimmt oder diesem ähnlich ist.

Als Alternative zu der Summation von Einzelgewichten kann auch vorgesehen sein, daß nur dasjenige Alignment, das zu dem höchsten Einzelgewicht führt oder, allgemeiner, nur die Alignments mit den m größten Einzelgewichten, berücksichtigt werden.

Mit der vorangehend beschriebenen Gewichtung können sowohl ähnliche Sequenzen, die z.B. am Anfang und am Ende der Testsequenz vorhanden sind, als auch sogenannte Tandem-Repeats, bei denen sich die ähnlichen Bereiche beide am Ende der Sequenz befinden, erfaßt werden.

Invers komplementäre Wiederholungen können in der gleichen Weise wie einfache Wiederholungen behandelt werden. Die potentielle Bildung von Sekundärstrukturen auf RNA-Ebene oder cruciformer Strukturen auf DNS-Ebene läßt sich an der Testsequenz durch das Vorhandensein solcher invers komplementärer Wiederholungen (inverse Repeats) erkennen. Cruciforme Strukturen auf DNS-Ebene können die Translation behindern und zu genetischer Instabilität führen. Man vermutet, daß die Bildung von Sekundärstrukturen auf RNA-Ebene sich negativ auf die Translationseffizienz auswirkt. Dabei sind insbesondere solche inverse Repeats von Bedeutung, die Haarnadelschleifen bzw. cruciforme Strukturen ausbilden. Fehlhybridisierungen oder Haarnadelschleifen können sich auch bei der Synthetisierung jener aus Oligonucleotiden negativ auswirken.

Die Überprüfung auf invers komplementäre Wiederholungen erfolgt vom Grundsatz her analog zur Überprüfung auf einfache Wiederholungen. Die Testsequenz bzw. der Vergleichsbereich der Testsequenz wird jedoch mit der invers komplementären Sequenz verglichen. In einer Fortbildung kann die thermodynamische Stabilität bei dem Vergleich ("alignment") berücksichtigt werden, im einfachsten Fall durch die Verwendung einer Scoring Matrix. Dabei wird z.B. ein Match CC bzw. GG aufgrund der stabileren Basenpaarung stärker gewichtet als eine Überweinstimmung TT oder AA. Entsprechend können auch Fehlstellen (mismatches) variabel gewichtet werden. Eine spezifischere Gewichtung kann dadurch erfolgen, daß Nearest-Neighbour-Parameter zur Berechnung der thermodynamischen Stabilität verwendet werden, was allerdings den Algorithmus komplexer macht. Hinsichtlich eines möglichen Algorithmus wird beispielsweise auf L. Kaderali, A. Schliep, Bioinformatics 18 (10) 2002, 1340 bis 1349 verwiesen.

Bei allen Bewertungskriterien kann die Erfindung vorsehen, daß die entsprechende Gewichtungsfunktion positionsabhängig ist. Beispielsweise kann die Generierung einer RE-Schnittsequenz an einer bestimmten Stelle stärker gewichtet werden oder Sekundärstrukturen können am 5'-Ende stärker gewichtet werden, da sie dort stärker inhibierend sind. Ebenso kann der Codonkontext, d.h. das oder die Vorgänger- bzw. Nachfolgerkodons, berücksichtigt werden.. Weiterhin kann für bestimmte Codons, deren Verwendung an den Domänengrenzen eine Rolle bei der cotranslatorischen Proteinfaltung spielt, ein Beitrag zur Gütefunktion vorgesehen sein, der davon abhängt, ob dieses Codon näher an der Domänengrenze liegt oder nicht. Weitere Kriterien, die in die Gütefunktion eingehen können, sind z.B. biophysikalische Eigenschaften, wie die Steifigkeit oder die Krümmung der DNS-Sequenz Je nach Anwendungsgebiet können auch Kriterien einfließen, die mit weiteren DNS-Sequenzen assoziiert sind. Beispielsweise ist im Bereich der DNS-Vakzinierung entscheidend, daß die für die Vakzinierung verwendeten Sequenzen keine signifikante Ähnlichkeit mit den pathogenen Elementen des natürlichen Virusgenoms aufweisen, um unerwünschte Rekombinationsereignisse sicher auszuschließen. In gleicher Weise sollten die für gentherapeutische Zwecke verwendeten Vektoren eine möglichst geringe Ähnlichkeit zu Sequenzen des menschlichen Genoms aufweisen, um einerseits homologe Rekombination in das menschliche Genom auszuschließen und andererseits ein selektives Abschalten von vitalen Genen in Transkriptom durch RNA-Interferenz-Phänomene (RNAI - Phänomene) zu vermeiden. Letzteres ist auch von allgemeiner Bedeutung bei der Herstellung von rekombinanten Zellfabriken und insbesondere bei transgenen Organismen.

Erfindungsgemäß können die verschiedenen Kriteriumsgewichte für verschiedene Kriterien unterschiedlich in die Gesamtgewichtsfunktion eingehen. Dabei ist der durch das entsprechende Kriterium maximal erreichbare Unterschied in dem Wert der Gütefunktion für die gebildete Testsequenz wichtig. Einen hohen Anteil an bestimmten Kriteriumsgewichten haben jedoch DNA-Basen, welche durch unterschiedliche KDS nicht geändert werden können, wie z.B. die in die Berechnung des durchschnittlichen GC-Gehalts miteinbezogenen Nucleotide vor der KDS und die innerhalb synonymer Codons unveränderlichen Nucleotide Die individuelle Gewichtung eines Kriteriums gegenüber anderen Kriterien kann daher davon abhängig gemacht werden, wie stark die Güte der Testsequenz von der Zielvorgabe abweicht. Es kann sinnvoll sein, die Kriteriumsgewichte zur weiteren Verarbeitung in mathematischen Funktionen zu Berechnung der Gütefunktion aufzuspalten in einen Teil, der den bei Verwendung unterschiedlicher KDS variablen Anteil eines Kriteriums bemißt und einen Teil, der die unveränderlichen Anteile bemißt.

Die vorangehend beschriebenen Ausführungsformen der Erfindung werden nachfolgend anhand zweier konkreter Beispiele weiter erläutert.

**Beispiel 1**

Zu der nachfolgend gezeigten (fiktiven) Aminosäuresequenz ASSeql soll die zugehörige optimale DNS-Sequenz ermittelt werden. Als Referenz dient eine konventionelle Rückübersetzung mit Optimierung auf optimale Codon-Usage.

**ASSeq1:**

| 1 | 2 | 3 | 4 | 5 | 6 | 7 | 8 | 9 | 10 | 11 | 12 | 13 | 14 |
|---|---|---|---|---|---|---|---|---|---|---|---|---|---|
| E__ | Q__ | F__ | I__ | I__ | K__ | N__ | M__ | F__ | I__ | I__ | K__ | N__ | A__ |
| GAA | CAG | TTT | ATT | ATT | AAA | AAC | ATG | TTT | ATT | ATT | AAA | AAC | GCG |
| GAG | CAA | TTC | ATC | ATC | AAG | AAT | | TTC | ATC | ATC | AAG | AAT | GCC |
| | | | ATA | ATA | | | | | ATA | ATA | | | GCA |
| | | | | | | | | | | | | | GCT |

Folgende Kriterien werden der Optimierung zugrunde gelegt:

- Die Codon usage soll auf die Codon usage von E. Coli K12 optimiert werden.
- Der GC - Gehalt soll möglichst nahe bei 50 % liegen.
- Repetitionen sollen möglichst ausgeschlossen werden
- Die Nla III Erkennungssequenz CATG soll ausgeschlossen werden

Als Bewertungsfunktion für die Codon usage wird folgende Funktion verwendet:

$$CUScore = \langle CU \rangle$$

wobei $\langle CU \rangle$ bei diesem Beispiel das arithmetische Mittel der Relative adaptiveness über den Codonpositionen der Testsequenz ist.

Zur Darstellung der Codon usage eines Codons wird zur besseren Vergleichbarkeit der Codongüte verschiedener Aminosäuren das jeweils beste Codon für eine bestimmte Aminosäure gleich 100 gesetzt und die schlechteren Codons entsprechend ihrem tabellierten prozentualen Anteil reskaliert. Ein CUScore von 100 bedeutet also, daß ausschließlich die für E. Coli K12 optimalen Codons verwendet werden.

Das Gewicht für den prozentualen GC-Gehalt wird wie folgt berechnet:

$$GCScore = \left| \langle GC \rangle - GC_{Wunsch} \right|^{1.3} \times 0{,}8$$

Zur Ermittlung der Einzelgewichte der Alignments (Alignmentscore) wird ein optimales lokales Alignment der Testsequenz mit einem Teilbereich der Testsequenz, der maximal die letzten 36 Basen der kompletten Testsequenz umfasst, unter Ausschluss des Identitätsalignments (Alignment des vollständigen Teilbereiches mit sich selbst) generiert (vgl. Fig. 3, 4a, 4b).

Als Bewertungsparameter für eine Basenposition zur Berechnung der Dynamic-Programming Matrix werden dabei verwendet:

Übereinstimmung (Match) = 1;
Fehlpaarung (Mismatch) = -2;
Lücke (Gap) = -2.

Das entsprechende Kriteriumsgewicht wird durch eine Potenz des optimalen Alignment-Scores in dem überprüften Bereich der Testsequenz festgelegt:

$$REPScore = \left(Score_{Alignment}\right)^{1.3}$$

Für jede gefundene CATG-Sequenz wird ein Sitescore von 100000 vergeben.

Die Gesamtgütefunktion GesScore ergibt sich

$$GesScore = CUScore - GCScore - REPScore - SiteScore$$

Die KDS-Länge m beträgt 3 Codons (9 Basen).

Eine Optimierung lediglich auf optimale Codon-Usage resultiert in folgender Sequenz:

| | | | | | | | | | | | | | |
|---|---|---|---|---|---|---|---|---|---|---|---|---|---|
| 1 | 2 | 3 | 4 | 5 | 6 | 7 | 8 | 9 | 10 | 11 | 12 | 13 | 14 |
| E__ | Q__ | F__ | I__ | I__ | K__ | N__ | M__ | F__ | I__ | I__ | K__ | N__ | A__ |
| **GAA** | **CAG** | **TTT** | **ATT** | **ATT** | **AAA** | **AAC** | **ATG** | **TTT** | **ATT** | **ATT** | **AAA** | **AAC** | **GCG** |

Sie ist durch folgende Eigenschaften charakterisiert:

- Stark repetitiv, verursacht durch die zweimalig erscheinende Aminosäuresequenz F_I_I_K_N (gezeigt ist das repetitive Element mit dem höchsten Score (18)):

```
19 AACATGTTTATTATTAAAAAC
   |||| ||||||||||||||||
 2 AACA-GTTTATTATTAAAAAC
```

- GC-Gehalt: 21,4 %
- Die Nla III Erkennungssequenz CATG ist vorhanden
- Durchschnittliche Codon-Usage: 100

Wird die Optimierung nach dem erfindungsgemäßen Algorithmus mit den oben genannten Bewertungsfunktionen und Parametern vorgenommen, so erhält man folgende DNS-Sequenz:

| 1 | 2 | 3 | 4 | 5 | 6 | 7 | 8 | 9 | 10 | 11 | 12 | 13 | 14 |
|---|---|---|---|---|---|---|---|---|---|---|---|---|---|
| E__ | Q__ | F__ | I__ | I__ | K__ | N__ | M__ | F__ | I__ | I__ | K__ | N__ | A__ |
| **GAA** | **CAG** | **TTC** | **ATC** | **ATC** | **AAA** | **AAT** | **ATG** | **TTT** | **ATT** | **ATC** | **AAG** | **AAC** | **GCG** |

Sie ist durch folgende Eigenschaften charakterisiert:

- Kaum repetitiv (das nachfolgend gezeigte Alignment mit dem höchsten Beitrag hat einen Score 6)

```
11 TCATCA
   ||||||
 8 TCATCA
```

- GC-Gehalt: 31,0 %
- Die Nla III Erkennungssequenz CATG ist vermieden worden
- Durchschnittliche Codon-Usage: 88

Bei dem erfindungsgemäßen Optimierungsergebnis wurde an fünf Aminosäure-Positionen nicht das hinsichtlich der Codon usage optimale Codon gewählt. Die erfindungsgemäße aufgefundene Sequenz stellt jedoch eine optimale Balance der unterschiedlichen Anforderungen in Bezug auf Codon-Usage, GC-Gehalt und ideale Sequenzeigenschaften (Vermeidung von Repetitionen) dar.

Bei den Aminosäuren mit den Nummern 3,4,5 ist der höhere GC-Anteil der hinsichtlich der Codon usage schlechteren Codons der Grund für die Wahl. An Position 6 überwiegt jedoch beim Vergleich der Codons AAA und AAG die wesentlich bessere Codon usage des AAA Codons, obwohl die Wahl des AAG Codons zu einem besseren GC-Score führen würde. Bei Bildung der KDS an Basenposition 13 wird für die Aminosäure Nr. 7 noch das Codon AAC bevorzugt, da bei einer Fenstergröße für die KDS von 3 Codons noch nicht erkennbar ist, daß diese Wahl zu Bildung des zu vermeidenden DNS-Motivs CATG führen wird (für Methionin ist der genetische Code nicht degeneriert, d.h. es gibt nur ein Codon zur Expression von Methionin). Bei der Bildung der KDS an Basenposition 16 wird dies jedoch bereits erkannt und folgerichtig das Codon AAT gewählt. Bei der Wahl der Codons für die Aminosäuren 9 bis 13 spielt neben Codon-Usage und GC-Gehalt auch die Vermeidung einer repetitiven DNS-Sequenz. Aufgrund der identischen Aminosäuresequenzen der Aminosäuren Nr. 3 bis 7 und 9 bis 13 eine entscheidende Rolle. Aus diesem Grunde werden für die Aminosäuren 9 und 10 im Gegensatz zu vorher (Asr. 3,4) die Codons TTT und ATT bevorzugt.

Die nachfolgende Tabelle illustriert die einzelnen Schritte des Algorithmus, die zu dem oben angegebenen Optimierungsergebnis geführt haben. Sie ermöglicht es, den Ablauf des Algorithmus Schritt für Schritt nachzuvollziehen. Für jede Startposition werden dabei detailliert alle von der Software gebildeten Kombinations-DNS-Sequenzen (KDS) aufgelistet.

Zu jeder möglichen KDS werden folgende Angaben gemacht:

- die aus der jeweiligen KDS und der bereits optimierten DNS-Sequenz gebildete Testsequenz, welche zur Evaluierung der KDS herangezogen wird,

- die Scores, welche für Codon usage, GC-Gehalt, Repetitivität und aufgefundenen DNS-Sites ermittelt wurden (CU, GC, Rep, Site)
- das für die jeweilige Testsequenz ermittelte repetitive Element mit dem höchsten Alignment-Score,
- der ermittelte Gesamtscore.

Die KDS sind dabei nach fallendem Gesamtscore sortiert, d.h. das erste Codon der ersten gezeigten KDS wird an die bereits optimierte DNS-Sequenz angefügt.

KDS-Startposition 1 bei Aminosäure 1 E

| KDS<br>Testsequenz | CU | GC | Site | Rep | Alignment | Gesamt-Score |
|---|---|---|---|---|---|---|
| GAACAGTTC<br>GAACAGTTC | 92 | 5 | 0 | 0,0 | G / G | 87,0 |
| GAACAGTTT<br>GAACAGTTT | 100 | 19 | 0 | 0,0 | TT / TT | 81,0 |
| GAGCAGTTT<br>GAGCAGTTT | 82 | 5 | 0 | 0,0 | AG / AG | 77,0 |
| GAGCAGTTC<br>GAGCAGTTC | 73 | 5 | 0 | 0,0 | AG / AG | 68,0 |
| GAACAATTC<br>GAACAATTC | 76 | 19 | 0 | 0,0 | AA / AA | 57,0 |
| GAGCAATTC<br>GAGCAATTC | 58 | 5 | 0 | 0,0 | G / G | 53,0 |
| GAACAATTT<br>GAACAATTT | 85 | 38 | 0 | 0,0 | AA / AA | 47,0 |
| GAGCAATTT<br>GAGCAATTT | 66 | 19 | 0 | 0,0 | TT / TT | 47,0 |

KDS-Startposition 4 bei Aminosäure 2 Q

| KDS<br>Testsequenz | CU | GC | Site | Rep | Alignment | Gesamt-Score |
|---|---|---|---|---|---|---|
| CAGTTCATC<br>GAACAGTTCATC | 86 | 8 | 0 | 0,0 | CA / CA | 78,0 |
| CAGTTTATC<br>GAACAGTTTATC | 94 | 19 | 0 | 0,0 | TT / TT | 75,0 |
| CAGTTCATT<br>GAACAGTTCATT | 92 | 19 | 0 | 0,0 | CA / CA | 73,0 |
| CAGTTTATT<br>GAACAGTTTATT | 100 | 33 | 0 | 0,0 | TT / TT | 67,0 |
| CAATTCATC<br>GAACAATTCATC | 70 | 19 | 0 | 0,0 | AA / AA | 51,0 |
| CAATTTATC<br>GAACAATTTATC | 79 | 33 | 0 | 0,0 | AA / AA | 46,0 |
| CAGTTCATA<br>GAACAGTTCATA | 63 | 19 | 0 | 0,0 | CA / CA | 44,0 |
| CAATTCATT<br>GAACAATTCATT | 76 | 33 | 0 | 0,0 | ATT / ATT | 43,0 |
| CAGTTTATA<br>GAACAGTTTATA | 71 | 33 | 0 | 0,0 | TT / TT | 38,0 |
| CAATTTATT<br>GAACAATTTATT | 85 | 48 | 0 | 0,0 | ATT / ATT | 37,0 |
| CAATTCATA<br>GAACAATTCATA | 48 | 33 | 0 | 0,0 | AA / AA | 15,0 |
| CAATTTATA<br>GAACAATTTATA | 56 | 48 | 0 | 0,0 | AA / AA | 8,0 |

KDS-Startposition 7 bei Aminosäure 3 F

| KDS<br>Testsequenz | CU | GC | Site | Rep | Alignment | Gesamt-Score |
|---|---|---|---|---|---|---|
| TTCATCATC<br>GAACAGTTCATCATC | 80 | 10 | 0 | 0,0 | TCATC / TCATC | 70,0 |

| | | | | | | |
|---|---|---|---|---|---|---|
| TTTATCATC<br>GAACAGTTTATCATC | 88 | 19 | 0 | 0,0 | ATC / ATC | 69,0 |
| TTCATTATC<br>GAACAGTTCATTATC | 86 | 19 | 0 | 0,0 | CA / CA | 67,0 |
| TTCATCATT<br>GAACAGTTCATCATT | 86 | 19 | 0 | 0,0 | TCAT / TCAT | 67,0 |
| TTTATTATC<br>GAACAGTTTATTATC | 94 | 30 | 0 | 0,0 | TTAT / TTAT | 64,0 |
| TTTATCATT<br>GAACAGTTTATCATT | 94 | 30 | 0 | 0,0 | CA / CA | 64,0 |
| TTCATTATT<br>GAACAGTTCATTATT | 92 | 30 | 0 | 0,0 | ATT / ATT | 62,0 |
| TTTATTATT<br>GAACAGTTTATTATT | 100 | 42 | 0 | 0,0 | TTATT / TTATT | 58,0 |
| TTCATCATA<br>GAACAGTTCATCATA | 57 | 19 | 0 | 0,0 | TCAT / TCAT | 38,0 |
| TTCATAATC<br>GAACAGTTCATAATC | 57 | 19 | 0 | 0,0 | AA / AA | 38,0 |
| TTTATCATA<br>GAACAGTTTATCATA | 65 | 30 | 0 | 0,0 | CA / CA | 35,0 |
| TTTATAATC<br>GAACAGTTTATAATC | 65 | 30 | 0 | 0,0 | AA / AA | 35,0 |
| TTCATTATA<br>GAACAGTTCATTATA | 63 | 30 | 0 | 0,0 | CA / CA | 33,0 |
| TTCATAATT<br>GAACAGTTCATAATT | 63 | 30 | 0 | 0,0 | AA / AA | 33,0 |
| TTTATTATA<br>GAACAGTTTATTATA | 71 | 42 | 0 | 0,0 | TTAT / TTAT | 29,0 |
| TTTATAATT<br>GAACAGTTTATAATT | 71 | 42 | 0 | 0,0 | AA / AA | 29,0 |
| TTCATAATA<br>GAACAGTTCATAATA | 34 | 30 | 0 | 0,0 | ATA / ATA | 4,0 |
| TTTATAATA<br>GAACAGTTTATAATA | 43 | 42 | 0 | 0,0 | ATA / ATA | 1,0 |

KDS-Startposition 10 bei Aminosäure 4 I

| KDS<br>Testsequenz | CU | GC | Site | Rep | Alignment | Gesamt-Score |
|---|---|---|---|---|---|---|
| ATCATCAAA<br>GAACAGTTCATCATCAAA | 88 | 19 | 0 | 0,0 | TCATCA / TCATCA | 69,0 |
| ATTATCAAA<br>GAACAGTTCATTATCAAA | 94 | 28 | 0 | 0,0 | TCA / TCA | 66,0 |
| ATCATTAAA<br>GAACAGTTCATCATTAAA | 94 | 28 | 0 | 0,0 | TCAT / TCAT | 66,0 |
| ATTATTAAA<br>GAACAGTTCATTATTAAA | 100 | 38 | 0 | 0,0 | ATTA / ATTA | 62,0 |
| ATCATCAAG<br>GAACAGTTCATCATCAAG | 65 | 11 | 0 | 0,0 | TCATCA / TCATCA | 54,0 |
| ATTATCAAG<br>GAACAGTTCATTATCAAG | 71 | 19 | 0 | 0,0 | TCA / TCA | 52,0 |
| ATCATTAAG<br>GAACAGTTCATCATTAAG | 71 | 19 | 0 | 0,0 | TCAT / TCAT | 52,0 |
| ATTATTAAG<br>GAACAGTTCATTATTAAG | 77 | 28 | 0 | 0,0 | ATTA / ATTA | 49,0 |

| | | | | | | |
|---|---|---|---|---|---|---|
| ATCATAAAA<br>GAACAGTTCATCATAAAA | 65 | 28 | 0 | 0,0 | TCAT<br>TCAT | 37,0 |
| ATAATCAAA<br>GAACAGTTCATAATCAAA | 65 | 28 | 0 | 0,0 | TCA<br>TCA | 37,0 |
| ATTATAAAA<br>GAACAGTTCATTATAAAA | 71 | 38 | 0 | 0,0 | AAA<br>AAA | 33,0 |
| ATAATTAAA<br>GAACAGTTCATAATTAAA | 71 | 38 | 0 | 0,0 | TAA<br>TAA | 33,0 |
| ATCATAAAG<br>GAACAGTTCATCATAAAG | 43 | 19 | 0 | 0,0 | TCAT<br>TCAT | 24,0 |
| ATAATCAAG<br>GAACAGTTCATAATCAAG | 43 | 19 | 0 | 0,0 | TCA<br>TCA | 24,0 |
| ATTATAAAG<br>GAACAGTTCATTATAAAG | 49 | 28 | 0 | 0,0 | AA<br>AA | 21,0 |
| ATAATTAAG<br>GAACAGTTCATAATTAAG | 49 | 28 | 0 | 0,0 | TAA<br>TAA | 21,0 |
| ATAATAAAA<br>GAACAGTTCATAATAAAA | 43 | 38 | 0 | 0,0 | ATAA<br>ATAA | 5,0 |
| ATAATAAAG<br>GAACAGTTCATAATAAAG | 20 | 28 | 0 | 0,0 | ATAA<br>ATAA | -8,0 |

KDS-Startposition 13 bei Aminosäure 5 I

| KDS<br>Testsequenz | CU | GC | Site | Rep | Alignment | Gesamt-Score |
|---|---|---|---|---|---|---|
| ATCAAAAAC<br>GAACAGTTCATCATCAAAAAC | 94 | 19 | 0 | 0,0 | TCATCA<br>TCATCA | 75,0 |
| ATTAAAAAC<br>GAACAGTTCATCATTAAAAAC | 100 | 27 | 0 | 0,0 | TCAT<br>TCAT | 73,0 |
| ATCAAAAAT<br>GAACAGTTCATCATCAAAAAT | 88 | 27 | 0 | 0,0 | TCATCA<br>TCATCA | 61,0 |
| ATTAAAAAT<br>GAACAGTTCATCATTAAAAAT | 94 | 35 | 0 | 0,0 | TCAT<br>TCAT | 59,0 |
| ATTAAGAAC<br>GAACAGTTCATCATTAAGAAC | 77 | 19 | 0 | 0,0 | GAAC<br>GAAC | 58,0 |
| ATCAAGAAC<br>GAACAGTTCATCATCAAGAAC | 71 | 13 | 0 | 0,0 | TCATCA<br>TCATCA | 58,0 |
| ATCAAGAAT<br>GAACAGTTCATCATCAAGAAT | 65 | 19 | 0 | 0,0 | TCATCA<br>TCATCA | 46,0 |
| ATTAAGAAT<br>GAACAGTTCATCATTAAGAAT | 71 | 27 | 0 | 0,0 | TCAT<br>TCAT | 44,0 |
| ATAAAAAAC<br>GAACAGTTCATCATAAAAAAC | 71 | 27 | 0 | 0,0 | TCAT-A-AAAAA<br>TCATCATAAAAA | 44,0 |
| ATAAAAAAT<br>GAACAGTTCATCATAAAAAAT | 65 | 35 | 0 | 0,0 | TCAT-A-AAAAA<br>TCATCATAAAAA | 30,0 |
| ATAAAGAAC<br>GAACAGTTCATCATAAAGAAC | 49 | 19 | 0 | 0,0 | GAAC<br>GAAC | 30,0 |
| ATAAAGAAT<br>GAACAGTTCATCATAAAGAAT | 43 | 27 | 0 | 0,0 | TCAT<br>TCAT | 16,0 |

KDS-Startposition 16 bei Aminosäure 6 K

| KDS<br>Testsequenz | CU | GC | Site | Rep | Alignment | Gesamt-Score |
|---|---|---|---|---|---|---|
| AAAAATATG<br>GAACAGTTCATCATCAAAAATATG | 94 | 26 | 0 | 0,0 | TCATCA<br>TCATCA | 68,0 |

| KDS<br>Testsequenz | CU | GC | Site | Rep | Alignment | Gesamt-Score |
|---|---|---|---|---|---|---|
| AAGAATATG<br>GAACAGTTCATCATCAAGAATATG | 71 | 19 | 0 | 0,0 | TCATCA<br>TCATCA | 52,0 |
| AAAAACATG<br>GAACAGTTCATCATCAAAAACATG | 100 | 19 | 200000 | 0,0 | TCATCA<br>TCATCA | 919,0 |
| AAGAACATG<br>GAACAGTTCATCATCAAGAACATG | 77 | 13 | 200000 | 0,0 | TCATCA<br>TCATCA | 936,0 |

KDS-Startposition 19 bei Aminosäure 7 N

| KDS<br>Testsequenz | CU | GC | Site | Rep | Alignment | Gesamt-Score |
|---|---|---|---|---|---|---|
| AATATGTTT<br>GAACAGTTCATCATCAAAAATATGTTT | 94 | 35 | 0 | 0,0 | TCATCA<br>TCATCA | 59,0 |
| AATATGTTC<br>GAACAGTTCATCATCAAAAATATGTTC | 86 | 28 | 0 | 0,0 | TCATCA<br>TCATCA | 58,0 |
| AACATGTTT<br>GAACAGTTCATCATCAAAAACATGTTT | 100 | 28 | 200000 | 0,0 | TCATCA<br>TCATCA | 928,0 |
| AACATGTTC<br>GAACAGTTCATCATCAAAAACATGTTC | 92 | 21 | 200000 | 0,0 | AACATGTTC<br>AACA-GTTC | 929,0 |

KDS-Startposition 22 bei Aminosäure 8 M

| KDS<br>Testsequenz | CU | GC | Site | Rep | Alignment | Gesamt-Score |
|---|---|---|---|---|---|---|
| ATGTTTATC<br>GAACAGTTCATCATCAAAAATATGTTTATC | 94 | 35 | 0 | 0,0 | TCATCA<br>TCATCA | 59,0 |
| ATGTTTATT<br>GAACAGTTCATCATCAAAAATATGTTTATT | 100 | 42 | 0 | 0,0 | TCATCA<br>TCATCA | 58,0 |
| ATGTTCATT<br>GAACAGTTCATCATCAAAAATATGTTCATT | 92 | 35 | 0 | 0,0 | GTTCAT<br>GTTCAT | 57,0 |
| ATGTTCATC<br>GAACAGTTCATCATCAAAAATATGTTCATC | 86 | 28 | 0 | 12,5 | GTTCATC<br>GTTCATC | 45,0 |
| ATGTTTATA<br>GAACAGTTCATCATCAAAAATATGTTTATA | 71 | 42 | 0 | 0,0 | TCATCA<br>TCATCA | 29,0 |
| ATGTTCATA<br>GAACAGTTCATCATCAAAAATATGTTCATA | 63 | 35 | 0 | 0,0 | GTTCAT<br>GTTCAT | 28,0 |

KDS-Startposition 25 bei Aminosäure 9 F

| KDS<br>Testsequenz | CU | GC | Site | Rep | Alignment | Gesamt-Score |
|---|---|---|---|---|---|---|
| TTTATTATC<br>GAACAGTTCATCATCAAAAATATGTTTATTATC | 94 | 42 | 0 | 0,0 | TCATCA<br>TCATCA | 52,0 |
| TTTATCATT<br>GAACAGTTCATCATCAAAAATATGTTTATCATT | 94 | 42 | 0 | 0,0 | TCATCA<br>TCATCA | 52,0 |
| TTCATTATT<br>GAACAGTTCATCATCAAAAATATGTTCATTATT | 92 | 42 | 0 | 0,0 | GTTCAT<br>GTTCAT | 50,0 |
| TTTATCATC<br>GAACAGTTCATCATCAAAAATATGTTTATCATC | 88 | 35 | 0 | 12,5 | GTTTATCATC<br>GTTCATCATC | 40,0 |
| TTTATTATT<br>GAACAGTTCATCATCAAAAATATGTTTATTATT | 100 | 49 | 0 | 12,5 | TCATCA--AAAATATGTTTATTATT<br>TCATCATCAAAAA-ATATGT-TTATT | 38,0 |
| TTCATTATC<br>GAACAGTTCATCATCAAAAATATGTTCATTATC | 86 | 35 | 0 | 12,5 | GTTCATTATC<br>GTTCATCATC | 38,0 |
| TTCATCATT<br>GAACAGTTCATCATCAAAAATATGTTCATCATT | 86 | 35 | 0 | 17,4 | GTTCATCAT<br>GTTCATCAT | 34,0 |

| | | | | | | |
|---|---|---|---|---|---|---|
| TTCATCATC<br>GAACAGTTCATCATCAAAAATATGTTCATCATC | 80 | 28 | 0 | 20,0 | GTTCATCATC<br>GTTCATCATC | 32,0 |
| TTTATCATA<br>GAACAGTTCATCATCAAAAATATGTTTATCATA | 65 | 42 | 0 | 0,0 | TCATCA<br>TCATCA | 23,0 |
| TTTATAATC<br>GAACAGTTCATCATCAAAAATATGTTTATAATC | 65 | 42 | 0 | 0,0 | TCATCA<br>TCATCA | 23,0 |
| TTTATTATA<br>GAACAGTTCATCATCAAAAATATGTTTATTATA | 71 | 49 | 0 | 0,0 | TCATCA<br>TCATCA | 22,0 |
| TTTATAATT<br>GAACAGTTCATCATCAAAAATATGTTTATAATT | 71 | 49 | 0 | 0,0 | TCATCA<br>TCATCA | 22,0 |
| TTCATAATT<br>GAACAGTTCATCATCAAAAATATGTTCATAATT | 63 | 42 | 0 | 0,0 | GTTCAT<br>GTTCAT | 21,0 |
| TTCATTATA<br>GAACAGTTCATCATCAAAAATATGTTCATTATA | 63 | 42 | 0 | 0,0 | GTTCAT<br>GTTCAT | 21,0 |
| TTCATAATC<br>GAACAGTTCATCATCAAAAATATGTTCATAATC | 57 | 35 | 0 | 12,5 | GTTCATAATC<br>GTTCATCATC | 9,0 |
| TTCATCATA<br>GAACAGTTCATCATCAAAAATATGTTCATCATA | 57 | 35 | 0 | 17,4 | GTTCATCAT<br>GTTCATCAT | 5,0 |
| TTTATAATA<br>GAACAGTTCATCATCAAAAATATGTTTATAATA | 43 | 49 | 0 | 0,0 | TCATCA<br>TCATCA | -6,0 |
| TTCATAATA<br>GAACAGTTCATCATCAAAAATATGTTCATAATA | 34 | 42 | 0 | 0,0 | GTTCAT<br>GTTCAT | -8,0 |

KDS-Startposition 28 bei Aminosäure - 10 I

| KDS<br>Testsequenz | CU | GC | Site | Rep | Alignment | Gesamt-Score |
|---|---|---|---|---|---|---|
| ATTATCAAA<br>GAACAGTTCATCATCAAAAATATGTTTATTATCAAA | 94 | 49 | 0 | 12,5 | GTTTATTATCAAA<br>GTTCATCATCAAA | 32,0 |
| ATCATTAAA<br>GAACAGTTCATCATCAAAAATATGTTTATCATTAAA | 94 | 49 | 0 | 12,5 | GTTTATCATTAAA<br>GTTCATCATCAAA | 32,0 |
| ATTATCAAG<br>GAACAGTTCATCATCAAAAATATGTTTATTATCAAG | 71 | 42 | 0 | 0,0 | TCATCA<br>TCATCA | 29,0 |
| ATCATTAAG<br>GAACAGTTCATCATCAAAAATATGTTTATCATTAAG | 71 | 42 | 0 | 0,0 | TCATCA<br>TCATCA | 29,0 |
| ATTATTAAA<br>GAACAGTTCATCATCAAAAATATGTTTATTATTAAA | 100 | 57 | 0 | 14,9 | TCATCA--AAAATATGTTTATTATTA<br>TCATCATCAAAA-ATATGT-TTATTA | 28,0 |
| ATCATCAAA<br>GAACAGTTCATCATCAAAAATATGTTTATCATCAAA | 88 | 42 | 0 | 20,0 | GTTTATCATCAAA<br>GTTCATCATCAAA | 26,0 |
| ATTATAAAA<br>GAACAGTTCATCATCAAAAATATGTTTATTATAAAA | 71 | 57 | 0 | 0,0 | TCATCA<br>TCATCA | 14,0 |
| ATAATTAAA<br>GAACAGTTCATCATCAAAAATATGTTTATAATTAAA | 71 | 57 | 0 | 0,0 | TCATCA<br>TCATCA | 14,0 |
| ATTATTAAG<br>GAACAGTTCATCATCAAAAATATGTTTATTATTAAG | 77 | 49 | 0 | 14,9 | TCATCA--AAAATATGTTTATTATTA<br>TCATCATCAAAA-ATATGT-TTATTA | 13,0 |
| ATCATCAAG<br>GAACAGTTCATCATCAAAAATATGTTTATCATCAAG | 65 | 35 | 0 | 17,4 | GTTTATCATCAA<br>GTTCATCATCAA | 13,0 |
| ATAATCAAA<br>GAACAGTTCATCATCAAAAATATGTTTATAATCAAA | 65 | 49 | 0 | 12,5 | GTTTATAATCAAA<br>GTTCATCATCAAA | 3,0 |
| ATCATAAAA<br>GAACAGTTCATCATCAAAAATATGTTTATCATAAAA | 65 | 49 | 0 | 14,9 | GTTTATCAT-AAAA<br>GTTCATCATCAAAA | 1,0 |
| ATAATCAAG<br>GAACAGTTCATCATCAAAAATATGTTTATAATCAAG | 43 | 42 | 0 | 0,0 | TCATCA<br>TCATCA | 1,0 |
| ATTATAAAG<br>GAACAGTTCATCATCAAAAATATGTTTATTATAAAG | 49 | 49 | 0 | 0,0 | TCATCA<br>TCATCA | 0,0 |

| | | | | | | |
|---|---|---|---|---|---|---|
| ATAATTAAG<br>GAACAGTTCATCATCAAAAATATGTTTATAATTAAG | 49 | 49 | 0 | 0,0 | TCATCA<br>TCATCA | 0,0 |
| ATCATAAAG<br>GAACAGTTCATCATCAAAAATATGTTTATCATAAAG | 43 | 42 | 0 | 12,5 | GTTTATCAT-AAA<br>GTTCATCATCAAA | -12,0 |
| ATAATAAAA<br>GAACAGTTCATCATCAAAAATATGTTTATAATAAAA | 43 | 57 | 0 | 0,0 | TCATCA<br>TCATCA | -14,0 |
| ATAATAAAG<br>GAACAGTTCATCATCAAAAATATGTTTATAATAAAG | 20 | 49 | 0 | 0,0 | TCATCA<br>TCATCA | -29,0 |

KDS-Startposition 31 bei Aminosäure 11 I

| KDS<br>Testsequenz | CU | GC | Site | Rep | Alignment | Gesamt-Score |
|---|---|---|---|---|---|---|
| ATCAAGAAC<br>GAACAGTTCATCATCAAAAATATGTTTATTATCAAGAAC | 71 | 42 | 0 | 0,0 | TCATCA<br>TCATCA | 29,0 |
| ATTAAAAAC<br>GAACAGTTCATCATCAAAAATATGTTTATTATTAAAAAC | 100 | 57 | 0 | 14,9 | TCATCA--AAAATATGTTTATTATTA<br>TCATCATCAAAA-ATATGT-TTATTA | 28,0 |
| ATCAAAAAC<br>GAACAGTTCATCATCAAAAATATGTTTATTATCAAAAAC | 94 | 49 | 0 | 17,4 | GTTTATTATCAAAAA<br>GTTCATCATCAAAAA | 28,0 |
| ATTAAAAAT<br>GAACAGTTCATCATCAAAAATATGTTTATTATTAAAAAT | 94 | 64 | 0 | 14,9 | TCATCA--AAAATATGTTTATTATTA<br>TCATCATCAAAA-ATATGT-TTATTA | 15,0 |
| ATTAAGAAC<br>GAACAGTTCATCATCAAAAATATGTTTATTATTAAGAAC | 77 | 49 | 0 | 14,9 | TCATCA--AAAATATGTTTATTATTA<br>TCATCATCAAAA-ATATGT-TTATTA | 13,0 |
| ATCAAAAAT<br>GAACAGTTCATCATCAAAAATATGTTTATTATCAAAAAT | 88 | 57 | 0 | 20,0 | GTTTATTATCAAAAAT<br>GTTCATCATCAAAAAT | 11,0 |
| ATCAAGAAT<br>GAACAGTTCATCATCAAAAATATGTTTATTATCAAGAAT | 65 | 49 | 0 | 12,5 | GTTTATTATCAAGAAT<br>GTTCATCATCAAAAAT | 3,0 |
| ATAAAGAAC<br>GAACAGTTCATCATCAAAAATATGTTTATTATAAAGAAC | 49 | 49 | 0 | 0,0 | TCATCA<br>TCATCA | 0,0 |
| ATTAAGAAT<br>GAACAGTTCATCATCAAAAATATGTTTATTATTAAGAAT | 71 | 57 | 0 | 14,9 | TCATCA--AAAATATGTTTATTATTA<br>TCATCATCAAAA-ATATGT-TTATTA | -1,0 |
| ATAAAAAAC<br>GAACAGTTCATCATCAAAAATATGTTTATTATAAAAAAC | 71 | 57 | 0 | 14,9 | TCATCA--AAAATATGTTTATTA-TA-AAAAA<br>TCATCATCAAAA-ATATGT-TTATTATAAAAA | -1,0 |
| ATAAAAAAT<br>GAACAGTTCATCATCAAAAATATGTTTATTATAAAAAAT | 65 | 64 | 0 | 14,9 | TCATCA--AAAATATGTTTATTA-TA-AAAAA<br>TCATCATCAAAA-ATATGT-TTATTATAAAAA | -14,0 |
| ATAAAGAAT<br>GAACAGTTCATCATCAAAAATATGTTTATTATAAAGAAT | 43 | 57 | 0 | 0,0 | TCATCA<br>TCATCA | -14,0 |

KDS-Startposition 34 bei Aminosäure 12 K

| KDS<br>Testsequenz | CU | GC | Site | Rep | Alignment | Gesamt-Score |
|---|---|---|---|---|---|---|
| AAGAACGCG<br>GAACAGTTCATCATCAAAAATATGTTTATTATCAAGAACGCG | 77 | 28 | 0 | 0,0 | TCATCA<br>TCATCA | 49,0 |
| AAAAACGCG<br>GAACAGTTCATCATCAAAAATATGTTTATTATCAAAAACGCG | 100 | 35 | 0 | 17,4 | GTTTATTATCAAAAA<br>GTTCATCATCAAAAA | 48,0 |
| AAGAACGCC<br>GAACAGTTCATCATCAAAAATATGTTTATTATCAAGAACGCC | 69 | 28 | 0 | 0,0 | TCATCA<br>TCATCA | 41,0 |
| AAAAACGCC<br>GAACAGTTCATCATCAAAAATATGTTTATTATCAAAAACGCC | 92 | 35 | 0 | 17,4 | GTTTATTATCAAAAA<br>GTTCATCATCAAAAA | 40,0 |
| AAAAATGCG<br>GAACAGTTCATCATCAAAAATATGTTTATTATCAAAAATGCG | 94 | 42 | 0 | 20,0 | GTTTATTATCAAAAAT<br>GTTCATCATCAAAAAT | 32,0 |
| AAGAACGCA<br>GAACAGTTCATCATCAAAAATATGTTTATTATCAAGAACGCA | 63 | 35 | 0 | 0,0 | TCATCA<br>TCATCA | 28,0 |
| AAAAACGCA<br>GAACAGTTCATCATCAAAAATATGTTTATTATCAAAAACGCA | 86 | 42 | 0 | 17,4 | GTTTATTATCAAAAA<br>GTTCATCATCAAAAA | 27,0 |

| | | | | | | |
|---|---|---|---|---|---|---|
| AAAAATGCC<br>GAACAGTTCATCATCAAAAATATGTTTATTATCAAAAATGCC | 86 | 42 | 0 | 20,0 | GTTTATTATCAAAAAT<br>GTTCATCATCAAAAAT | 24,0 |
| AAGAACGCT<br>GAACAGTTCATCATCAAAAATATGTTTATTATCAAGAACGCT | 59 | 35 | 0 | 0,0 | TCATCA<br>TCATCA | 24,0 |
| AAGAATGCG<br>GAACAGTTCATCATCAAAAATATGTTTATTATCAAGAATGCG | 71 | 35 | 0 | 12,5 | GTTTATTATCAAGAAT<br>GTTCATCATCAAAAAT | 23,0 |
| AAAAACGCT<br>GAACAGTTCATCATCAAAAATATGTTTATTATCAAAAACGCT | 81 | 42 | 0 | 17,4 | GTTTATTATCAAAAA<br>GTTCATCATCAAAAA | 22,0 |
| AAGAATGCC<br>GAACAGTTCATCATCAAAAATATGTTTATTATCAAGAATGCC | 63 | 35 | 0 | 12,5 | GTTTATTATCAAGAAT<br>GTTCATCATCAAAAAT | 15,0 |
| AAAAATGCA<br>GAACAGTTCATCATCAAAAATATGTTTATTATCAAAAATGCA | 80 | 49 | 0 | 20,0 | GTTTATTATCAAAAAT<br>GTTCATCATCAAAAAT | 11,0 |
| AAAAATGCT<br>GAACAGTTCATCATCAAAAATATGTTTATTATCAAAAATGCT | 75 | 49 | 0 | 20,0 | GTTTATTATCAAAAAT<br>GTTCATCATCAAAAAT | 6,0 |
| AAGAATGCA<br>GAACAGTTCATCATCAAAAATATGTTTATTATCAAGAATGCA | 57 | 42 | 0 | 12,5 | GTTTATTATCAAGAAT<br>GTTCATCATCAAAAAT | 2,0 |
| AAGAATGCT<br>GAACAGTTCATCATCAAAAATATGTTTATTATCAAGAATGCT | 53 | 42 | 0 | 12,5 | GTTTATTATCAAGAAT<br>GTTCATCATCAAAAAT | -2,0 |

## Beispiel 2

Bei diesem Beispiel wird die Optimierung von GFP auf Expression in E. Coli betrachtet.

Herkunft der Aminosäuresequenz:
DEFINITION Aequorea victoria green-fluorescent protein mRNA, complete cds. ACCESSION M62654

MSKGEELFTGVVPILVELDGDVNGHKFSVSGEGEGDATYGKLTLKFICTTGKLPVPWPTLVTTFSYGVQCFSRYP
DHMKQHDFFKSAMPEGYVQERTIFYKDDGNYKSRAEVKFEGDTLVNRIELKGIDFKEDGNILGHKMEYNYNSHNV
YIMADKQKNGIKVNFKIRHNIEDGSVQLADHYQQNTPIGDGPVLLPDNHYLSTQSALSKDPNEKRDHMILLEFVT
AAGITHGMDELYK

Verwendete Codon-Usage-Table: Escherichia coli K12

Herkunft: Codon usage Database auf www.kazusa.or.jp/codon

Nachfolgend bedeuten:

<CU> : durchschnittliche renormierte Codon-Usage der KDS (15 Basen lang)
<GC> : durchschnittlicher prozentualer GC-Gehalt der letzten 35 Basen der Testsequenz
$GC_{Wunsch}$: Angestrebter GC-Gehalt

Die Größe des Fenster, auf dem der GC-Gehalt für die graphische Darstellung in Fig. 5b bis 8b berechnet wurde, betrug 40 Basen

Fig. 5a und 5b zeigen die Ergebnisse für die Gütefunktion:

$$Score = \langle CU \rangle$$

Fig. 6a und 6b zeigen die Ergebnisse für die Gütefunktion

$$Score = \langle CU \rangle - \left| \langle GC \rangle - GC_{Wunsch} \right|^{1.3} \times 0.8$$

Fig. 7a und 7b zeigen die Ergebnisse für die Gütefunktion

$$Score = \langle CU \rangle - \left|\langle GC \rangle - GC_{Wunsch}\right|^{1.3} \times 1.5$$

Fig. 8a und 8b zeigen die Ergebnisse für die Gütefunktion

$$Score = \langle CU \rangle - \left|\langle GC \rangle - GC_{Wunsch}\right|^{1.3} \times 5$$

Die Figuren 5 bis 8 verdeutlichen den Einfluß der unterschiedlichen Gewichtung zweier Optimierungskriterien auf das Optimierungsergebnis. Ziel ist, die GC-Gehaltsverteilung über die Sequenz zu glätten und den Wert 50% anzunähern. In dem in Fig. 5a und 5b gezeigten Fall wurde lediglich auf die optimale Codon-Usage optimiert, was in einer sehr heterogenen und vom Ziel-Gehalt teilweise stark abweichenden GC-Verteilung resultiert. In dem Fall der Fig. 6a und 6b verbindet sich in idealer Weise eine Glättung des GC-Gehaltes auf einen Wert um 50% mit einer guten bis sehr guten Codon-Usage. Die Fälle der Fig. 7a und 7b bzw. 8a und 8b verdeutlichen schließlich, daß eine weitere GC-Gehalts-Optimierung zwar möglich ist, aber mit einer stellenweise schlechten Codon-Usage erkauft werden muß.

**Beispiel 3**

Die Effizienz des erfindungsgemäßen Verfahrens wird durch das nachfolgende Ausführungsbeispiel illustriert, bei dem Expressions-Konstrukte mit adaptierten und RNA- und Codon-optimierten Leserahmen hergestellt wurden und bei dem die jeweilige Expression des Proteins quantifiziert wurde.

Ausgewählte Cytokin- und Chemokingene aus unterschiedlichen Organismen (Mensch: IL15, GM-CSF und Maus: GM-CSF, MIP1alpha) wurden zur Herstellung von Expressionsplasmiden in das Plasmid pcDNA3.1(+) (Invitrogen) kloniert. Die Leserahmen der entsprechenden Gene wurden unter Verwendung einer Kodonwahl, wie sie in humanen bzw. murinen Zellen bevorzugt zu finden ist, und des hier beschriebenen Optimierungsverfahrens auf eine maximale Expression in den betreffenden Organismen optimiert. Die entsprechenden Gene wurden künstlich aufgebaut, nachdem die Aminosäuresequenz der Gene zunächst in eine Nukleotidsequenz, wie sie durch das beschriebene Verfahren unter Berücksichtigung verschiedener Parameter errechnet wurde, übersetzt wurde.

Für die Optimierung der Cytokingene wurden folgende Parameter zugrundegelegt:
Zur Bewertung der Testsequenz wurde folgende Gütefunktion verwendet:

$$GesScore = CUScore - GCScore - REPScore - SEKscore - SiteScore$$

Die KDS-Länge betrug 5 Kodons.

Die Einzelscores sind dabei wie folgt definiert:

a)

$$CUScore = \langle CU \rangle$$

wobei <CU> den arithmetischen Mittelwert der Relative Adaptiveness-Werte der KDS-Kodons, multipliziert mit 100, darstellt, d.h. zur Darstellung der Codon usage eines Codons wird zur besseren Vergleichbarkeit der Codongüte verschiedener Aminosäuren das jeweils beste Codon für eine bestimmte Aminosäure gleich 100 gesetzt und die schlechteren Codons entsprechend ihrem tabellierten prozentualen Anteil reskaliert. Ein *CUScore* von 100 bedeutet also, daß ausschließlich die für das Expressionssystem optimalen Codons verwendet werden. Bei den zu optimierenden Cytokin Genen wurde der CUScore auf Grund der in der nachfolgenden Tabelle aufgeführten Codonhäufigkeiten beim Menschen (*Homo sapiens*) errechnet. In den Optimierungen werden ausschließlich Kodons verwendet, deren relative Adaptiveness größer als 0.6 ist.

| AmAcid | Codon | Frequency | AmAcid | Codon | Frequency |
|---|---|---|---|---|---|
| Ala | GCG | 0.10 | Leu | TTG | 0.12 |
| | GCA | 0.23 | | TTA | 0.08 |
| | GCT | 0.26 | | CTG | 0.38 |

(fortgesetzt)

| AmAcid | Codon | Frequency | AmAcid | Codon | Frequency |
|---|---|---|---|---|---|
| | GCC | 0.40 | | CTA | 0.09 |
| Arg | AGG | 0.20 | | CTT | 0.13 |
| | AGA | 0.20 | | CTC | 0.20 |
| | CGG | 0.20 | Lys | AAG | 0.56 |
| | CGA | 0.11 | | AAA | 0.44 |
| | CGT | 0.08 | Met | ATG | 1.00 |
| | CGC | 0.19 | Phe | TTT | 0.45 |
| Asn | AAT | 0.45 | | TTC | 0.55 |
| | AAC | 0.55 | Pro | CCG | 0.11 |
| Asp | GAT | 0.46 | | CCA | 0.27 |
| | GAC | 0.54 | | CCT | 0.28 |
| Cys | TGT | 0.45 | | CCC | 0.34 |
| | TGC | 0.55 | Ser | AGT | 0.15 |
| End | TGA | 0.61 | | AGC | 0.24 |
| | TAG | 0.17 | | TCG | 0.05 |
| | TAA | 0.21 | | TCA | 0.15 |
| Gln | CAG | 0.73 | | TCT | 0.18 |
| | CAA | 0.27 | | TCC | 0.22 |
| Glu | GAG | 0.58 | Thr | ACG | 0.11 |
| | GAA | 0.42 | | ACA | 0.29 |
| Gly | GGG | 0.25 | | ACT | 0.24 |
| | GGA | 0.25 | | ACC | 0.37 |
| | GGT | 0.16 | Trp | TGG | 1.00 |
| | GGC | 0.34 | Tyr | TAT | 0.44 |
| His | CAT | 0.41 | | TAC | 0.56 |
| | CAC | 0.59 | Val | GTG | 0.45 |
| Ile | ATA | 0.18 | | GTA | 0.12 |
| | ATT | 0.35 | | GTT | 0.18 |
| | ATC | 0.47 | | GTC | 0.24 |

*Quelle: GenBank Release 138.0 [October 15 2003] codon usage database, http://www.kazusa.or.jp/codon/*

b)

$$GCScore = \left|\langle GC \rangle - GC_{Wunsch}\right| \times 2$$

mit

<GC> : durchschnittlicher prozentualer GC-Gehalt der letzten 35 Basen der Testsequenz
$GC_{Wunsch}$: Angestrebter prozentualer GC-Gehalt von 60%

c)

$$REPScore = \left(Score_{Alignment},_{\max}\right)$$

Zur Ermittlung der Einzelgewichte der Alignments (Alignmentscore) wird ein lokales Alignment eines endständigen Teilbereiches der Testsequenz, der maximal die letzten 35 Basen der kompletten Testsequenz umfasst, mit dem davorliegenden Bereich der Testsequenz durchgeführt.

Als Bewertungsparameter für eine Basenposition werden dabei verwendet:

Übereinstimmung = 10;
Mismatch = -30;
Lücke (Gap) = -30.

Das entsprechende Kriteriumsgewicht *REPScore* ist definiert als der höchste erreichte Alignment-Score $Score_{Alignment,maxi}$ in dem überprüften Bereich der Testsequenz. Ist der Wert von $Score_{Alignment,max}$ < 100, so wird *REPScore* = 0 gesetzt.

d)

$$SEKScore = \left(Score_{InvAlignment\,\max}\right)$$

Das Kriteriumsgewicht SEKScore gewichtet inverse Alignments in der erzeugten Sequenz.

Zur Ermittlung des Einzelgewichts eines Alignments ($Score_{InvAlignment, max}$) wird ein lokales Alignment des Invers-komplementären der Testsequenz mit dem Teilbereich der Testsequenz durchgeführt, der maximal die letzten 35 Basen der kompletten Testsequenz umfaßt.

Als Bewertungsparameter für eine Basenposition werden dabei verwendet:

Übereinstimmung = 10;
Mismatch = -30;
Lücke (Gap) = -30.

Das entsprechende Kriteriumsgewicht SEKScore ist definiert als der höchste erreichte Alignment-Score $Score_{InvAlignment,max}$ in dem überprüften Bereich der Testsequenz. Ist der Wert von $Score_{InvAlignment,max}$ < 100, so wird *SEKScore* = 0 gesetzt.

e) Sitescore

In der folgenden Tabelle sind diejenigen Sequenzmotive aufgelistet, die bei der Ermittlung des SITEScore berücksichtigt wurden. Insoweit in der Rubrik "REVERSE" ein y aufgeführt ist, wurde sowohl das angegebene Sequenzmotiv als auch das zugehörige invers-komplementäre Sequenzmotiv berücksichtigt. Falls in dieser Rubrik ein n angegeben ist, wurde nur das angegebene Sequenzmotiv, nicht aber das hierzu invers-komplementäre Sequenzmotiv berücksichtigt. Für jedes Auftreten der in der Tabelle aufgeführten Sequenzmotive (bzw. deren Invers-komplementärem, falls REVERSE=y) innerhalb der letzten 35 Basen der Testsequenz wird das Kriteriumsgewicht *SITEScore* um den Wert 100000 erhöht.

| NAME | SEQUENZ | REVERSE |
|---|---|---|
| KpnI | GGTACC | n |
| SacI | GAGCTC | n |
| Eukaria: (consensus) branch point | YTRAY | n |
| Eukaria: (consensus) Spice Acceptor | YYYYYYYYYN(1,10)AG | n |
| Eukaria: (consensus) Splice-Donorl | RGGTANGT | n |
| Eukaria: poly(A)-site (1) | AATAAA | n |
| Eukaria: poly(A)-site (2) | TTTTTATA | n |
| Eukaria: poly(A)-site (3) | TATATA | n |

(fortgesetzt)

| NAME | SEQUENZ | REVERSE |
|---|---|---|
| Eukaria: poly(A)-site (4) | TACATA | n |
| Eukaria: poly(A)-site (5) | TAGTAGTA | n |
| Eukaria: poly(A)-site (6) | ATATATTT | n |
| Eukaria: (consensus) Splice-Donor2 | ACGTANGT | n |
| Eukaria: (Cryptic) Splice-Donor1 | RGGTNNGT | n |
| BsmBI | CGTCTC | y |
| BbsI | GAAGAC | y |
| Eukaria: (Cryptic) Splice-Donor2 | RGGTNNHT | n |
| Eukaria: (Cryptic) Splice-Donor3 | NGGTNNGT | n |
| Eukaria: RNA inhib. Sequenz | WWWATTTAWWW | n |
| GC-Stretch | SSSSSSSSS | n |
| Chi-Sequenz | GCTGGTGG | y |
| Repeats | RE (\w{9,})\1 | n |
| Prokaria: RBS-Entry (2) | AAGGAGN(3,13)ATG | y |
| Prokaria: RBS-Entry (1) | AGGAGGN(3,13)ATG | y |
| Prokaria: RBS-Entry (3) | TAASGAGGTN(3,13)DTG | y |
| Prokaria: RBS-Entry (4) | AGAGAGN(3,13)ATG | y |
| Prokaria: RBS-Entry (5) | AAGGAGGN(3,13)ATG | y |
| Prokaria: RBS-Entry (6) | AACGGAGGN(3,13)ATG | y |
| Prokaria: RBS-Entry (7) | AAGAAGGAAN(3,13)ATG | y |
| HindIII | AAGCTT | n |
| NotI | GCGGCCGC | n |
| BamHI | GGATCC | n |
| EcoRI | GAATTC | n |
| XbaI | TCTAGA | n |
| XhoI | CTCGAG | n |

Zur Subklonierung wurden entsprechende singuläre Restriktionsschnittstellen eingefügt. Die kompletten Nukleotidsequenzen sind im Anhang angegeben. Die so modifizierten Sequenzen wurden als vollsynthetische Gene hergestellt (Geneart, Regensburg). Die resultierenden kodierenden DNA Fragmente wurde unter Verwendung der Restriktionsschnittstellen *Hind*III und *Not*I in den Expressionsvektor pcDNA3.1(+) unter die transkriptionelle Kontrolle des Cytomegalo-Virus (CMV) early promotor/enhancer gestellt. Zur Herstellung analoger, jedoch in ihrer Kodonwahl unveränderten Expressionsplasmiden (wildtyp Referenzkonstrukte), wurden die kodierenden Regionen (c-DNA Konstrukte wurden von RZPD bezogen) nach PCR Amplifikation mit entsprechenden Oligonukleotiden ebenfalls unter Verwendung der *Hind*III und *Not*I Restriktionsschnittstellen in pcDNA3.1(+) kloniert.

Zur Quantifizierung der Cytokin/Chemokin-Expression wurden humane Zellen mit den jeweiligen Expressionskonstrukten transfiziert und die Proteinmenge in den Zellen und im Zellkulturüberstand mittels kommerzieller ELISA Testkits gemessen.

Sämtliche Zellkulturprodukte waren von Life Technologies (Karlsruhe). Säugerzelllinien wurden bei 37°C und 5% $CO_2$ kultiviert. Die humane Lungenkarzinomzelllinie H1299 wurden in Dulbecco's Modifiziertem Eagle Medium (DMEM) mit L-Glutamin, D-Glucose (4,5 mg/ml), Natriumpyruvat, 10% inaktiviertem fötalem Rinderserum, Penicillin (100 U/ml) und Streptomycin (100 µg/ml) kultiviert. Die Zellen wurden nach Erreichen der Konfluenz im Verhältnis 1 : 10 subkultiviert.

2,5 x $10^5$ Zellen wurden in 6-well Zellkulturschalen ausgesät und nach 24 h durch Calciumphosphat Kopräzipitation (Graham und Eb, 1973) mit 15 µg Expressions-Plasmiden oder pcDNA 3.1 Vektor (Mock-Kontrolle) transfiziert. Zellen und Kulturüberstände wurden 48 h nach der Transfektion geerntet. Unlösliche Bestandteile in den Überständen wurden 10 min bei 10000xg und 4°C abzentrifugiert. Die transfizierten Zellen wurden zweimal mit eiskaltem PBS (10 mM $Na_2HPO_4$, 1,8 mM $KH_2PO_4$, 137 mM NaCl, 2,7 mM KCl) gewaschen, mit 0,05% Trypsin/EDTA abgelöst, 10 Min. bei 300xg abzentrifugiert und in 100 µl Lyse-Puffer (50 mM Tris-HCl, pH 8,0, 150 mM NaCl, 0,1% SDS (w/v), 1% Nonidet P40 (v/v), 0,5% Na-deoxycholat (w/v)) 30 min auf Eis lysiert. Unlösliche Bestandteile des Zelllysates wurden 30 min bei 10000xg und 4°C abzentrifugiert. Die Gesamtproteinmenge des Zelllysat-Überstandes wurde mit dem Bio-Rad Protein Assay (Bio-Rad, München) nach Herstellerangaben bestimmt.

Die spezifischen Proteinkonzentrationen in den Zelllysaten und Zellkulturüberständen wurden durch ELISA Tests (BD Pharmingen für IL15 und GM-CSF; R & D Systems für MIP1alpha) quantifiziert. Entsprechende Mengen an Gesamtprotein des Zelllysates (0,2 bis 5 µg) und Verdünnungen des Überstandes (unverdünnt bis 1:200) wurden nach den Angaben des Herstellers ausgewertet und die Gesamtkonzentration an Hand einer Eichkurve berechnet. Fig. 9 zeigt eine repräsentative Eichkurve für die Berechnung der Maus-MIP1alpha-Konzentration. Rekombinantes Maus-MIP1alpha wurde nach den Angaben des Herstellers durch serielle zweifach-Verdünnungen auf ansteigende Konzentrationen eingestellt und parallel mit den Proben aus den Zellkülturexperimenten in den MIP1alpha spezifischen ELISA Test eingesetzt. Die Konzentrationen (X-Achse) wurden gegen die gemessenen O.D. Werte (450 nm, Y-Achse) aufgetragen und unter Verwendung von MS Excel wurde eine Regressionsgerade errechnet (der Regressionskoeffizient $R^2$ ist angegeben).

Ergänzend dazu wurden für geeignete Proben ein Nachweis durch Westernblotanalysen durchgeführt. Für GM-CSF Proben wurden Gesamtproteine aus je 1 ml Zellkulturüberstand durch Na-DOC (Natrium deoxycholat) und TCA (Trichloressigsäure) präzipitiert und in 60 µl 1-fach Probenpuffer (Laemmli, 1970) resuspendiert. Für die Analysen wurden jeweils 20 µl eingesetzt. Für den IL15 Nachweis wurden 25 µg Gesamtprotein, aus Zelllysaten verwendet. Die Proben wurden 5 min auf 95°C erhitzt, über ein 15 %-iges SDS/Polyacrylamidgel aufgetrennt (Laemmli, 1970) auf eine Nitrocellulose-Membran elektrotransferiert (Bio-Rad) und mit entsprechenden monoklonalen Antikörpern (BD Pharmingen) analysiert, mittels einem sekundären, AP- (Alkalische Phosphatase) gekoppelten Antikörper detektiert und durch chromogene Färbung nachgewiesen. Fig. 12A bis C zeigen die Expressionsanalyse des synthetischen Leserahmen und der Wildtyp-Leserahmen. H1299 Zellen wurden mit den angegebenen Konstrukten transfiziert und die Proteinproduktion wurde durch konventionelle Immunoblot Analysen nachgewiesen. Dabei zeigt Fig. 12A die Analyse der Zellkulturüberstände nach Na-Doc/TCA Präzipitation von humanen GM-CSF transfizierten H1299 Zellen, Fig. 12B die Analyse der Zellkulturüberstände nach Na-Doc/TCA Präzipitation von murinen GM-CSF transfizierten H1299 Zellen, Fig. 12C die Analyse der Zelllysate von humanen IL15 transfizierten H1299 Zellen. Molekulargewichte (precision plus protein standard, Bio-Rad) sowie Auftrag der wildtyp, synthetischen und Mock-transfizierten Proben sind angegeben. Mock-Transfektion entspricht einer Transfektion mit ursprünglichem pcDNA3.1 Plasmid.

Eine Übersicht der Expressionsunterschiede mit Mittelwerten aller ELISA-ausgewerteten Experimente ist in der nachfolgenden Tabelle angegeben. Die Angaben entsprechen dem prozentualen Unterschied der Gesamtproteinmenge (Summe aus Proteinmenge in Zelllysat und Überstand) im Verhältnis zum entsprechenden wildtyp Konstrukt (wt entspricht 100%).

**Vergleich der Gesamtproteinmengen nach Transfektion von wildtyp vs. synthetischen Expressionskonstrukten**

| Konstrukt | Organismus | MW* | StdDev** | n= |
|---|---|---|---|---|
| GM-CSF | Mensch | **173%** | 53% | 4 |
| IL15 | Mensch | **181%** | 37% | 3 |
| GM-CSF | Maus | **127%** | 12% | 2 |
| MIPlalpha | Maus | **146%** | 48% | 2 |

* Prozentualer Mittelwert der Proteinmenge aus n Versuchen (in Doppelansätzen) im Verhältnis zur Gesamtproteinmenge des entsprechenden wildtyp Konstruktes
** Standardabweichung

Fig. 10 zeigt in Form eines Balkendiagrammes die relative Proteinmenge in Bezug auf das jeweilige Wildtyp-Konstrukt.(entspricht 100%) und illustriert die prozentuale Steigerung der Gesamtproteinmenge nach Transvektion von synthetischen Expressionskonstrukten ge- , genüber Wildtyp-Expressionskonstrukten. H1299 Zellen wurden mit 15 µg der angegebenen Cytokin/Chemokin-Konstrukte transfiziert. Die jeweilige Proteinproduktion wurde durch konventionelle ELISA Tests im Zellkulturüberstand und im Zelllysat anhand entsprechender Standardkurven (siehe Abb. 9) quantifiziert. Das Verhältnis der Gesamproteintmenge von synthetischem zu wildtyp Protein wurde in jedem Experiment (bestehend aus zwei unabhängigen Ansätzen) errechnet und als % Gesamtprotein von wildtyp angegeben. Die Balken repräsentieren den Mittelwert von 4 Experimenten für das humane GM-CSF, von 3 Experimenten für humanes IL15 und von 2 Experimenten für Maus MIP1alpha und GM-CSF, jeweils in unabhängigen Zweifachansätzen. Die Fehlerbalken entsprechen den Standardabweichung.

In Fig. 11 ist eine repräsentative ELISA Analyse der Zelllysate und Überstände transfizierter H1299 Zellen für das humane GM-CSF dargestellt. H1299 Zellen wurden mit jeweils 15 µg wildtyp und optimierter humaner GM-CSF Konstrukte transfiziert. Die jeweilige Proteinkonzentration wurde durch konventionelle ELISA Tests im Zellkulturüberstand und im Zelllysat anhand entsprechender Standardkurven quantifiziert. Die Balken repräsentieren den Wert der Gesamtproteinmenge im Zelllysat (ZL), im Zellkulturüberstand (ÜS) und die Summe aus diesen Werten (Gesamt) für jeweils 2 unabhängige Ansätze (1 und 2).

Diese Analyse zeigt, dass die Expressionszunahme nach Optimierung (hu GM-CSF opt) konstant in Zelllysat und Überstand nachweisbar ist. Zudem verdeutlicht sie exemplarisch, dass die Sekretion der Cytokine durch die Optimierung nach diesem Verfahren nicht beeinflusst wird. Für alle optimierten Konstrukte konnte eine deutliche und reproduzierbare Erhöhung der Proteinexpression nachgewiesen werden, wobei in jedem einzelnen Experiment die Syntheseleistungen der optimierten Gene gegenüber den wildtyp Genen verbessert waren.

Die Expression wurde zusätzlich in Westernblotanalysen überprüft (Fig. 12A bis C). Das humane und das murine GM-CSF konnten im Zellkulturüberstand (nach Na-DOC/TCA-Fällung) nachgewiesen werden (Fig. 12A und B), während das humane IL15 in den Zelllysaten detektiert werden konnte (Fig. 12C). Die Proteine wurden im Vergleich zu kommerziell erhältlichen rekombinanten Proteinen (BD) analysiert und das Molekulargewicht entsprechend bestätigt. In diesen transienten Transfektionsexperimenten war es nicht möglich das murine MIP1alpha durch Immunoblotfärbung nachzuweisen. Der Vergleich der wildtyp mit den synthetischen Proteinen in diesen repräsentativen Immunoblots bestätigt die Daten der ELISA-Auswertungen einer verbesserten Proteinsynthese durch Multiparameter Optimierung dieser Gene.

Die in den Ansprüchen, den Zeichnungen und der Beschreibung offenbarten Merkmale können sowohl einzeln als auch in beliebiger Kombination für die Verwirklichung der Erfindung in ihren verschiedenen Ausführungsformen wesentlich sein.

Anhang: SEQ-IDs und Alignments der verwendeten DNA Sequenzen SEQ-ID der angegebenen Konstrukte:

SEQ-ID1 (Mensch GM-CSF witdtyp):

```
  1 atgtggctgc agagcctgct gctcttgggc actgtggcct gcagcatctc tgcacccgcc
 61 cgctcgccca gccccagcac gcagccctgg gagcatgtga atgccatcca ggaggcccgg
121 cgtctcctga acctgagtag agacactgct gctgagatga atgaaacagt agaagtcatc
181 tcagaaatgt ttgacctcca ggagccgacc tgcctacaga cccgcctgga gctgtacaag
241 cagggcctgc ggggcagcct caccaagctc aagggcccct tgaccatgat ggccagccac
301 tacaagcagc actgccctcc aaccccggaa acttcctgtg caacccagat tatcaccttt
361 gaaagtttca aagagaacct gaaggacttt ctgcttgtca tcccctttga ctgctgggag
421 ccagtccagg agtag
```

SEQ-ID2 (Mensch GM-CSF optimiert):

```
  1 atgtggctgc agagcctgct gctgctggga acagtggcct gtagcatctc tgcccctgcc
 61 agaagcccta gccctagcac acagccttgg gagcacgtga atgccatcca ggaggccagg
121 agactgctga acctgagcag agatacagcc gccgagatga acgagaccgt ggaggtgatc
181 agcgagatgt tcgacctgca ggagcctaca tgcctgcaga cccggctgga gctgtataag
241 cagggcctga gaggctctct gaccaagctg aagggccccc tgacaatgat ggccagccac
301 tacaagcagc actgccctcc tacccctgag acaagctgcg ccacccagat catcaccttc
361 gagagcttca aggagaacct gaaggacttc ctgctggtga tccccttcga ttgctgggag
421 cccgtgcagg agtag
```

SEQ-ID3 (Mensch IL15 wildtyp):

```
  1 atgagaattt cgaaaccaca tttgagaagt atttccatcc agtgctactt gtgtttactt
 61 ctaaacagtc attttctaac tgaagctggc attcatgtct tcattttggg ctgtttcagt
121 gcagggcttc ctaaaacaga agccaactgg gtgaatgtaa taagtgattt gaaaaaaatt
181 gaagatctta ttcaatctat gcatattgat gctactttat atacggaaag tgatgttcac
241 cccagttgca aagtaacagc aatgaagtgc tttctcttgg agttacaagt tatttcactt
301 gagtccggag atgcaagtat tcatgataca gtagaaaatc tgatcatcct agcaaacaac
361 agtttgtctt ctaatgggaa tgtaacagaa tctggatgca aagaatgtga ggaactggag
421 gaaaaaaata ttaaagaatt tttgcagagt tttgtacata ttgtccaaat gttcatcaac
481 acttcttag
```

SEQ-ID4 (Mensch IL15 optimiert):

```
  1 atgcggatca gcaagcccca cctgaggagc atcagcatcc agtgctacct gtgcctgctg
 61 ctgaacagcc acttcctgac agaggccggc atccacgtgt ttatcctggg ctgcttctct
121 gccggcctgc ctaagacaga ggccaactgg gtgaacgtga tcagcgacct gaagaagatc
181 gaggacctga tccagagcat gcacatcgac gccaccctgt acacagagag cgacgtgcac
241 cctagctgta aggtgaccgc catgaagtgc ttcctgctgg agctgcaggt gatcagcctg
301 gagagcggcg atgccagcat ccacgacacc gtggagaacc tgatcatcct ggccaacaac
361 agcctgagca gcaacggcaa tgtgaccgag agcggctgca aggagtgtga ggagctggag
421 gagaagaaca tcaaggagtt cctgcagagc ttcgtgcaca tcgtgcagat gttcatcaac
481 accagctag
```

SEQ-ID5 (Maus GM-CSF wildtyp):

```
  1 atgtggctgc agaatttact tttcctgggc attgtggtct acagcctctc agcacccacc
 61 cgctcaccca tcactgtcac ccggccttgg aagcatgtag aggccatcaa agaagccctg
121 aacctcctgg atgacatgcc tgtcacattg aatgaagagg tagaagtcgt ctctaacgag
181 ttctccttca agaagctaac atgtgtgcag acccgcctga agatattcga gcagggtcta
241 cggggcaatt tcaccaaact caagggcgcc ttgaacatga cagccagcta ctaccagaca
301 tactgccccc caactccgga aacggactgt gaaacacaag ttaccaccta tgcggatttc
361 atagacagcc ttaaaacctt tctgactgat atcccctttg aatgcaaaaa accaggccaa
421 aaatag
```

SEQ-ID6 (Maus GM-CSF optimiert):

```
  1 atgtggctgc agaacctgct gttcctgggc atcgtggtgt acagcctgag cgcccccacc
 61 aggagcccca tcaccgtgac caggccctgg aagcacgtgg aggccatcaa ggaggccctg
121 aacctgctgg acgacatgcc cgtgaccctg aacgaggagg tggaggtggt gagcaacgag
181 ttcagcttca agaagctgac ctgcgtgcag accaggctga agatcttcga gcagggcctg

241 aggggcaact tcaccaagct gaagggcgcc ctgaacatga ccgccagcta ctaccagacc
301 tactgccccc ccacccccga gaccgactgc gagacccagg tgaccaccta cgccgacttc
361 atcgacagcc tgaagacctt cctgaccgac atccccttcg agtgcaagaa gcccggccag
421 aagtag
```

SEQ-ID7 (Maus MIPlalpha wildtyp):

```
  1 atgaaggtct ccaccactgc ccttgctgtt cttctctgta ccatgacact ctgcaaccaa
 61 gtcttctcag cgccatatgg agctgacacc ccgactgcct gctgcttctc ctacagccgg
121 aagattccac gccaattcat cgttgactat tttgaaacca gcagcctttg ctcccagcca
181 ggtgtcattt tcctgactaa gagaaaccgg cagatctgcg ctgactccaa agagacctgg
241 gtccaagaat acatcactga cctggaactg aatgcctag
```

SEQ-ID8 (Maus MIPlalpha optimiert):

```
  1 atgaaggtga gcaccacagc tctggctgtg ctgctgtgca ccatgaccct gtgcaaccag
 61 gtgttcagcg ctccttacgg cgccgatacc cctacagcct gctgcttcag ctacagcagg
121 aagatcccca ggcagttcat cgtggactac ttcgagacca gcagcctgtg ttctcagccc
181 ggcgtgatct tcctgaccaa gcggaacaga cagatctgcg ccgacagcaa ggagacatgg
241 gtgcaggagt acatcaccga cctggagctg aacgcctag
```

**Alignments der verwendeten DNA Sequenzen**

**1. Humanes GM-CSF:**

Obere Zeile: SEQ-ID1 (Mensch GM-CSF witdtyp), from 1 to 435
Untere Zeile: SEQ-ID2 (Mensch GM-CSF optimiert), from 1 to 435
Wildtyp:optimiert identity= 83.45%(363/435) gap=0.00%(0/435)

```
1      ATGTGGCTGCAGAGCCTGCTGCTCTTGGGCACTGTGGCCTGCAGCATCTCTGCACCCGCC
       |||||||||||||||||||||||  |||| || |||||||| ||||||||||| || |||
1      ATGTGGCTGCAGAGCCTGCTGCTGCTGGGAACAGTGGCCTGTAGCATCTCTGCCCCTGCC

61     CGCTCGCCCAGCCCCAGCACGCAGCCCTGGGAGCATGTGAATGCCATCCAGGAGGCCCGG
        |    || ||||| ||||| ||||| |||||||| ||||||||||||||||||||| ||
61     AGAAGCCCTAGCCCTAGCACACAGCCTTGGGAGCACGTGAATGCCATCCAGGAGGCCAGG

121    CGTCTCCTGAACCTGAGTAGAGACACTGCTGCTGAGATGAATGAAACAGTAGAAGTCATC
        | || ||||||||||| ||||| || || || |||||||| || || || || || |||
121    AGACTGCTGAACCTGAGCAGAGATACAGCCGCCGAGATGAACGAGACCGTGGAGGTGATC

181    TCAGAAATGTTTGACCTCCAGGAGCCGACCTGCCTACAGACCCGCCTGGAGCTGTACAAG
          || ||||| ||||| |||||||| || ||||| |||||||| ||||||||||| |||
181    AGCGAGATGTTCGACCTGCAGGAGCCTACATGCCTGCAGACCCGGCTGGAGCTGTATAAG

241    CAGGGCCTGCGGGGCAGCCTCACCAAGCTCAAGGGCCCCTTGACCATGATGGCCAGCCAC
       ||||||||| | |||   || |||||||| ||||||||| |||| |||||||||||||||
241    CAGGGCCTGAGAGGCTCTCTGACCAAGCTGAAGGGCCCCCTGACAATGATGGCCAGCCAC

301    TACAAGCAGCACTGCCCTCCAACCCCGGAAACTTCCTGTGCAACCCAGATTATCACCTTT
       |||||||||||||||||||| ||||| || ||   ||| || |||||||| |||||||| 
301    TACAAGCAGCACTGCCCTCCTACCCCTGAGACAAGCTGCGCCACCCAGATCATCACCTTC

361    GAAAGTTTCAAAGAGAACCTGAAGGACTTTCTGCTTGTCATCCCCTTTGACTGCTGGGAG
       || || ||||| ||||||||||||||||| ||||| || |||||||| || |||||||||
361    GAGAGCTTCAAGGAGAACCTGAAGGACTTCCTGCTGGTGATCCCCTTCGATTGCTGGGAG

421    CCAGTCCAGGAGTAG
       || || |||||||||
421    CCCGTGCAGGAGTAG
```

**2. Humanes IL15:**

Obere Zeile: SEQ-ID3 (Mensch IL15 wildtyp), from 1 to 489

Untere Zeile: SEQ-ID4 (Mensch IL15 optimiert), from 1 to 489
Wildtyp:optimiert identity= 70.55%(345/489) gap=0.00%(0/489)

```
1     ATGAGAATTTCGAAACCACATTTGAGAAGTATTTCCATCCAGTGCTACTTGTGTTTACTT
      ||| | ||    || || ||  |||| || ||   ||||||||||||| ||||  | || 
1     ATGCGGATCAGCAAGCCCCACCTGAGGAGCATCAGCATCCAGTGCTACCTGTGCCTGCTG

61    CTAAACAGTCATTTTCTAACTGAAGCTGGCATTCATGTCTTCATTTTGGGCTGTTTCAGT
      || ||||| || || || || | ||| ||||| || || || ||  ||||||| |||  |
61    CTGAACAGCCACTTCCTGACAGAGGCCGGCATCCACGTGTTTATCCTGGGCTGCTTCTCT

121   GCAGGGCTTCCTAAAACAGAAGCCAACTGGGTGAATGTAATAAGTGATTTGAAAAAAATT
      || || || ||||| ||||| |||||||||||||| || || || ||  |||| || |  
121   GCCGGCCTGCCTAAGACAGAGGCCAACTGGGTGAACGTGATCAGCGACCTGAAGAAGATC

181   GAAGATCTTATTCAATCTATGCATATTGATGCTACTTTATATACGGAAAGTGATGTTCAC
      || || || || ||    |||||| || || || ||  | || || || || || || |||
181   GAGGACCTGATCCAGAGCATGCACATCGACGCCACCCTGTACACAGAGAGCGACGTGCAC

241   CCCAGTTGCAAAGTAACAGCAATGAAGTGCTTTCTCTTGGAGTTACAAGTTATTTCACTT
      || || || || || || || |||||||||| || ||  ||||| | || ||  ||    || 
241   CCTAGCTGTAAGGTGACCGCCATGAAGTGCTTCCTGCTGGAGCTGCAGGTGATCAGCCTG

301   GAGTCCGGAGATGCAAGTATTCATGATACAGTAGAAAATCTGATCATCCTAGCAAACAAC
      |||  ||| ||||| || || || || || || || || |||||||||| | |||||||
301   GAGAGCGGCGATGCCAGCATCCACGACACCGTGGAGAACCTGATCATCCTGGCCAACAAC

361   AGTTTGTCTTCTAATGGGAATGTAACAGAATCTGGATGCAAAGAATGTGAGGAACTGGAG
      ||  ||      || || ||||| || ||    || ||||| || |||||||| ||||||
361   AGCCTGAGCAGCAACGGCAATGTGACCGAGAGCGGCTGCAAGGAGTGTGAGGAGCTGGAG

421   GAAAAAAATATTAAAGAATTTTTGCAGAGTTTTGTACATATTGTCCAAATGTTCATCAAC
      || || || || || || ||  ||||||| || || || || || || ||||||||||||
421   GAGAAGAACATCAAGGAGTTCCTGCAGAGCTTCGTGCACATCGTGCAGATGTTCATCAAC

481   ACTTCTTAG
      ||    |||
481   ACCAGCTAG
```

**3. Maus GM-CSF:**

Obere Zeile: SEQ-ID5 (Maus GM-CSF wildtyp), from 1 to 426
Untere Zeile: SEQ-ID6 (Maus GM-CSF optimiert), from 1 to 426
Wildtyp:optimiert identity= 80.75%(344/426) gap=0.00%(0/426)

```
1      ATGTGGCTGCAGAATTTACTTTTCCTGGGCATTGTGGTCTACAGCCTCTCAGCACCCACC
       ||||||||||||||  | || ||||||||||| ||||| ||||||||    || ||||||
1      ATGTGGCTGCAGAACCTGCTGTTCCTGGGCATCGTGGTGTACAGCCTGAGCGCCCCCACC

61     CGCTCACCCATCACTGTCACCCGGCCTTGGAAGCATGTAGAGGCCATCAAAGAAGCCCTG
        |    |||||||| || ||| |||| |||||||| || ||||||||||| || ||||||
61     AGGAGCCCCATCACCGTGACCAGGCCCTGGAAGCACGTGGAGGCCATCAAGGAGGCCCTG

121    AACCTCCTGGATGACATGCCTGTCACATTGAATGAAGAGGTAGAAGTCGTCTCTAACGAG
       ||||| ||||| |||||||| || ||  |||| || ||||| || || ||    ||||||
121    AACCTGCTGGACGACATGCCCGTGACCCTGAACGAGGAGGTGGAGGTGGTGAGCAACGAG

181    TTCTCCTTCAAGAAGCTAACATGTGTGCAGACCCGCCTGAAGATATTCGAGCAGGGTCTA
       |||  |||||||||||| || || ||||||||| | |||||||| ||||||||||| || 
181    TTCAGCTTCAAGAAGCTGACCTGCGTGCAGACCAGGCTGAAGATCTTCGAGCAGGGCCTG

241    CGGGGCAATTTCACCAAACTCAAGGGCGCCTTGAACATGACAGCCAGCTACTACCAGACA
        ||||||| |||||||| || ||||||||| |||||||||| ||||||||||||||||| 
241    AGGGGCAACTTCACCAAGCTGAAGGGCGCCCTGAACATGACCGCCAGCTACTACCAGACC

301    TACTGCCCCCCAACTCCGGAAACGGACTGTGAAACACAAGTTACCACCTATGCGGATTTC
       ||||||||||| || || || || ||||| || || || || |||||||| || || |||
301    TACTGCCCCCCCACCCCCGAGACCGACTGCGAGACCCAGGTGACCACCTACGCCGACTTC

361    ATAGACAGCCTTAAAACCTTTCTGACTGATATCCCCTTTGAATGCAAAAAACCAGGCCAA
       || |||||||| || ||||| ||||| || |||||||| || ||||| || || ||||| 
361    ATCGACAGCCTGAAGACCTTCCTGACCGACATCCCCTTCGAGTGCAAGAAGCCCGGCCAG

421    AAATAG
       || |||
421    AAGTAG
```

**4. Maus MIP1alpha**

Obere Zeile: SEQ-ID7 (Maus MIPlalpha wildtyp), from 1 to 279
Untere Zeile: SEQ-ID8 (Maus MIPlalpha optimiert), from 1 to 279
Wildtyp:optimiert identity= 78.49%(219/279) gap=0.00%(0/279)

```
1      ATGAAGGTCTCCACCACTGCCCTTGCTGTTCTTCTCTGTACCATGACACTCTGCAACCAA
       ||||||||   |||||| || || ||||| || || || |||||||| || |||||||| 
1      ATGAAGGTGAGCACCACAGCTCTGGCTGTGCTGCTGTGCACCATGACCCTGTGCAACCAG

61     GTCTTCTCAGCGCCATATGGAGCTGACACCCCGACTGCCTGCTGCTTCTCCTACAGCCGG
       || |||   || || || || || ||||| || ||||||||||||  ||||||| ||
61     GTGTTCAGCGCTCCTTACGGCGCCGATACCCCTACAGCCTGCTGCTTCAGCTACAGCAGG

121    AAGATTCCACGCCAATTCATCGTTGACTATTTTGAAACCAGCAGCCTTTGCTCCCAGCCA
       ||||| ||  | || |||||||| ||||| || || ||||||||||| || || ||||| 
121    AAGATCCCCAGGCAGTTCATCGTGGACTACTTCGAGACCAGCAGCCTGTGTTCTCAGCCC

181    GGTGTCATTTTCCTGACTAAGAGAAACCGGCAGATCTGCGCTGACTCCAAAGAGACCTGG
       || || || |||||||| ||| | ||| | ||||||||||| |||  ||| ||||| |||
181    GGCGTGATCTTCCTGACCAAGCGGAACAGACAGATCTGCGCCGACAGCAAGGAGACATGG

241    GTCCAAGAATACATCACTGACCTGGAACTGAATGCCTAG
       || || || |||||||| |||||||| ||||| ||||||
241    GTGCAGGAGTACATCACCGACCTGGAGCTGAACGCCTAG
```

## Patentansprüche

1. Verfahren zum Optimieren einer Nucleotidsequenz zur Maximierung der Expression eines Proteins in einem vorgegebenen Organismus auf der Grundlage der Aminosäurensequenz des Proteins, welches die folgenden auf einem Computer durchgeführten Schritte umfasst:

   - Generieren einer ersten Testsequenz von n Codons, welche n aufeinanderfolgenden Aminosäuren in der Proteinsequenz entsprechen, wobei n eine natürliche Zahl und kleiner oder gleich N, der Zahl der Aminosäuren der Proteinsequenz, ist,
   - Festlegen von m Optimierungspositionen in der Testsequenz, welche der Position von m Codons entsprechen, an denen die Besetzung mit einem Codon, bezogen auf die Testsequenz, optimiert werden soll, wobei m<= n und m < N ist,
   - Generieren einer oder mehrerer weiterer Testsequenzen aus der ersten Testsequenz, indem an einer oder mehreren der m Optimierungspositionen ein Codon der ersten Testsequenz durch ein anderes Codon ersetzt wird, welches dieselbe Aminosäure exprimiert,
   - Bewerten jeder der Testsequenzen mit einer Gütefunktion und Ermitteln der hinsichtlich der Gütefunktion optimalen Testsequenz,
   - Festlegen von p Codons der optimalen Testsequenz, welche sich an einer der m Optimierungspositionen befinden, als Ergebniscodons, welche die Codons der optimierten Nucleotidsequenz an den Positionen bilden, die der Position der besagten p Codons in der Testsequenz entspricht, wobei p eine natürliche Zahl ist, p<= m ist und in einem oder mehreren Iterationsschritten p gleich Eins ist,
   - Iterieren der vorangehenden Schritte, wobei in jedem Iterationsschritt die Testsequenz an den Positionen, welche Positionen von festgelegten Ergebniscodons in der optimierten Nucleotidsequenz entsprechen, das entsprechende Ergebniscodon enthält und die Optimierungspositionen von Positionen von Ergebniscodons verschieden sind.

2. Verfahren nach Anspruch 1, **dadurch gekennzeichnet, dass** in einem oder mehreren Iterationsschritten die m Optimierungspositionen der Testsequenzen unmittelbar auf ein oder mehrere Ergebniscodons folgen, welche als Teil der optimierten Nucleotidsequenz festgelegt worden sind.

3. Verfahren nach Anspruch 1 oder 2, **dadurch gekennzeichnet, dass** in einem oder mehreren Iterationsschritten die p Codons, die als Ergebniscodons der optimierten Nucleotidsequenz festgelegt werden, p aufeinanderfolgende Codons sind.

4. Verfahren nach einem der Ansprüche 1 bis 3, **dadurch gekennzeichnet, dass** in einem Iterationsschritt Testsequenzen mit allen möglichen Codonbesetzungen für die m Optimierungspositionen aus der ersten Testsequenz generiert werden und die optimale Testsequenz unter diesen Testsequenzen ermittelt wird.

5. Verfahren nach Anspruch 1, **dadurch gekennzeichnet,**

**dass** in einem oder mehreren Iterationsschritten die m Optimierungspositionen der Testsequenzen unmittelbar auf ein oder mehrere Ergebniscodons folgen, welche als Teil der optimierten Nucleotidsequenz festgelegt worden sind, wobei m für alle Iterationsschritte konstant ist,
**dass** das Codon, das als Ergebniscodon der optimierten Nucleotidsequenz festgelegt wird, unmittelbar auf ein oder mehrere Ergebniscodons folgt, welche als Teil der optimierten Nucleotidsequenz in einem früheren Iterationsschritt festgelegt worden sind, dass in einem Iterationsschritt Testsequenzen mit allen möglichen Codonbesetzungen für die m Optimierungspositionen aus der ersten Testsequenz generiert werden und die optimale Testsequenz unter diesen Testsequenzen ermittelt wird,
und **dass** in zwei oder mehr aufeinanderfolgenden Iterationsschritten ein Teil der m Optimierung-positionen, auf welchen die Codons variiert werden, identisch sind.

6. Verfahren nach Anspruch 5, **dadurch gekennzeichnet, dass** in einem oder mehreren Iterationsschritten die m Optimierungspositionen der Testsequenzen unmittelbar auf ein oder mehrere Ergebniscodons folgen, welche als Teil der optimierten Nucleotidsequenz festgelegt worden sind,
dass m in einem Bereich von 3 bis 20 liegt,
dass die Testsequenz nach benutzerdefinierten Kriterien evaluiert wird und/oder Kriteriumsgewichte für individuelle Testkriterien, die in einer Bewertungsfunktion addiert werden, nach benutzerdefinierten Vorgaben gewichtet werden, dass die Testsequenz nach jedem Schritt um p Codons verlängert wird, dass die genannten Schritte so oft iteriert werden, bis alle Codons der Nucleotidsequenz mit Ergebniscodons besetzt worden sind, wobei in allen Iterationsschritten ausschliesslich des letzten Iterationsschrittes die Zahl p der Ergebniscodons Eins ist und beim letzten Iterationsschritt, wenn n = N und die Zahl derjenigen Positionen, die in der Testsequenz nicht mit Ergebniscodons besetzt sind, gleich dem Wert m ist, die Zahl p der Ergebniscodons m ist.

7. Verfahren nach einem der Ansprüche 1 bis 6, **gekennzeichnet durch**:

- Bewerten jeder Testsequenz mit einer Gütefunktion,
- Ermitteln eines Extremwertes innerhalb der Werte der Gütefunktion für alle in einem Iterationsschritt generierten Teilsequenzen,
- Festlegen von p Codons der Testsequenz, welche dem extremalen Wert der Gewichtsfunktion entspricht, als Ergebniscodons an den entsprechenden Positionen, wobei p eine natürliche Zahl und p<= m ist,

wobei die Gütefunktion eines oder mehrere der folgenden Kriterien berücksichtigt: Codon usage für einen vorgegebenen Organismus, GC-Gehalt, repetitive Sequenzen, Sekundärstrukturen, invers komplementäre Sequenzwiederholungen und Sequenzmotive.

8. Verfahren nach Anspruch 7, **dadurch gekennzeichnet, dass** die Gütefunktion ein Kriteriumsgewicht für Sequenzmotive umfasst, welches betragsmässig wesentlich grösser ist als andere Gewichte, so dass es nicht durch diese anderen Kriteriumsgewichte kompensiert werden kann und das oder die entsprechenden Motive in der Sequenz aus dieser ausgeschlossen werden.

9. Verfahren nach Anspruch 8, **dadurch gekennzeichnet, dass** eines oder mehrere der folgenden Motive berücksichtigt wird:

RE-Schnittsequenzen, Enhancersequenzen, immunstimulatorische CpG-Motive und immunsupprimierende CpG-Motive,
cis-aktive Sequenz-Motive, insbesondere DNS/Protein-Interaktionsbindestellen und RNS/Protein-Interaktionsbindestellen, bevorzugt Spleissmotive,
Transkriptionsfaktorbindestellen, Transkriptionsterminatorenbindestellen, Polyadenylierungssignale, Endonucleaseerkennungssequenzen, immunomodulatorische DNS-Motive, Ribosomenbindestellen, Erkennungssequenzen für rekombinationsaktive Enzyme, Erkennungssequenzen für DNS-modifizierende Enzyme, Erkennungssequenzen für RNSmodifizierende Enzyme, Sequenzmotive, die in einem vorgegebenen Organismus unterrepräsentiert sind, repetitive Sequenzen, Sekundärstrukturen, inverse repeats, Nla III Erkennungssequenz CATG, BsmBI, BbsI, Chi-Sequenz, KpnI, SacI.

10. Verfahren nach einem der Ansprüche 1 bis 9, **dadurch gekennzeichnet, dass** die Gütefunktion eines oder mehrere der folgenden Kriterien berücksichtigt:

- Ausschluss von invers komplementären Sequenzidentitäten von mehr als 20 Nucleotiden zum Transkriptom eines vorgegebenen Organismus,
- Ausschluss von Homologiebereichen von mehr als 100 Basenpaaren zu einer vorgegebenen DNS-Sequenz,
- Ausschluss von Homologiebereichen mit mehr als 90 % Ähnlichkeit der Nucleotidsequenz zu einer vorgegebenen DNS-Sequenz.

**11.** Verfahren nach einem der Ansprüche 1 bis 10, **gekennzeichnet durch** den Schritt des Synthetisierens der optimierten Nucleotidsequenz.

**12.** Vorrichtung zum Optimieren einer Nucleotidsequenz zur Expression eines Proteins auf der Grundlage der Aminosäurensequenz des Proteins, welche eine Recheneinrichtung aufweist, wobei die Vorrichtung eine Einrichtung zum Durchführen der Schritte eines Verfahrens nach einem der Ansprüche 1 bis 11 umfasst.

**13.** Computerprogramm, welches von einem Computer ausführbaren Programmcode enthält, der, wenn er auf einem Computer ausgeführt wird, den Computer veranlasst, ein Verfahren nach einem der Ansprüche 1 bis 11 durchzuführen.

**14.** Computerlesbarer Datenträger, auf welchem in computerlesbarer Form ein Programm nach Anspruch 13 gespeichert ist.

## Claims

**1.** Method for optimizing a nucleotide sequence to maximize the expression of a protein in a given organism based on the amino acid sequence of the protein, comprising the following steps performed on a computer:

- generating a first test sequence of n codons which correspond to n consecutive amino acids in the protein sequence, where n is a natural number and is less than or equal to N, the number of amino acids of the protein sequence;
- specifying m optimization positions in the test sequence which correspond to the position of m codons, in which positions the occupation by a codon, with respect to the test sequence, is to be optimized, where m <= n and m < N;
- generating one or more further test sequences from the first test sequence by replacing, in one or more of the m optimization positions, a codon from the first test sequence with another codon which expresses the same amino acid;
- rating each of the test sequences with a quality function and determining the optimal test sequence in terms of the quality function;
- specifying p codons from the optimal test sequence which are located in one of the m optimization positions as result codons which form the codons of the optimized nucleotide sequence in the positions corresponding to the position of said p codons in the test sequence, where p is a natural number, p <= m, and p is equal to one in one or more iteration steps;
- iterating the preceding steps, wherein, in each iteration step, the test sequence contains the corresponding result codon in the positions which correspond to positions of specified result codons in the optimized nucleotide sequence, and the optimization positions are different to positions of result codons.

**2.** Method according to claim 1, **characterized in that**, in one or more iteration steps, the m optimization positions of the test sequences directly follow one or more result codons which have been specified as part of the optimized nucleotide sequence.

**3.** Method according to either claim 1 or claim 2, **characterized in that**, in one or more iteration steps, the p codons which are specified as result codons of the optimized nucleotide sequence are p consecutive codons.

**4.** Method according to any of claims 1 to 3, **characterized in that**, in an iteration step, test sequences are generated with all possible codon occupations for the m optimization positions from the first test sequence and the optimal test sequence is determined from among these test sequences.

**5.** Method according to claim 1, **characterized in that**,
in one or more iteration steps, the m optimization positions of the test sequences directly follow one or more result codons which have been specified as part of the optimized nucleotide sequence, where m is constant for all iteration steps, **in that** the codon which is specified as the result codon of the optimized nucleotide sequence is immediately followed by one or more result codons which have been specified as part of the optimized nucleotide sequence in an earlier iteration step, **in that**, in an iteration step, test sequences with all possible codon occupations for the m optimization positions are generated from the first test sequence and the optimal test sequence is determined from among these test sequences,
and **in that**, in two or more consecutive iteration steps, some of the m optimization positions in which the codons are varied are identical.

**6.** Method according to claim 5, **characterized in that**, in one or more iteration steps, the m optimization positions of the test sequences directly follow one or more result codons which have been specified as part of the optimized nucleotide sequence,
**in that** m is in a range of from 3 to 20,
**in that** the test sequence is evaluated according to user-defined criteria and/or criterion weights for individual test criteria, which are added in a rating function, are weighted according to user-defined specifications,
**in that** the test sequence is extended by p codons after each step, **in that** said steps are iterated until all codons of the nucleotide sequence have been occupied by result codons, the number p of the result codons being one in all iteration steps excluding the last iteration step, and in the last iteration step, if n = N and the number of those positions not occupied by result codons in the test sequence is equal to the value m, the number p of the result codons being m.

**7.** Method according to any of claims 1 to 6, **characterized by**:

- rating each test sequence with a quality function;
- determining an extreme value within the values of the quality function for all subsequences generated in an iteration step;
- specifying p codons of the test sequence which corresponds to the extremal value of the weight function as result codons in the corresponding positions, where p is a natural number and p <= m,

the quality function taking into consideration one or more of the following criteria: codon usage for a given organism; GC content; repetitive sequences; secondary structures; reverse complementary sequence repeats; and sequence motifs.

**8.** Method according to claim 7, **characterized in that** the quality function comprises a criterion weight for sequence motifs which is substantially larger in terms of magnitude than other weights so that it cannot be compensated for by these other criterion weights and the one or more corresponding motifs in the sequence are excluded therefrom.

**9.** Method according to claim 8, **characterized in that** one or more of the following motifs is taken into consideration:

RE cleaving sequences; enhancer sequences; immunostimulatory CpG motifs and immunosuppressive CpG motifs;
cis-active sequence motifs, in particular DNA/protein interaction binding sites and RNA/protein interaction binding sites, preferably splicing motifs; transcription factor binding sites;
transcription terminator binding sites; polyadenylation signals; endonuclease recognition sequences; immunomodulatory DNA motifs; ribosome binding sites; recombination-active enzyme recognition sequences; DNA-modifying enzyme recognition sequences; RNA-modifying enzyme recognition sequences; sequence motifs that are underrepresented in a given organism; repetitive sequences; secondary structures; inverted repeats; NIa III recognition sequence CATG; BsmBI; BbsI; Chi sequence; KpnI; SacI.

**10.** Method according to any of claims 1 to 9, **characterized in that** the quality function takes into consideration one or more of the following criteria:

- exclusion of reverse complementary sequence identities of more than 20 nucleotides to the transcriptome of a given organism;
- exclusion of homology regions of more than 100 base pairs to a given DNA sequence;
- exclusion of homology regions having more than 90% similarity of the nucleotide sequence to a given DNA sequence.

**11.** Method according to any of claims 1 to 10, **characterized by** the step of synthesizing the optimized nucleotide sequence.

**12.** Device for optimizing a nucleotide sequence for expressing a protein based on the amino acid sequence of the protein, which device has a computing unit, wherein the device comprises a unit for performing the steps of a method according to any of claims 1 to 11.

**13.** Computer program containing program code executable by a computer which, when executed on a computer, causes the computer to perform a method according to any of claims 1 to 11.

**14.** Computer-readable data carrier on which a program according to claim 13 is stored in a computer-readable form.

## Revendications

**1.** Procédé d'optimisation d'une séquence de nucléotides pour la maximisation de l'expression d'une protéine dans un organisme donné sur la base de la séquence d'acides aminés de la protéine, lequel procédé comprend les étapes, effectuées sur un ordinateur, consistant à :

- générer une première séquence de test de n codons correspondant à n acides aminés consécutifs dans la séquence protéique, n étant un nombre naturel et inférieur ou égal à N, nombre d'acides aminés de la séquence protéique,
- définir m positions d'optimisation de la séquence de test qui correspondent à la position de m codons, auxquels l'occupation avec un codon doit être optimisé sur la base de la séquence de test, avec $m \leq n$ et $m < N$,
- générer une ou de plusieurs autres séquences de test à partir de la première séquence de test par remplacement, à une ou plusieurs des m positions d'optimisation, d'un codon de la première séquence de test par un autre codon qui exprime le même acide aminé,
- évaluer chacune des séquences de test avec une fonction de qualité et la déterminer la séquence de test optimale en termes de fonction de qualité,
- définir p codons de la séquence de test optimale qui sont situés à l'une des m positions d'optimisation, comme codons résultants qui forment les codons de la séquence de nucléotides optimisée aux positions, qui correspond à la position desdits p codons de la séquence de test, p étant un nombre naturel, et $p \leq m$, et p est égal à un dans une ou plusieurs étapes d'itération,
- itérer les étapes précédentes, à chaque étape d'itération, la séquence de test aux positions qui correspondent aux positions de codons résultants définis de la séquence de nucléotides optimisée, contenant le codon résultant correspondant et les positions d'optimisation étant différentes des positions de codons résultants.

**2.** Procédé selon la revendication 1, **caractérisé en ce que**, à une ou plusieurs étapes d'itération, les m positions d'optimisation des séquences de test suivent directement un ou plusieurs codons résultants qui ont été définis comme faisant partie de la séquence de nucléotides optimisée.

**3.** Procédé selon la revendication 1 ou 2, **caractérisé en ce que**, à une ou plusieurs étapes d'itération, les p codons qui sont définis comme des codons résultants de la séquence de nucléotides optimisés sont des p codons qui se suivent.

**4.** Procédé selon l'une des revendications 1 à 3, **caractérisé en ce que**, à une étape d'itération, des séquences de test sont générées avec toutes les occupations de codons possible pour les m positions d'optimisation à partir de la première séquence de test et la séquence de test optimale est déterminée sur la base de ces séquences de test.

**5.** Procédé selon la revendication 1, **caractérisé en ce que**,
à une ou plusieurs étapes d'itération, les m positions d'optimisation des séquences de test suivent immédiatement un ou plusieurs codons résultants qui ont été déterminés comme faisant partie de la séquence de nucléotides optimisée, m étant constant pour toutes les étapes d'itération,
**en ce que** le codon défini comme codon de résultat de la séquence de nucléotides optimisée suit immédiatement un ou plusieurs codons résultants qui ont été définis comme faisant partie de la séquence de nucléotides optimisée à une étape d'itération précédente, **en ce que** des séquences de test présentant toutes les occupations de codons possibles pour les m positions d'optimisation sont générées à partir de la première séquence de test à une étape d'itération, et la séquence optimale est déterminée parmi ces séquences de test,

et **en ce que**, à deux ou plusieurs étapes d'itération consécutives, une partie des m positions d'optimisation, auxquelles les codons sont modifiés, sont identiques.

6. Procédé selon la revendication 5, **caractérisé en ce que**, à une ou plusieurs étapes d'itération, les m positions d'optimisation des séquences de test suivent directement un ou plusieurs codons résultants qui ont été définis comme faisant partie de la séquence de nucléotides optimisée,
**en ce que** m est compris entre 3 et 20,
**en ce que** la séquence de test est évaluée en fonction des critères définis par l'utilisateur et/ou des poids de critère pour des critères de test individuels, qui sont ajoutés dans une fonction d'évaluation, sont pondérés en fonction des spécifications définies par l'utilisateur,
**en ce que** la séquence de test est étendue de p codons après chaque étape, **en ce que** lesdites étapes sont itérées jusqu'à ce que tous les codons de la séquence de nucléotides aient été occupés par des codons résultants, à toutes les étapes d'itération à l'exclusion de la dernière étape d'itération, le nombre p des codons résultants étant égal à un, et à la dernière étape d'itération, lorsque n = N et que le nombre de positions non occupées par les codons résultants dans la séquence de test est égal à la valeur m, le nombre p des codons résultants est m.

7. Procédé selon l'une des revendications 1 à 6, **caractérisé par** :

   - l'évaluation de chaque séquence de test par une fonction de qualité,
   - la détermination d'une valeur extrême dans les valeurs de la fonction de qualité pour toutes les séquences partielles générées à une étape d'itération,
   - la définition de p codons de la séquence de test correspondant à la valeur extrême de la fonction de pondération comme codons résultants aux positions correspondantes, p étant un nombre naturel et $p \leq m$,
   - la fonction de qualité prenant en considération un ou plusieurs des critères suivants : usage de codons pour un organisme donné, teneur en GC, séquences répétitives, structures secondaires, les répétitions de séquences complémentaires inverses et motifs de séquence.

8. Procédé selon la revendication 7, **caractérisé en ce que** la fonction de qualité comprend un poids de critère pour les motifs de séquence, lequel poids de critère est sensiblement supérieur à d'autres poids, de sorte qu'il ne puisse pas être compensé par ces autres poids de critère et que le ou les motifs correspondants dans la séquence en soient exclus.

9. Procédé selon la revendication 8, **caractérisé en ce qu'**un ou plusieurs des motifs suivants sont pris en considération :

   séquences de coupe RE, séquences d'activateur, motifs CpG immunostimulants et motifs CpG immunosuppresseurs,
   motifs de séquence cis-actifs, en particulier les sites de liaison d'interaction ADN/protéine et les sites de liaison d'interaction ARN/protéine, de préférence les motifs de séquence, les sites de liaison de facteur de transcription, les sites de liaison de terminaison de transcription, les signaux de polyadénylation, les séquences de reconnaissance d'endonucléase, les motifs ADN immunostimulants, les sites de liaison de ribosomes, les sites de reconnaissance d'enzymes activant la recombinaison, les sites de reconnaissance d'enzymes modifiant l'ADN, les de reconnaissance d'enzymes modifiant l'ARN, les motifs de séquence sous-représentés dans un organisme donné, les séquences répétitives, les structures secondaires, les répétitions inverses, la séquence de reconnaissance CATG de NIa III, BsmBI, BbsI, la séquence Chi, KpnI, SacI.

10. Procédé selon l'une des revendications 1 à 9, **caractérisé en ce que** la fonction de qualité d'un ou de plusieurs des critères suivants sont pris en considération :

    - exclusion d'identités de séquences complémentaires inverses de plus de 20 nucléotides pour le transcriptome d'un organisme donné,
    - exclusion de régions d'homologie de plus de 100 paires de bases pour une séquence d'ADN donnée,
    - exclusion de régions d'homologie avec plus de 90 % de similarité de la séquence de nucléotides pour une séquence d'ADN donnée.

11. Procédé selon l'une des revendications 1 à 10, **caractérisé par** l'étape de synthèse de la séquence de nucléotides optimisée.

**12.** Dispositif d'optimisation d'une séquence de nucléotides pour l'expression d'une protéine sur la base de la séquence d'acides aminés de la protéine, lequel dispositif comporte un moyen de calcul, le dispositif comprenant un moyen pour permettant d'effectuer les étapes d'un procédé selon l'une des revendications 1 à 11.

**13.** Programme informatique qui contient un code de programme exécutable par ordinateur qui, lorsqu'il est exécuté sur un ordinateur, amène celui-ci à exécuter un procédé selon l'une des revendications 1 à 11.

**14.** Support de données lisible par ordinateur, sur lequel un programme selon la revendication 13 est stocké sous une forme lisible par un ordinateur.

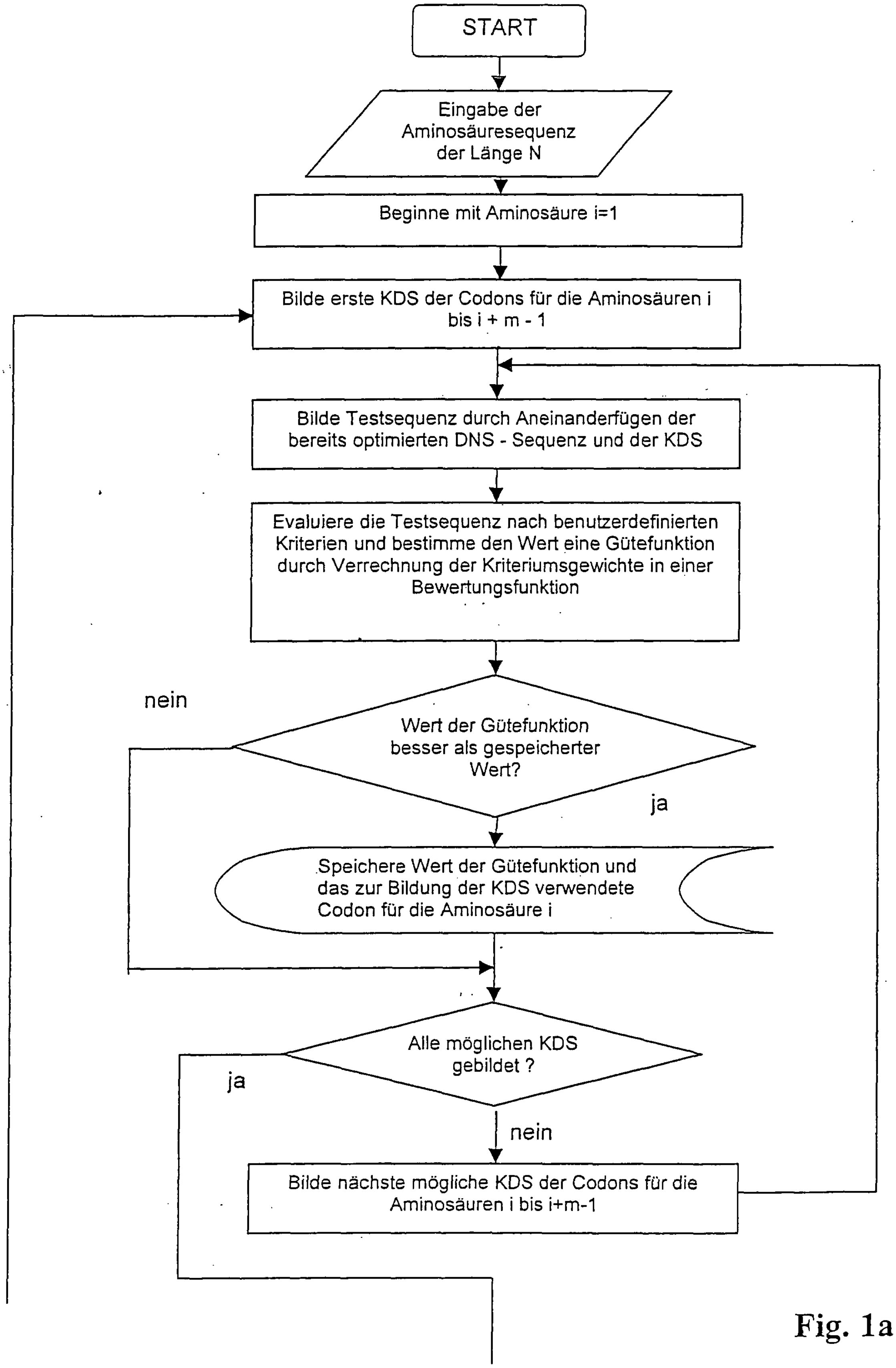
START
Eingabe der Aminosäuresequenz der Länge N
Beginne mit Aminosäure i=1
Bilde erste KDS der Codons für die Aminosäuren i bis i + m - 1
Bilde Testsequenz durch Aneinanderfügen der bereits optimierten DNS - Sequenz und der KDS
Evaluiere die Testsequenz nach benutzerdefinierten Kriterien und bestimme den Wert eine Gütefunktion durch Verrechnung der Kriteriumsgewichte in einer Bewertungsfunktion
nein
Wert der Gütefunktion besser als gespeicherter Wert?
ja
Speichere Wert der Gütefunktion und das zur Bildung der KDS verwendete Codon für die Aminosäure i
Alle möglichen KDS gebildet ?
ja
nein
Bilde nächste mögliche KDS der Codons für die Aminosäuren i bis i+m-1

Fig. 1a

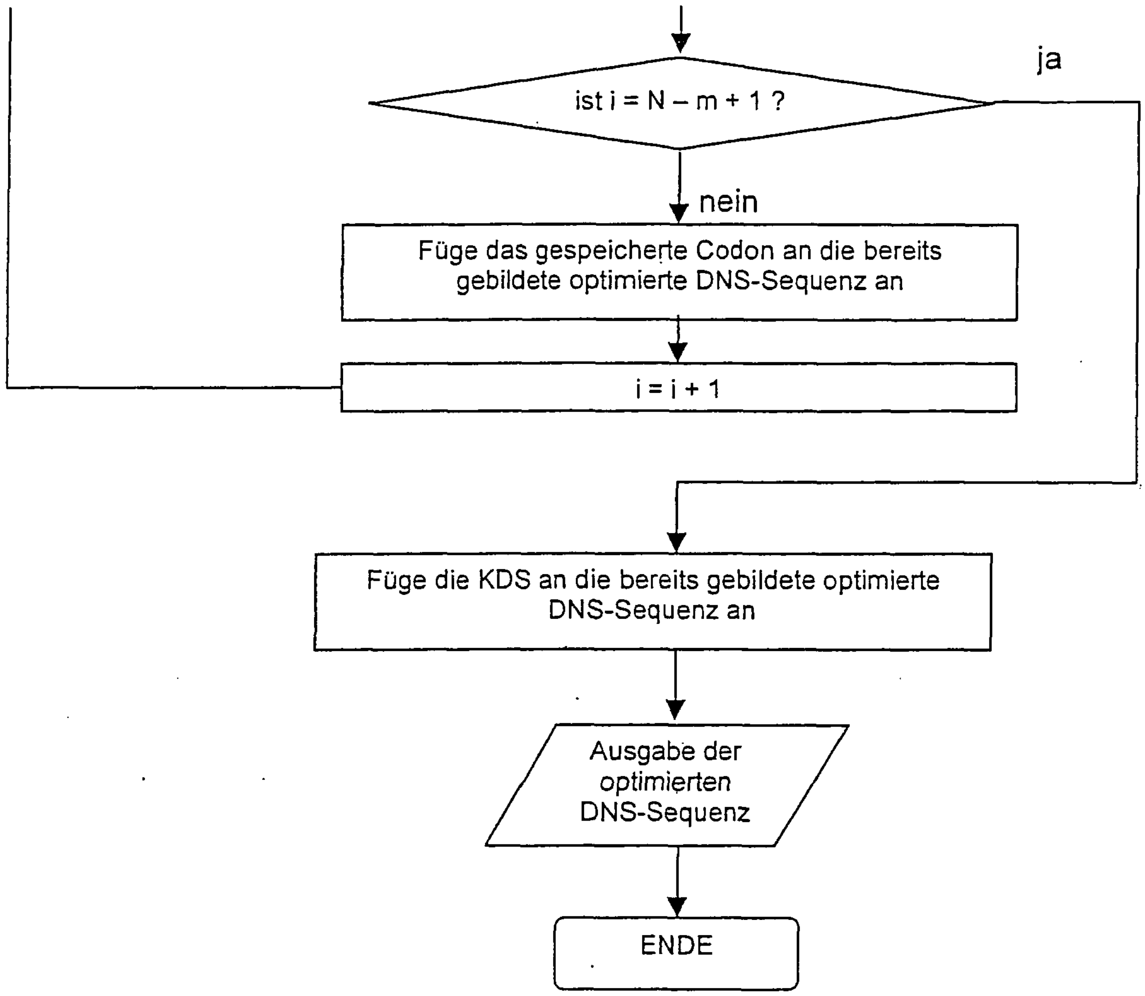
ja
ist i = N – m + 1 ?
nein
Füge das gespeicherte Codon an die bereits gebildete optimierte DNS-Sequenz an
i = i + 1
Füge die KDS an die bereits gebildete optimierte DNS-Sequenz an
Ausgabe der optimierten DNS-Sequenz
ENDE

Fig. 1b

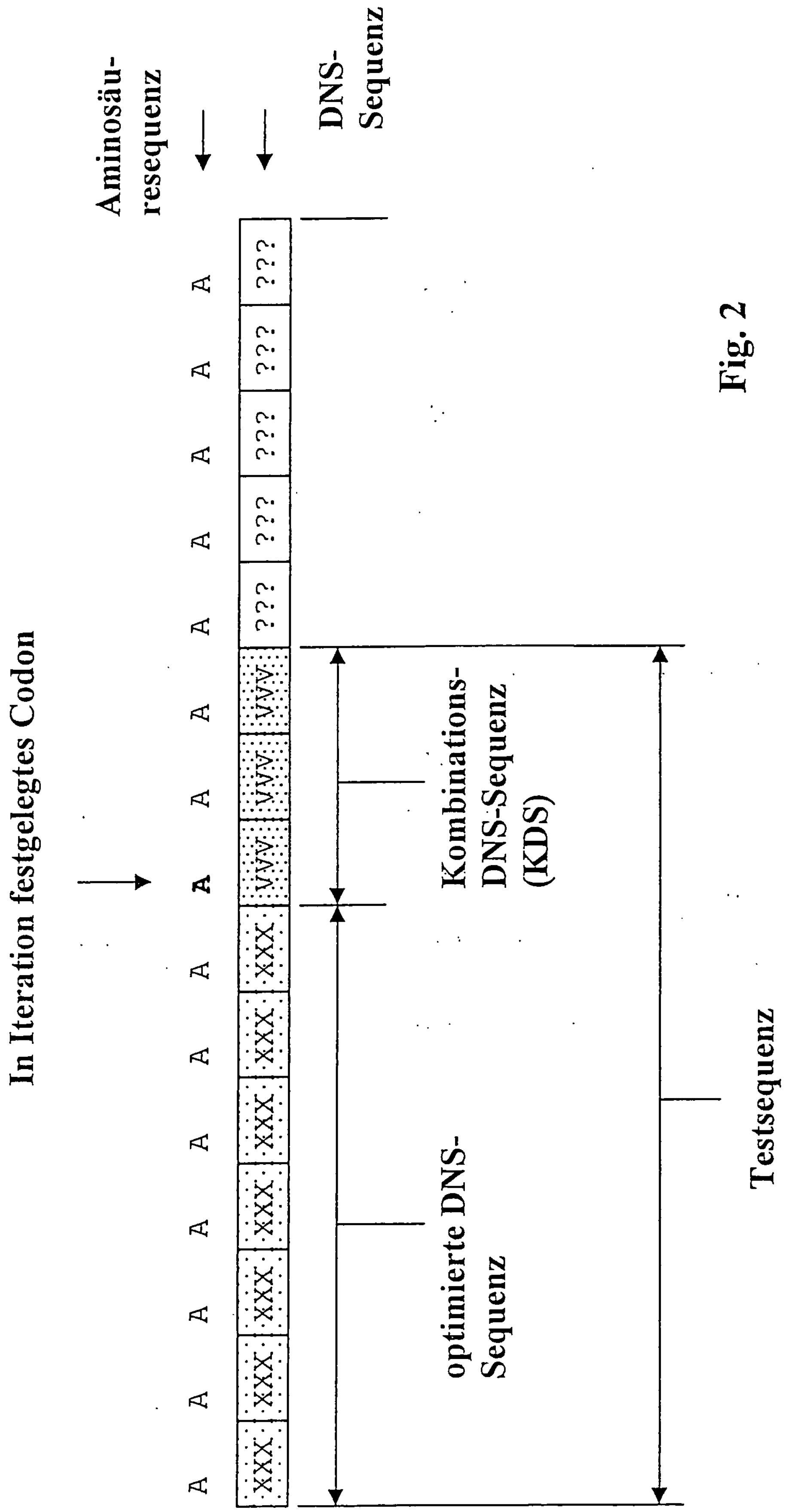
Aminosäuresequenz
DNS-Sequenz
In Iteration festgelegtes Codon
A
XXX
VVV
???
optimierte DNS-Sequenz
Kombinations-DNS-Sequenz (KDS)
Testsequenz

Fig. 2

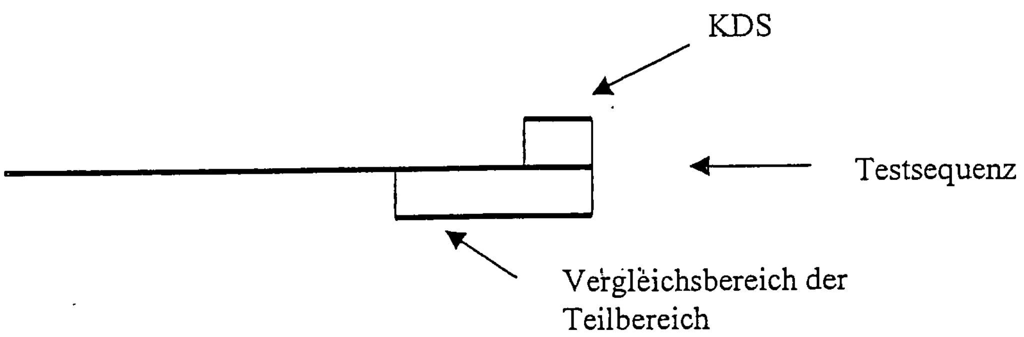


Fig. 3

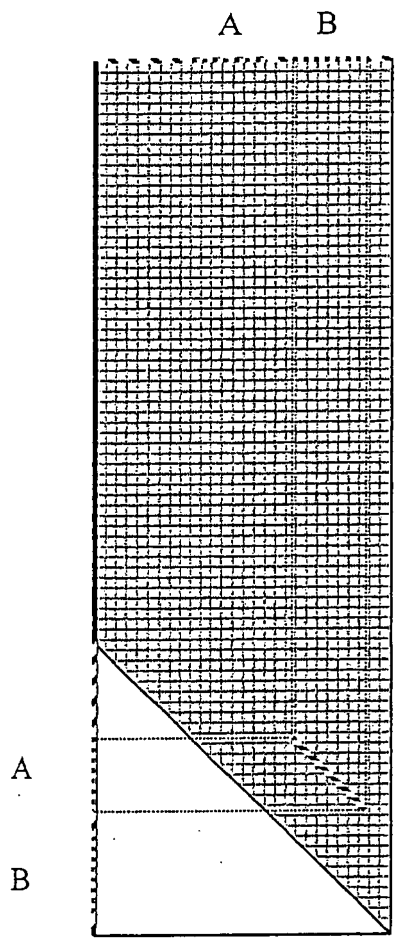


Fig. 4a

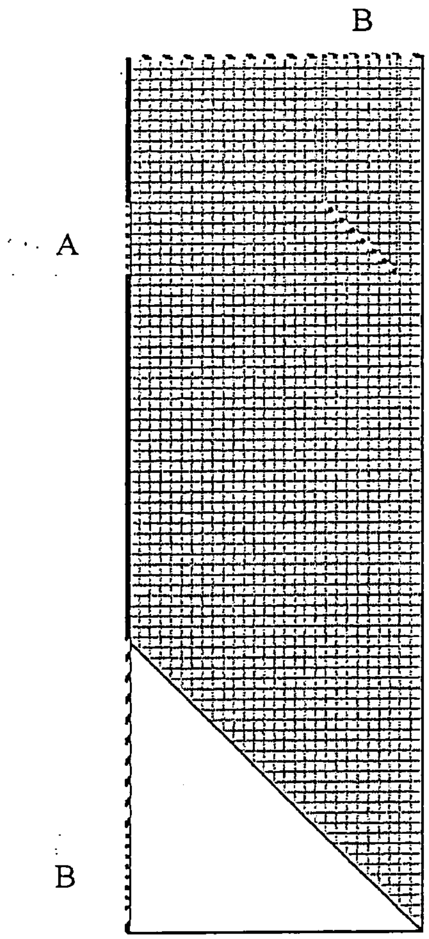


Fig. 4b

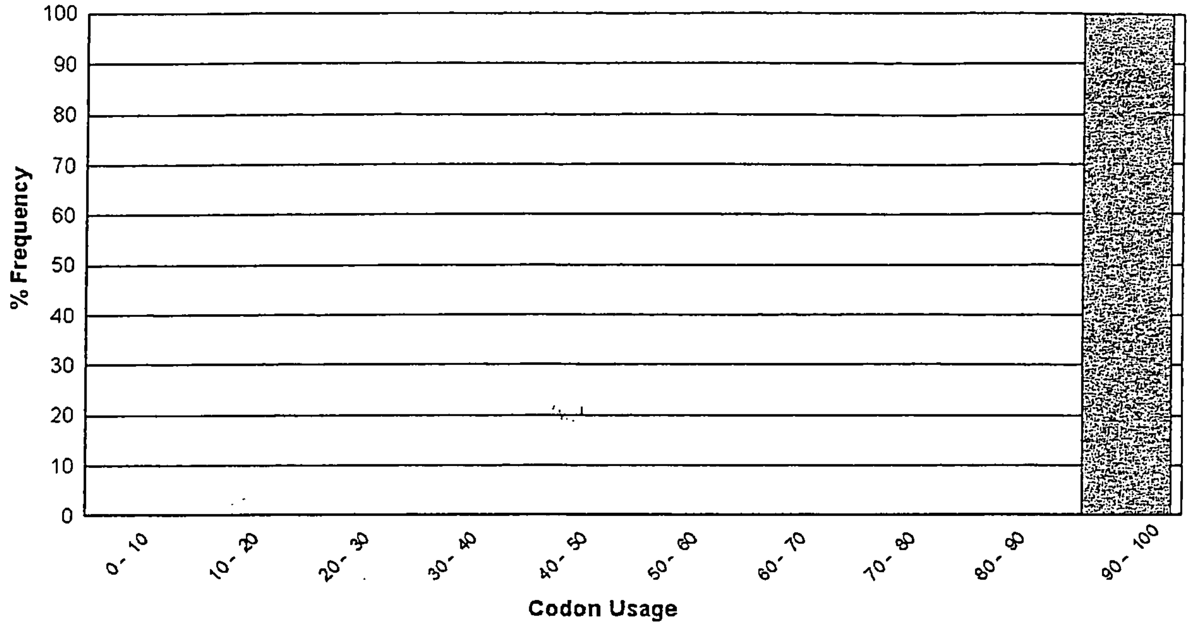
% Frequency
100
90
80
70
60
50
40
30
20
10
0
0 - 10
10 - 20
20 - 30
30 - 40
40 - 50
50 - 60
60 - 70
70 - 80
80 - 90
90 - 100
Codon Usage

Fig. 5a

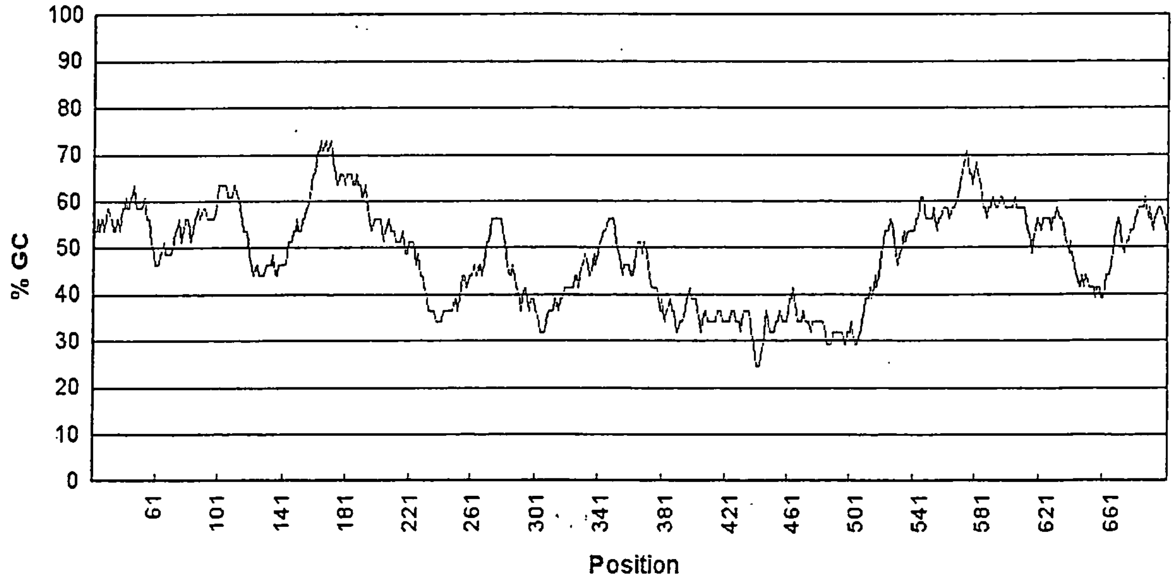
% GC
100
90
80
70
60
50
40
30
20
10
0
61
101
141
181
221
261
301
341
381
421
461
501
541
581
621
661
Position

Fig. 5b

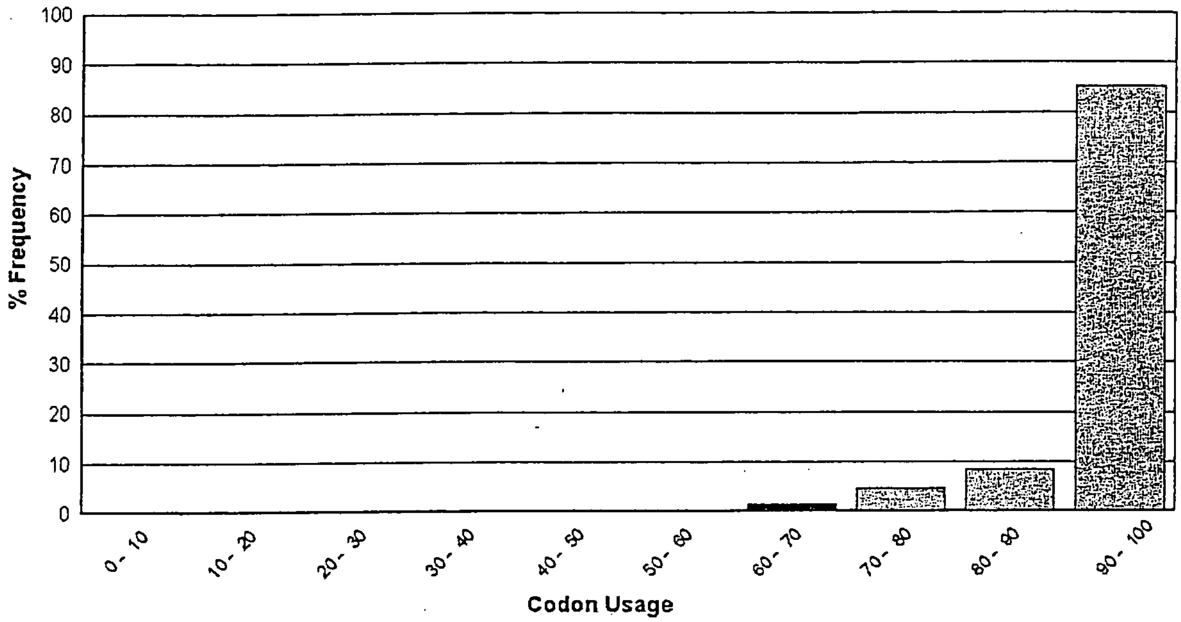
% Frequency
100
90
80
70
60
50
40
30
20
10
0
0 - 10
10 - 20
20 - 30
30 - 40
40 - 50
50 - 60
60 - 70
70 - 80
80 - 90
90 - 100
Codon Usage

Fig. 6a

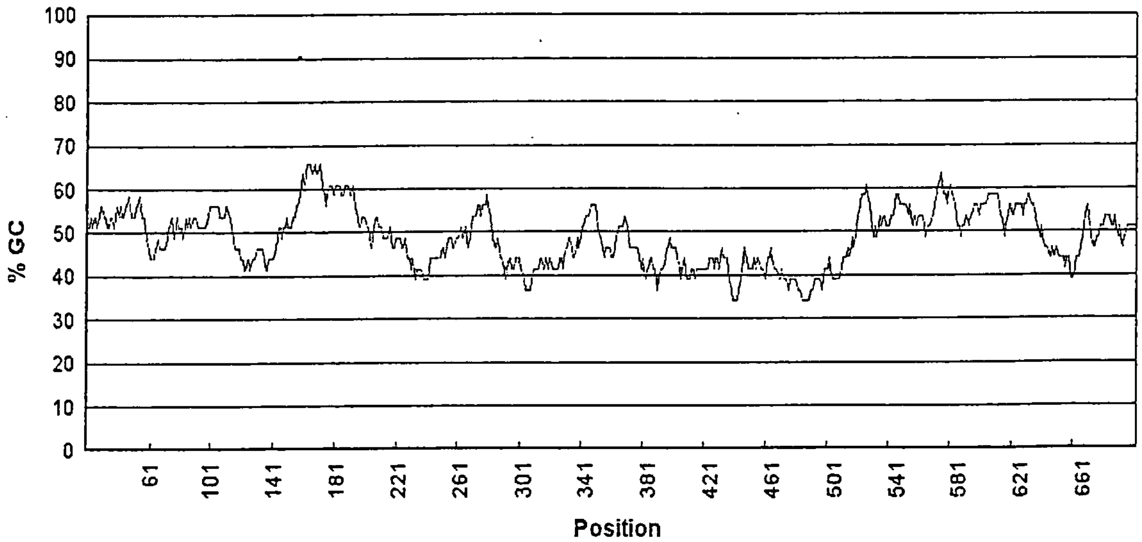
% GC
100
90
80
70
60
50
40
30
20
10
0
61
101
141
181
221
261
301
341
381
421
461
501
541
581
621
661
Position

Fig. 6b

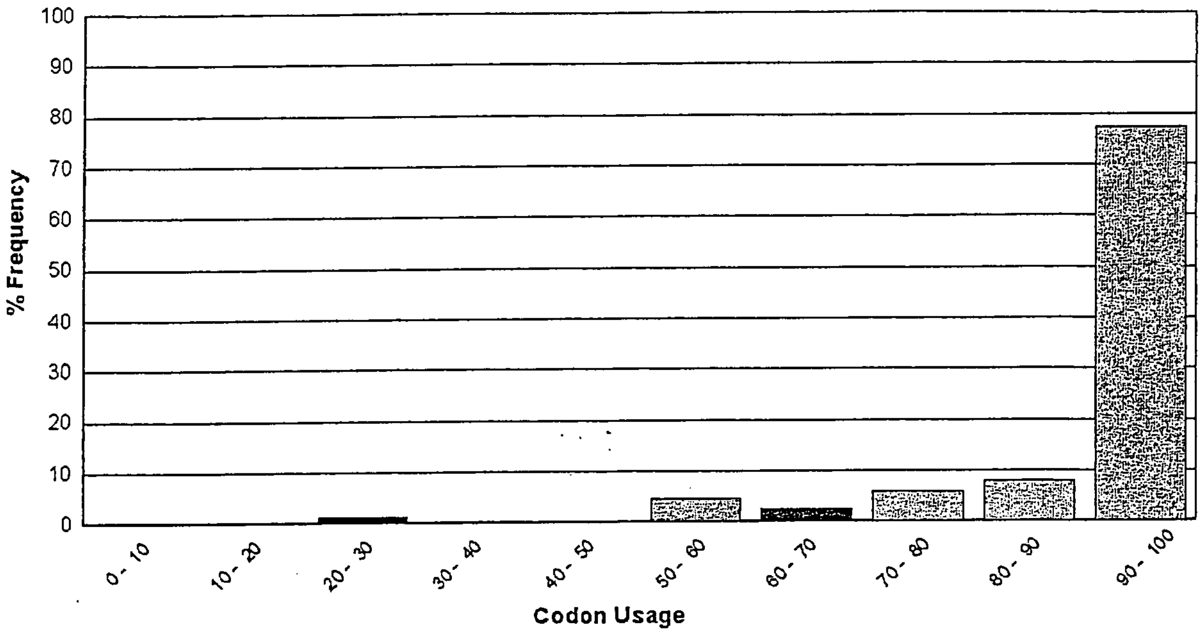
100
90
80
70
60
50
40
30
20
10
0
% Frequency
0 - 10
10 - 20
20 - 30
30 - 40
40 - 50
50 - 60
60 - 70
70 - 80
80 - 90
90 - 100
Codon Usage

Fig. 7a

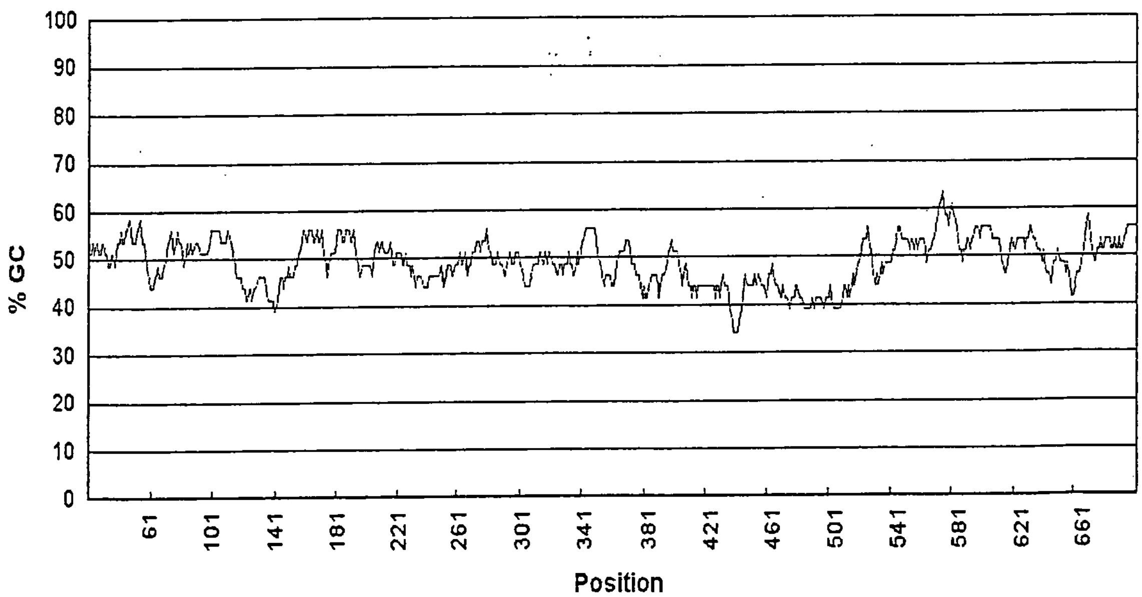
100
90
80
70
60
50
40
30
20
10
0
% GC
61
101
141
181
221
261
301
341
381
421
461
501
541
581
621
661
Position

Fig. 7b

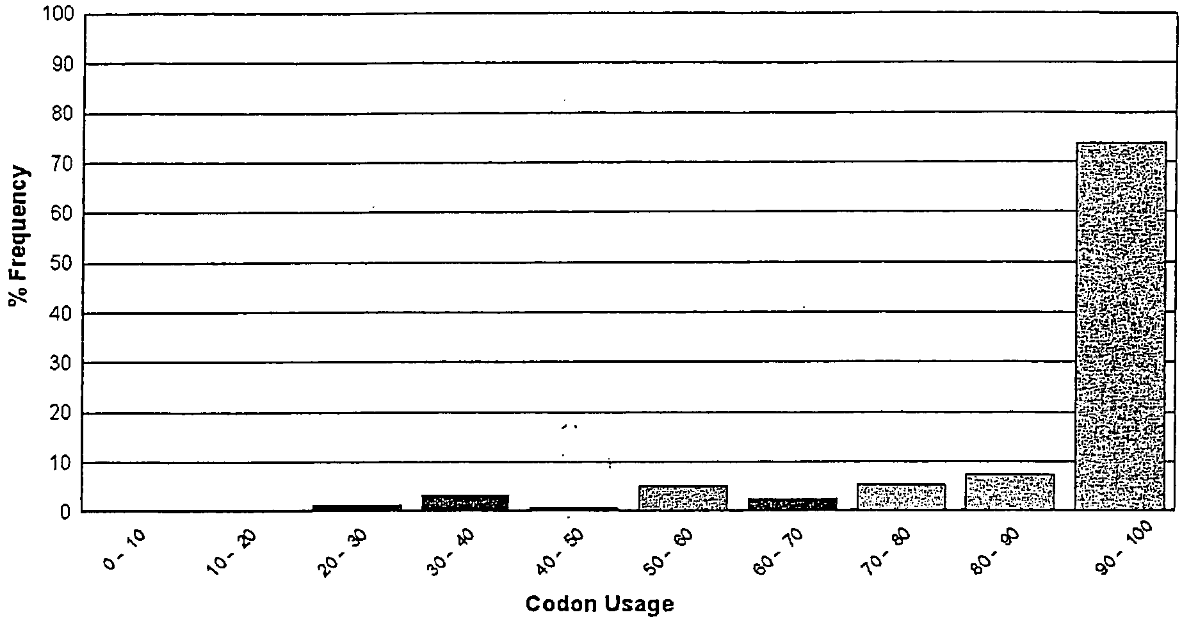
% Frequency
100
90
80
70
60
50
40
30
20
10
0
0 - 10
10 - 20
20 - 30
30 - 40
40 - 50
50 - 60
60 - 70
70 - 80
80 - 90
90 - 100
Codon Usage

Fig. 8a

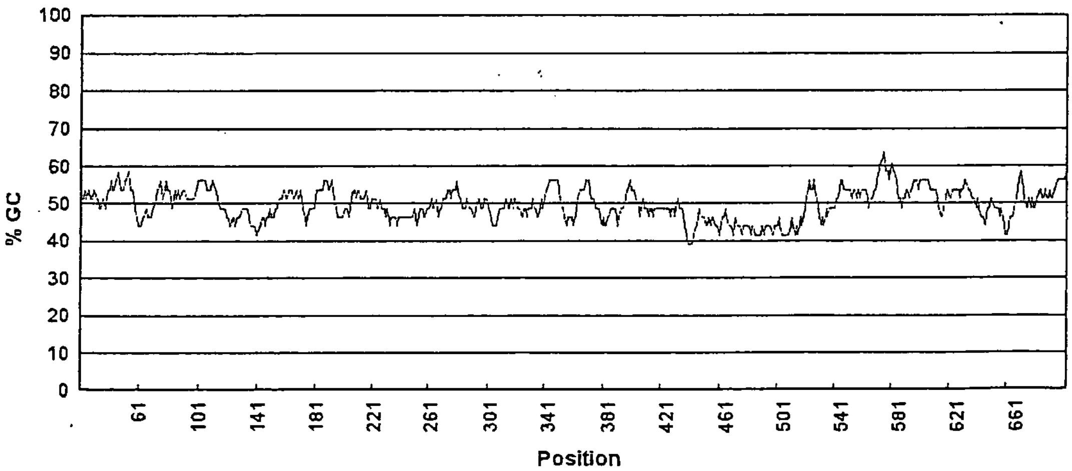
% GC
100
90
80
70
60
50
40
30
20
10
0
61
101
141
181
221
261
301
341
381
421
461
501
541
581
621
661
Position

Fig. 8b

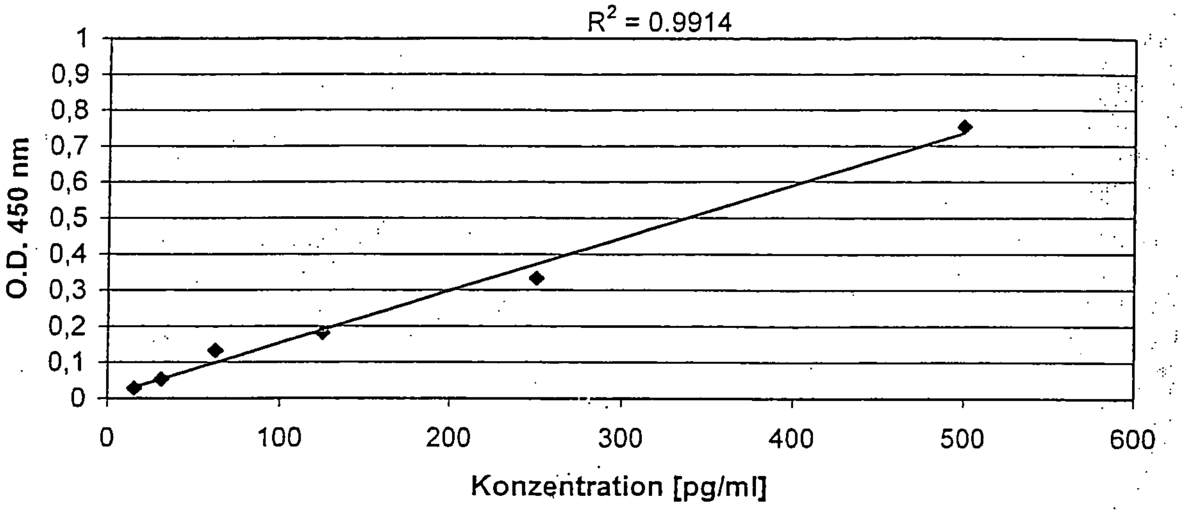
$R^2$ = 0.9914
1
0,9
0,8
0,7
0,6
0,5
0,4
0,3
0,2
0,1
0
O.D. 450 nm
0
100
200
300
400
500
600
Konzentration [pg/ml]

Fig. 9

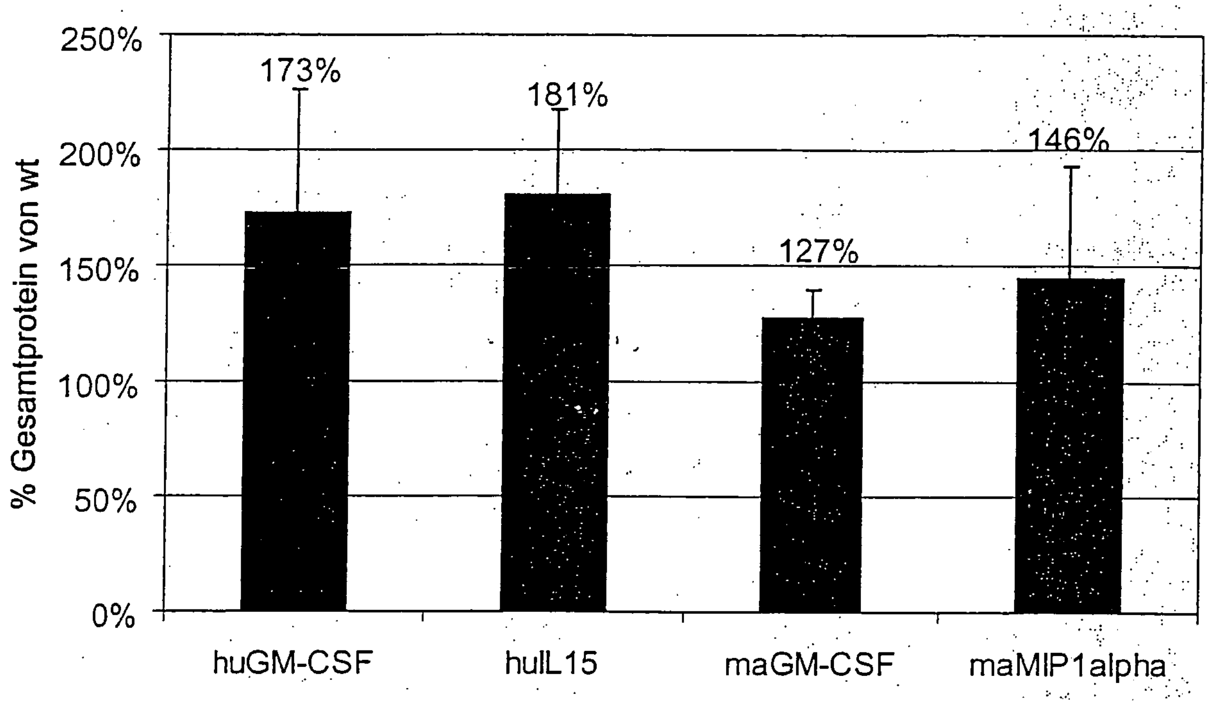
250%
200%
150%
100%
50%
0%
% Gesamtprotein von wt
173%
181%
127%
146%
huGM-CSF
huIL15
maGM-CSF
maMIP1alpha

Fig. 10

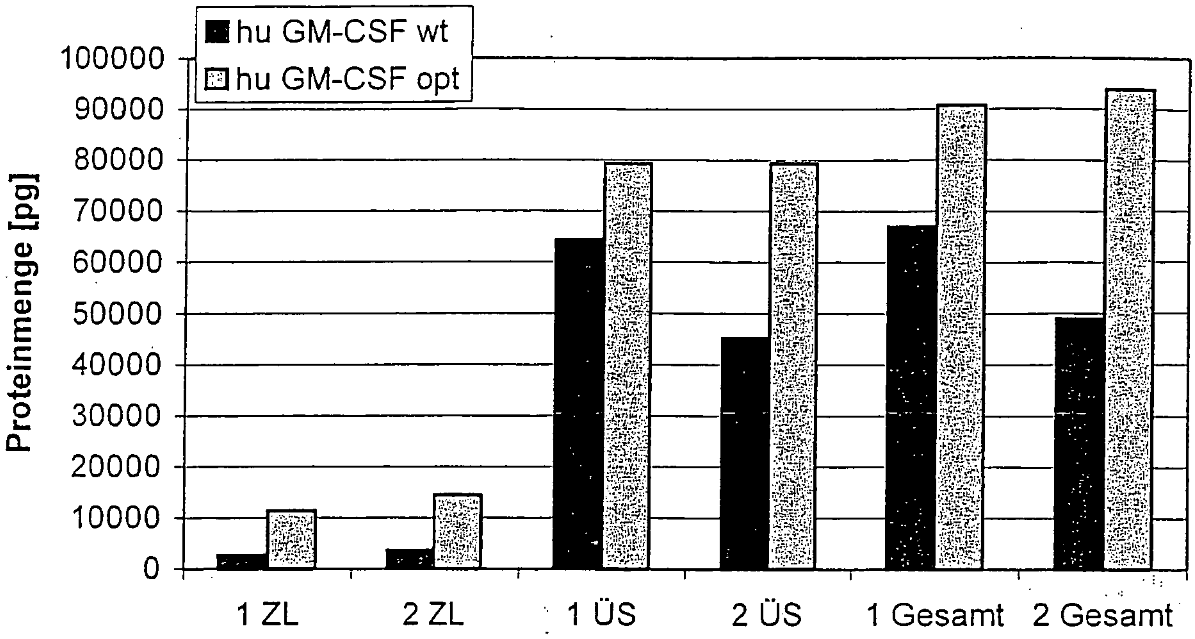
hu GM-CSF wt
hu GM-CSF opt
100000
90000
80000
70000
60000
50000
40000
30000
20000
10000
0
Proteinmenge [pg]
1 ZL
2 ZL
1 ÜS
2 ÜS
1 Gesamt
2 Gesamt

Fig. 11

Humanes GM-CSF

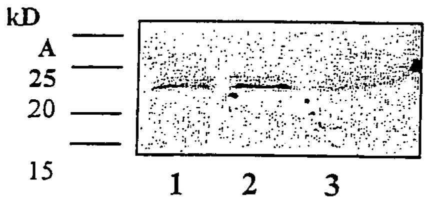


1: wildtyp
2: optimiert
3: Mock

Fig. 12A

Murines GM-CSF

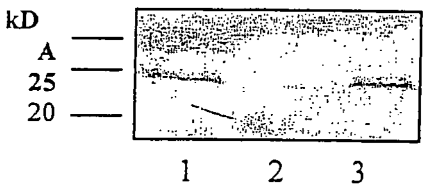


1: wildtyp
2: Mock
3: optimiert

Fig. 12B

Humanes IL15

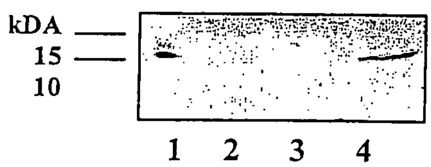


1: rek. IL15 (BD)
2: wildtyp
3: Mock

Fig. 12C

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **P.S. SARKAR ; SAMIR K. BRAHMACHARI.** *Nucleic Acids Research,* 1992, vol. 20, 5713
- **D.M. HOOVER ; J. LUBKOWSKI.** *Nucleic Acid Research,* 2002, vol. 30 (10), e43
- **P. M. SHARP ; W. H. LI.** *Nucleic Acids Research,* 1987, vol. 15 (3), 1281-1295
- **MICHAEL S. WATERMAN.** *Introduction to Computational Biology,* 2000, 207-209
- **DAN GUSFIELD.** *Algorithms on Strings, Trees and Sequences,* 1999, 215-235
- **P. M. SHARP ; W. H. LI.** *Nucleic Acid Research,* 1987, vol. 15, 1281-1295
- **BEISPIEL K. ; QUANDT U.A.** *Nucleic Acid Research,* 1995, vol. 23, 4878
- **M. S. WATERMAN ; M. EGGERT.** *J. Mol. Biology,* 1987, vol. 197, 723-728
- **L. KADERALI ; A. SCHLIEP.** *Bioinformatics,* 2002, vol. 18 (10), 1340-1349

SEQUENCE LISTING

<110> GeneArt AG

<120> Verfahren und Vorrichtung zum Optimieren einer Nucleotidsequenz zur Expression eines Proteins

<130> FB15647/A

<160> 8

<170> PatentIn version 3.2

<210> 1
<211> 435
<212> DNA
<213> Homo sapiens

<400> 1

atgtggctgc agagcctgct gctcttgggc actgtggcct gcagcatctc tgcacccgcc 60

cgctcgccca gccccagcac gcagccctgg gagcatgtga atgccatcca ggaggcccgg 120

cgtctcctga acctgagtag agacactgct gctgagatga atgaaacagt agaagtcatc 180

tcagaaatgt ttgacctcca ggagccgacc tgcctacaga cccgcctgga gctgtacaag 240

cagggcctgc ggggcagcct caccaagctc aagggcccct tgaccatgat ggccagccac 300

tacaagcagc actgccctcc aaccccggaa acttcctgtg caacccagat tatcaccttt 360

gaaagtttca aagagaacct gaaggacttt ctgcttgtca tcccctttga ctgctgggag 420

ccagtccagg agtag 435

<210> 2
<211> 435
<212> DNA
<213> Homo sapiens

<400> 2

atgtggctgc agagcctgct gctgctggga acagtggcct gtagcatctc tgcccctgcc 60

agaagcccta gccctagcac acagccttgg gagcacgtga atgccatcca ggaggccagg 120

agactgctga acctgagcag agatacagcc gccgagatga acgagaccgt ggaggtgatc 180

agcgagatgt tcgacctgca ggagcctaca tgcctgcaga cccggctgga gctgtataag 240

cagggcctga gaggctctct gaccaagctg aagggccccc tgacaatgat ggccagccac 300

tacaagcagc actgccctcc tacccctgag acaagctgcg ccacccagat catcaccttc 360

gagagcttca aggagaacct gaaggacttc ctgctggtga tccccttcga ttgctgggag 420

cccgtgcagg agtag 435

<210> 3
<211> 489
<212> DNA
<213> Homo sapiens

<400> 3

```
atgagaattt cgaaaccaca tttgagaagt atttccatcc agtgctactt gtgtttactt     60
ctaaacagtc attttctaac tgaagctggc attcatgtct tcattttggg ctgtttcagt    120
gcagggcttc ctaaaacaga agccaactgg gtgaatgtaa taagtgattt gaaaaaaatt    180
gaagatctta ttcaatctat gcatattgat gctactttat atacggaaag tgatgttcac    240
cccagttgca aagtaacagc aatgaagtgc tttctcttgg agttacaagt tatttcactt    300
gagtccggag atgcaagtat tcatgataca gtagaaaatc tgatcatcct agcaaacaac    360
agtttgtctt ctaatgggaa tgtaacagaa tctggatgca aagaatgtga ggaactggag    420
gaaaaaaata ttaaagaatt tttgcagagt tttgtacata ttgtccaaat gttcatcaac    480
acttcttag                                                            489
```

<210> 4
<211> 489
<212> DNA
<213> Homo sapiens

<400> 4

```
atgcggatca gcaagcccca cctgaggagc atcagcatcc agtgctacct gtgcctgctg     60
ctgaacagcc acttcctgac agaggccggc atccacgtgt ttatcctggg ctgcttctct    120
gccggcctgc ctaagacaga ggccaactgg gtgaacgtga tcagcgacct gaagaagatc    180
gaggacctga tccagagcat gcacatcgac gccaccctgt acacagagag cgacgtgcac    240
cctagctgta aggtgaccgc catgaagtgc ttcctgctgg agctgcaggt gatcagcctg    300
gagagcggcg atgccagcat ccacgacacc gtggagaacc tgatcatcct ggccaacaac    360
agcctgagca gcaacggcaa tgtgaccgag agcggctgca aggagtgtga ggagctggag    420
gagaagaaca tcaaggagtt cctgcagagc ttcgtgcaca tcgtgcagat gttcatcaac    480
accagctag                                                            489
```

<210> 5
<211> 426
<212> DNA
<213> Mus musculus

<400> 5

atgtggctgc agaatttact tttcctgggc attgtggtct acagcctctc agcacccacc 60

cgctcaccca tcactgtcac ccggccttgg aagcatgtag aggccatcaa agaagccctg 120

aacctcctgg atgacatgcc tgtcacattg aatgaagagg tagaagtcgt ctctaacgag 180

ttctccttca agaagctaac atgtgtgcag acccgcctga agatattcga gcagggtcta 240

cggggcaatt tcaccaaact caagggcgcc ttgaacatga cagccagcta ctaccagaca 300

tactgccccc caactccgga aacggactgt gaaacacaag ttaccaccta tgcggatttc 360

atagacagcc ttaaaacctt tctgactgat atcccctttg aatgcaaaaa accaggccaa 420

aaatag 426

<210> 6
<211> 426
<212> DNA
<213> Mus musculus

<400> 6

atgtggctgc agaacctgct gttcctgggc atcgtggtgt acagcctgag cgcccccacc 60

aggagcccca tcaccgtgac caggccctgg aagcacgtgg aggccatcaa ggaggccctg 120

aacctgctgg acgacatgcc cgtgaccctg aacgaggagg tggaggtggt gagcaacgag 180

ttcagcttca agaagctgac ctgcgtgcag accaggctga agatcttcga gcagggcctg 240

aggggcaact tcaccaagct gaagggcgcc ctgaacatga ccgccagcta ctaccagacc 300

tactgccccc ccacccccga gaccgactgc gagacccagg tgaccaccta cgccgacttc 360

atcgacagcc tgaagacctt cctgaccgac atccccttcg agtgcaagaa gcccggccag 420

aagtag 426

<210> 7
<211> 279
<212> DNA
<213> Mus musculus

<400> 7

atgaaggtct ccaccactgc ccttgctgtt cttctctgta ccatgacact ctgcaaccaa 60

gtcttctcag cgccatatgg agctgacacc ccgactgcct gctgcttctc ctacagccgg 120

aagattccac gccaattcat cgttgactat tttgaaacca gcagcctttg ctcccagcca 180

ggtgtcattt tcctgactaa gagaaaccgg cagatctgcg ctgactccaa agagacctgg 240

gtccaagaat acatcactga cctggaactg aatgcctag 279

<210> 8
<211> 279
<212> DNA
<213> Mus musculus

<400> 8

```
atgaaggtga gcaccacagc tctggctgtg ctgctgtgca ccatgaccct gtgcaaccag     60

gtgttcagcg ctccttacgg cgccgatacc cctacagcct gctgcttcag ctacagcagg    120

aagatcccca ggcagttcat cgtggactac ttcgagacca gcagcctgtg ttctcagccc    180

ggcgtgatct tcctgaccaa gcggaacaga cagatctgcg ccgacagcaa ggagacatgg    240

gtgcaggagt acatcaccga cctggagctg aacgcctag                           279
```